# EUROPEAN PATENT SPECIFICATION

(11) **EP 4 423 075 B1**
(45) Date of publication and mention of the grant of the patent: **25.03.2026**
(21) Application number: 22812531.6
(22) Date of filing: 27.10.2022
(51) Int. Cl.: C07D 401/06, C07D 401/14, C07D 413/14, A61P 35/00, A61K 31/4439, A61K 31/444

(54) **CCR6 RECEPTOR MODULATORS**
CCR6-REZEPTORMODULATOREN
MODULATEURS DU RÉCEPTEUR CCR6

(30) Priority: 28.10.2021 WO PCT/EP2021/080016
(43) Date of publication of application: 04.09.2024
(73) Proprietor: Idorsia Pharmaceuticals Ltd, 4123 Allschwil (CH)
(72) Inventor: ALLEMANN, Oliver, 4123 Allschwil (CH); HUBLER, Francis, 4123 Allschwil (CH); MEYER, Emmanuel, 4123 Allschwil (CH)
(74) Representative: Kamdzhilov, Yavor
(86) International application number: PCT/EP2022/080045
(87) International publication number: WO 2023/073082

(56) References cited:
- WO-A1-2015/057626
- WO-A1-2019/147862
- WO-A1-2020/058869
- WO-A1-2021/219849
- US-A1- 2015 111 870
- US-A1- 2019 233 397
- US-A1- 2020 095 239
- ROBERT REMY ET AL: "Essential role for CCR6 in certain inflammatory diseases demonstrated using specific antagonist and knockin mice", JCI INSIGHT, vol. 2, no. 15, 3 August 2017 (2017-08-03), XP055982063, DOI: 10.1172/jci.insight.94821

## Description

The present invention relates to novel compounds of Formula (I), or pharmaceutically acceptable salts thereof, and their use as CCR6 receptor modulators in the treatment or prevention of various diseases, conditions or disorders ameliorated by modulating said receptor. Furthermore, the present invention concerns related aspects such as pharmaceutical compositions containing one or more compounds of Formula (I) and processes for the preparation of said compounds.

Chemokine receptors comprise a family G-protein coupled receptors (GPCRs) that recognize and bind to peptide chemokine ligands. The predominant functions of chemokine receptors and their ligands are to induce leukocyte trafficking to-and-from lymphoid organs and tissues in the steady state, as well as in the context of an infection or inflammation. Additionally, chemokine signaling events can induce the activation of integrin molecules on the surface of immune cells, allowing firm adhesion to activated endothelium, facilitating migration from blood into inflamed tissue (Montresor A, Frontiers in Imm., 2012; Meissner A, Blood, 2003). Chemokine receptor 6 (CCR6, aliases BN-1, C-C CKR-6, CD196, CKRL3, CMKBR6, DCR2, DRY6, GPR29, GPRCY4, STRL22) is a GPCR mainly expressed on effector CD4+ T helper cells, but is also present on B cells, CD8+ cytotoxic T cells, regulatory T cells (Treg), immature dendritic cells (DC) and type 3 innate lymphoid cells (ILC3) (Cua DJ, Nat Rev Immunol. 2010 Jul; 10(7):479-89. doi: 10.1038/nri2800). CCR6 binds to the chemokine CCL20 (chemokine (C-C motif) ligand 20) (Greaves DR, J Exp Med. 1997 Sep 15; 186(6):837-44. doi: 10.1084/jem.186.6.837.). CCL20 is also called macrophage inflammatory protein 3a (MIP-3a), liver and activation-regulated chemokine (LARC), or Exodus-1 (Schutyser E, Cytokine Growth Factor Rev. 2003 Oct; 14(5):409-26. doi: 10.1016/s1359-6101(03)00049-2). CCR6/CCL20 interactions dictate the humoral response in the intestinal mucosa and are required for lymphocyte homeostasis in the mucosa of the small intestine (Cook DN, Immunity. 2000 May; 12(5):495-503. doi: 10.1016/s1074-7613(00)80201-0). Under steady state conditions, CCR6 and CCL20 regulate production of IgA in the intestine, where CCL20 expressed in Peyer's patches guides CCR6+IgA+ B cells to the mucosa and secretory IgA can be released into the gut lumen (Lin YL, Front Immunol. 2017; 8:805. doi: 10.3389/fimmu.2017.00805; Reboldi A, Science. 2016 May 13; 352(6287):aaf4822. doi: 10.1126/science.aaf4822). Under inflammatory conditions, expression of CCL20 is highly upregulated by proinflammatory cytokines including IL-17A, TNFa and IL-1b in both endothelial and epithelial cells (Harper EG, J Invest Dermatol. 2009 Sep; 129(9):2175-83. doi: 10.1038/jid.2009.65; PLoS One. 2015; 10(11):e0141710. doi: 10.1371/journal.pone.0141710) and tissue fibroblasts (Hattori T, Mediators Inflamm. 2015; 2015:436067. doi: 10.1155/2015/436067). Interleukin (IL)-17A expression is restricted to cells expressing the transcription factor RORgt (Cell. 2006 Sep 22; 126(6):1121-33. doi: 10.1016/j.cell.2006.07.035). IL-17A expression has been shown to segregate with CCR6 expression on human T cells (Singh SP, J Immunol. 2008 Jan 1; 180(1):214-21. doi: 10.4049/jimmunol.180.1.214; Nat Immunol. 2007 Jun; 8(6):639-46. doi: 10.1038/ni1467). CCR6 was also described as a target gene of RORgt (PLoS One. 2017; 12(8):e0181868. doi: 10.1371/journal.pone.0181868; Skepner J, J Immunol. 2014 Mar 15; 192(6):2564-75. doi: 10.4049/jimmunol.1302190), thus clarifying the co-expression of IL-17A and CCR6 in RORgt+ cell types.

Certain disclosures in the prior art may be regarded as relating to modulation of CCR6. For instance, Tawaraishia et al. (Bioorganic & Medicinal Chemistry Letters, Volume 28, Issue 18, 2018, Pages 3067-3072, ISSN 0960-894X, https://doi.org/10.1016/j.bmcl.2018.07.042) disclose a series of benzenesulfonyl-aminocyclohexane derivatives as selective CCR6 inhibitors. CN103588697 teaches sulfonamide derivatives as CCR6 antagonists and their use in treating CCR6-mediated diseases such as autoimmune diseases, inflammation, psoriasis, multiple sclerosis or cancer. WO2014/075580 describes the use of aurintricarboxylic acid for targeting chemokine receptors. WO2015/084842 teaches certain sulfonamides which may be used in treating CCR6 related diseases. WO2017/087607, WO2010/131145, WO2013/061004, WO2013/061005, WO2019/036374 and WO2020/058869 provide certain cyclobutenediones for use in the treatment of chemokine/CCR6 related diseases. WO2019/136370 teaches a method of treating a certain type of psoriasis. WO2019/147862 proposes azetidine derivatives which may be used as chemokine modulators. WO2021219849 relates to certain CCR6 receptor modulators.

Further, WO1999/43664 discloses certain pyrrolidinones with anti-inflammatory and analgesic properties. In WO2019/105915 certain heterocyclic compounds are provided which may be used as MAGL inhibitors. WO2015/057626, US2015/0105366, WO2014/062658, WO2015/057205 and Tanis VM et al. (Bioorg Med Chem Lett. 2019 Jun 15; 29(12):1463-1470. doi: 10.1016/j.bmcl.2019.04.021) relate to modulators of the RORyt receptor which may be used in treating rheumatoid arthritis or psoriasis. WO03/022808 proposes certain azetidine derivatives for use as pesticides. WO2008/103426 and WO2007/022351 disclose certain quaternary ammonium compounds useful as muscarinic receptor antagonists. WO2006/136830 teaches certain heteroaryl-alkylamines as protein kinase inhibitors. WO91/13359 proposes heterocyclic cholinergic enhancers. US3458635 teaches certain pyrrolidines which may be used for treating depression. GB 1304650 discloses spasmolytic pyrrolidines. US3479370, US3489769, US3499002, US 3542807 and US3651085 relate to certain pyrrolidines with analgesic/tranquilizing activity.

The present CCR6 modulators may be useful, alone, or in combination in the treatment or prevention of the following diseases or disorders: Rheumatoid arthritis (RA) causes chronic inflammation of the joints and chemokines regulate infiltration of the inflamed synovium by inflammatory cells. RA is characterized by the increased release of CCL20 and the subsequent recruitment of CCR6+ T cells to the inflamed joints. CCL20 is highly expressed in the synovial fluid of RA (Hirota, J Exp Med. 2007 Nov 26; 204(12):2803-12. doi: 10.1084/jem.20071397; Matsui T, Clin Exp Immunol. 2001 Jul; 125(1):155-61. doi: 10.1046/j.1365-2249.2001.01542.x). In patients with RA, CCR6+ Th cells have been found in the inflamed synovium and increased proportions of peripheral blood CCR6+ Th cells have been found in patients with early RA (van Hamburg JP, Arthritis Rheum. 2011 Jan; 63(1):73-83. doi: 10.1002/art.30093; Leipe J Arthritis Rheum. 2010 Oct; 62(10):2876-85. doi: 10.1002/art.27622; Nistala K, Arthritis Rheum. 2008 Mar; 58(3):875-87. doi: 10.1002/art.23291). The production of CCL20 is known to be up-regulated in synovium explants or fibroblast-like synoviocytes from RA patients after stimulation of TNF-a, IL-1b and IL-17 (Matsui T, Clin Exp Immunol. 2001 Jul; 125(1):155-61. doi: 10.1046/j.1365-2249.2001.01542.x; J Immunol. 2001 Nov 15; 167(10):6015-20. doi: 10.4049/jimmunol.167.10.6015; Chevrel G, Ann Rheum Dis. 2002 Aug; 61(8):730-3. doi: 10.1136/ard.61.8.730). CCR6+ B cells in RA synovium have been reported, contributing to pathogenesis by antigen presentation, autoantibody production and/or inflammatory cytokine production. Furthermore, Rituximab is an efficacious therapy for RA (Cohen SB, Arthritis Rheum. 2006 Sep; 54(9):2793-806. doi: 10.1002/art.22025), supporting a role for CCR6+ B cells in RA pathogenesis. Additionally, CCR6-deficient mice have impaired IgG1-dependent memory B cell responses (J Immunol. 2015 Jan 15; 194(2):505-13. doi: 10.4049/jimmunol.1401553). Preclinical rodent models showed that CCR6-deficient mice developed a less severe joint inflammation in the collagen-induced arthritis (CIA) model. Reduced production of collagen-specific antibodies in CCR6-deficient mice were observed compared to WT mice, and arthritic inflammation was also reduced (J Cell Mol Med. 2018 Nov; 22(11):5278-5285. doi: 10.1111/jcmm.13783). Furthermore, depletion of CCR6+ cells reduced the severity of SKG arthritis (Hirota K, J Exp Med. 2007 Nov 26; 204(12):2803-12. doi: 10.1084/jem.20071397).

CCR6+ Th17 are increased in peripheral blood in ankylosing spondylitis patients (Shen H, Arthritis Rheum. 2009 Jun; 60(6):1647-56. doi: 10.1002/art.24568). Circulating interleukin-17-secreting interleukin-23 receptor-positive γ/δ T cells were also reported in patients with active ankylosing spondylitis (Kenna TJ, Arthritis Rheum. 2012 May; 64(5):1420-9. doi: 10.1002/art.33507). Secukinumab, an IL-17A inhibitor, in was shown to be efficacious in ankylosing spondylitis (AS) (Baeten D, N Engl J Med. 2015 Dec 24; 373(26):2534-48. doi: 10.1056/NEJMoa1505066). CD32B expression on memory B cells in AS was increased and was associated with disease activity. Furthermore, CCR6⁺ cytotoxic T-cells and CD32B⁺ memory B-cells were highly enriched within the synovial compartment of AS patients (Sucur A, Clin Exp Rheumatol. 2019 Nov 20; PMID: 31820725).

Psoriasis is a commonly occurring autoimmune skin disease. The role of Th17-associated cytokines has been clinically validated and their role in psoriatic inflammation confirmed (Paul C, J Eur Acad Dermatol Venereol. 2015 Jun; 29(6):1082-90. doi: 10.1111/jdv.12751). An IL-17R-blocking antibody (brodalumab, AMG 827) were shown to reduce clinical manifestations of psoriasis and also to reduce CCL20 expression in skin biopsies from psoriasis patients (Papp KA, N Engl J Med. 2012 Mar 29; 366(13):1181-9. doi: 10.1056/NEJMoa1109017). Also, an IL-23 neutralizing antibody (guselkumab) was shown to be efficacious in reducing psoriatic inflammation (Reich K, Lancet. 2019 Sep 7; 394(10201):831-839. doi: 10.1016/S0140-6736(19)31773-8). CCR6-deficient mice failed to develop psoriasiform skin lesions following intradermal IL-23 injections (Hedrick MN, J Clin Invest. 2009 Aug; 119(8):2317-29. doi: 10.1172/jci37378). Small molecule CCR6 antagonists have also been shown to be efficacious in the Aldara and IL-36a-injection mouse psoriasis models (Campbell JJ, J Immunol. 2019 Mar 15; 202(6):1687-1692. doi: 10.4049/jimmunol.1801519; Campbell JJ, J Immunol. 2017 Nov 1; 199(9):3129-3136. doi: 10.4049/jimmunol.1700826).

Furthermore, CCR6-deficient mice have been shown to be protected from imiquimod-induced ear swelling (Yu S, J Invest Dermatol. 2019 Feb; 139(2): 485-488. doi: 10.1016/j.jid.2018.07.036).

Anti-CCR6 neutralizing antibodies have also shown efficacy in Aldara induced ear swelling in mice (Robert R, JCI Insight. 2017 Aug 3; 2(15): e94821. Published online 2017 Aug 3. doi: 10.1172/jci.insight.94821). An engineered disulfide-linked CCL20 dimer, which binds CCR6 but inhibits T cell migration, was shown to reduce skin swelling in an IL-23-dependent mouse model of psoriasis (Getschman AE, Proc Natl Acad Sci U S A. 2017 Nov 21;114(47):12460-12465. doi: 10.1073/pnas.1704958114). Collectively, these data show that a positive feedback consisting of epidermal and dermal production of CCL20, potent recruitment of CCR6+ T cells or into inflamed psoriatic skin, their activation by IL-23 and their expression of IL-17A and IL-22, drives a pathogenic Th17 response in psoriatic skin lesions. Inhibition of CCR6 has therefore been recognized as a potential therapeutic pathway to treat psoriasis (Hedrick MN, Expert Opin Ther Targets. 2010 Sep;14(9):911-22. doi: 10.1517/14728222.2010.504716; Mabuchi T, J Dermatol Sci. 2012 Jan;65(1):4-11. doi: 10.1016/j.jdermsci.2011.11.007). CCR6 expression was shown to be upregulated in synovial membranes of psoriatic arthritis (PsA) patients (Dolcino M, PLoS One. 2015 Jun 18;10(6):e0128262. doi: 10.1371/journal.pone.0128262). IL-17A- and GM-CSF-expressing CD4+ T cells isolated from synovial fluid of PsA patients also expressed CCR6 (Al-Mossawi et al., Nat Commun. 2017 Nov 15;8(1):1510. doi: 10.1038/s41467-017-01771-2). CCL20 was shown to be highly upregulated in synovial fluid retrieved from PsA patients (Melis L, Ann Rheum Dis. 2010 Mar;69(3):618-23. doi: 10.1136/ard.2009.107649).

Additional inflammatory skin disorders including rosacea have been shown to have highly elevated levels of CCL20 in inflamed skin (Buhl T, JID, 2015).

CCR6 and CCL20 are highly elevated in active Crohn's disease (CD) and ulcerative colitis (UC) (Skovdahl et al., PLoS One. 2015 Nov 4;10(11):e0141710. doi: 10.1371/journal.pone.0141710). Increased enterocyte CCL20 production has been proposed to play an important role in lymphocyte recruitment to the colonic epithelium in irritable bowel disease (IBD) (Kwon JH, Gut. 2002 Dec; 51(6):818-26. doi: 10.1136/gut.51.6.818). CCL20 and CCR6 expression are also correlated with histological severity in rectum resected from UC patients. CCL20 expression in chronic UC is higher than that in acute UC after pathological examination (Uchida K, Gastroenterol Res Pract. 2015; 2015:856532. doi: 10.1155/2015/856532). Expression of CCL20 was significantly up-regulated in the PBMCs of patients with UC compared with those of normal healthy controls. UC groups treated with sulfasalazine and GC showed decreases of CCL20 expression in PBMCs, accompanied by ameliorated disease. TNFα or IL-1β-induced CCL20 secretion was strongly reduced by sulfasalazine and/or GC treatment of human intestinal epithelial cell lines (Lee HJ, 2 Inflamm Bowel Dis. 2005 Dec; 11(12):1070-9. doi: 10.1097/01.mib.0000187576.26043.ac). CCR6 deficiency resulted in reduced intestinal pathology in mice treated with dextran sodium sulfate (DSS) to induce colonic inflammation (Varona R, Eur J Immunol. 2003 Oct; 33(10):2937-46. doi: 10.1002/eji.200324347).

Th17 cells expressing CCR6 were shown to be important effectors mediating dry eye disease (DED), an inflammatory state at the ocular surface, potentially resulting in corneal perforation. Antibody-mediated neutralization of CCL20 in a DED mouse model reduced Th17 recruitment into the ocular surface, resulting in improved clinical readouts (Dohlman TH, Invest Ophthalmol Vis Sci. 2013 Jun 12; 54(6):4081-91. doi: 10.1167/iovs.12-11216). Inhibition of the CCR6/CCL20 axis was therefore proposed as a therapeutic mechanism to treat DED.

CCR6 expression has been described on T cells isolated from the cerebrospinal fluid of multiple sclerosis (MS) patients (van Langelaar J, Brain, 2018 May 1; 141(5):1334-1349. doi: 10.1093/brain/awy069). CCR6 expression was also shown on T cells infiltrating the inflamed CNS in experimental autoimmune encephalomyelitis (EAE) (Mony JT, Front Cell Neurosci. 2014; 8:187. doi: 10.3389/fncel.2014.00187). Furthermore, CCL20 gene polymorphisms have been shown to be associated with MS patient cohorts (EI Sharkav et al., Gene. 2019 Feb 15; 685:164-169. doi: 10.1016/j.gene.2018.11.006). Preclinical data has shown that CCR6 is important for development of EAE (Reboldi A, Nat Immunol. 2009 May; 10(5):514-23. doi: 10.1038/ni.1716). This finding was confirmed in later study, showing that CCR6-deficient mice were resistant to disease induction with reduced peak severity. In the same study, vaccination with hCCL20 produced an anti-mouse CCL20 response in the host mice, which was sufficient to reduce clinical scores (Abraham M, Clin Immunol. 2017 Oct; 183:316-324. doi: 10.1016/j.clim.2017.09.018). However, conflicting data exists concerning the role for CCR6 in EAE development (J Neuroimmunol. 2009 Aug 18; 213(1-2):91-9. doi: 10.1016/j.jneuroim.2009.05.011). EAE severity and histopathology were significantly reduced after injection of anti-CCL20 upon first clinical manifestations (Kohler RE, J Immunol. 2003 Jun 15; 170(12):6298-306. doi: 10.4049/jimmunol.170.12.6298). Anti-CCR6 neutralizing antibodies were shown to reduce the severity of EAE in mice (Robert R, JCI Insight. 2017 Aug 3; 2(15): e94821. Published online 2017 Aug 3. doi: 10.1172/jci.insight.94821). IL-6 and IL-17 increase the expression of CCL20 from murine astrocytes (Meares GP, Glia. 2012 May; 60(5):771-81. doi: 10.1002/glia.22307).

CCR6 and CCL20 are proposed to influence kinetics of germinal center (GC) formation and B cell responses and CCR6 is considered a marker memory B cell precursors in both mouse and human germinal centers (Suan D, Immunity. 2017 Dec 19; 47(6):1142-1153.e4. doi: 10.1016/j.immuni.2017.11.022). Expression of CCR6 on naive, pre-GC, GC/plasma cell and memory B cells in peripheral B cells of systemic lupus erythematosus (SLE) patients was increased (Lee AYS, Clin Rheumatol. 2017 Jun; 36(6):1453-1456. doi: 10.1007/s10067-017-3652-3). CD4+CCR6+ cells may also contribute to disease severity in SLE patients and were shown to be increased in anti-DNA+ SLE patients, which correlated with disease severity and erythrocyte sedimentation rate (Zhong W, PeerJ. 2018; 6:e4294. doi: 10.7717/peerj.4294). Increased CCR6 expression in the salivary glands of patients with primary Sjögren's syndrome (pSS) was demonstrated [Scand J Immunol. 2020 Mar;91(3):e12852. doi: 10.1111/sji.12852]. A trend towards increased CCL20 mRNA expression was also observed. Significant reductions in CCR6+ Th cells (both CCR9- and CCR9+) in the circulation of patients with pSS as compared with healthy controls (HCs) were demonstrated [Scand J Immunol. 2020 Mar;91(3):e12852. doi: 10.1111/sji.12852].

In an animal model of autoimmune hepatitis (AIH), administering anti-TNF-a suppressed hepatic CCL20 expression. Mice receiving anti-CCL20 showed reduced AIH. Furthermore, TNFα stimulation enhanced CCL20 expression in hepatocytes. These findings suggest that TNFα is essential in the induction of AIH through upregulation of hepatic CCL20 expression, which recruits CCR6+ T cells which drive pathology (Clin Immunol. 2013 Jan; 146(1):15-25. doi: 10.1016/j.clim.2012.10.008).

The present CCR6 modulators may be useful, alone, or in combination in the treatment or prevention of autoimmune diseases or disorders including Posterior uveitis, allergic conjunctivitis, allergic disease in the gastrointestinal tract, type I diabetes and endometriosis (Medicina (Kaunas). 2018 Nov 16; 54(5). doi: 10.3390/medicina54050088). CCR6 modulators may also be useful, alone or in combination, to treat diseases of the ocular surface in which elevated levels of IL-17A have been recorded, including meibomian gland dysfunction; GVHD, graft-versus host disease; autoimmune keratitis, filamentary keratitis, dry eye syndrome with rheumatic arthritis; dry eye syndrome without systemic disease; Stevens-Johnson syndrome. (J Korean Med Sci. 2011 Jul;26(7):938-44. doi: 10.3346/jkms.2011.26.7.938).

The present CCR6 modulators may be useful, alone, or in combination in the treatment or prevention of malignant diseases. Modulation of the CCR6/CCL20 axis using siRNA, shRNA, CCR6 knock-out animals, CCL20 ligand treatment or antibodies has been shown to alter tumor growth and metastatic processes in experimental disease models as single agents, or in combination with immunotherapy (such as especially PD1 and/or PDL1 blockade) for the prevention / prophylaxis or treatment of cancers.

The therapeutic potential of modulating this axis for the treatment of malignancies has been described in tumor mouse models using small interfering RNA (siRNA) or small hairpin RNA (shRNA)-mediated silencing of CCR6 or CCL20. Specifically, in a mouse model of cutaneous T cell lymphoma (My-La cells), Abe et al. reported that the administration of a CCR6-targeted siRNA prolonged survival of animals when compared with control animals (Oncotarget. 2017 Jan 31; 8(5):7572-7585. doi: 10.18632/oncotarget.13810.). Using another approach, Ito and colleagues demonstrated that mice, injected with T lymphoma cells (My-La) harboring a CCR6 silencing siRNA construct, survived significantly longer than mice injected with control cells (Blood. 2014 Mar 6; 123(10):1499-511. doi: 10.1182/blood-2013-09-527739.). Zhu and co-workers demonstrated that, the average volume and weight of tumor nodules in mice injected subcutaneously with a set of colorectal cancer cell lines was decreased when CCR6 was silenced in the cancer cells by means of shRNA (PMID Biochim Biophys Acta Mol Basis Dis. 2018 Feb; 1864(2):387-397. doi: 10.1016/j.bbadis.2017.10.033.). In glioblastoma xenograft models using patient-derived glioblastoma cell lines, mice injected with cells harboring a shRNA construct silencing CCR6 expression survived longer than those injected with control cells. In addition, histology and immunohistochemistry revealed that tumors formed by glioma cells with CCR6-targeting shRNA were much smaller, and tumor vessel formation was significantly lower versus control tumors. Collectively, these data further support the notion that CCR6 signaling enhances the oncogenic potential of malignancies including lymphoma, colorectal tumors and glioblastoma (Oncogene. 2018 Jun; 37(23):3070-3087. doi: 10.1038/s41388-018-0182-7.). Specifically, the implication of the CCR6/CCL20 axis in tumorigenesis using CCR6 knock-out animals was reported in the literature. In the CMT93 mouse model of colorectal cancer (CRC), the infiltration of T regulatory cells was completely prevented in tumors of mice deficient in CCR6 in comparison to wildtype animals. The reported data further suggest that the homing and trafficking of tumor-infiltrating T regulatory cells to the tumor mass is dependent on the chemokine receptor CCR6 in vivo (PLoS One. 2011 Apr 29; 6(4):e19495. doi: 10.1371/journal.pone.0019495.). According to Nandi and colleagues, in a mouse model of spontaneous intestinal tumorigenesis [APCMIN/+ mice, heterozygous for a mutation in the adenomatous polyposis coli (APC) gene], mice deficient in CCR6 had a lower occurrence of spontaneous intestinal tumorigenesis (PLoS One. 2014; 9(5):e97566. doi: 10.1371/journal.pone.0097566.).

The potential role of the CCR6/CCL20 axis in tumorigenesis was also demonstrated by administrating the recombinant CCL20 chemokine. Specifically, in a mouse model of colorectal cancer (CMT93 cells), Liu and colleagues showed that tumor size was significantly increased in mice treated with recombinant mouse CCL20 compared with PBS controls, suggesting a critical role for CCL20 in colorectal cancer growth and development (PLoS One. 2011 Apr 29; 6(4):e19495. doi: 10.1371/journal.pone.0019495.).

Specifically, using neutralizing CCL20 antibodies, the potential role of the CCR6/CCL20 axis in tumor promotion was demonstrated in the literature using mouse models. Ikeda and co-workers used a specific cutaneous T cell lymphoma (CTCL) mouse model in which animals succumb to metastasis of CTCL cells into multiple organs. However, administration of a neutralizing CCL20 antibody significantly prolonged the survival of the xenografted mice (Oncotarget. 2016 Mar 22; 7(12):13563-74. doi: 10.18632/oncotarget.6916.). Lee and co-workers described in a mouse model of metastatic breast cancer (MDA-MB-231 cells were injected into the left cardiac ventricles of nude mice) that the administration of an anti-CCL20 antibody prevented the development of bone metastasis, one of the major site of breast cancer metastasis in human disease (Sci Rep. 2017 Aug 29; 7(1):9610. doi: 10.1038/s41598-017-09040-4.). In a humanized mouse model of nasopharyngeal carcinoma, Mrizak et al. observed a significant decrease of T regulatory cell recruitment into the tumor when mice were injected with anti-CCL20 monoclinal antibody in comparison to sham treated animals (J Natl Cancer Inst. 2015 Jan; 107(1):363. doi: 10.1093/jnci/dju363.). In addition, in a mouse model of hepatocarcinoma (Hepa1-6 cells), blockade of CCL20 activity in immunocompetent mice using an anti-CCL20 antibody, attenuated tumor incidence, restrained tumor growth and distal metastasis. Moreover, the authors reported that in this mouse model, tumor angiogenesis was significantly inhibited upon CCL20 neutralization. (He at al., PMID 28560063 - Am J Cancer Res. 2017; 7(5):1151-1163.). Using the same mouse model, the administration of the anti-CCL20 neutralizing antibody remarkably reduced the infiltration of T regulatory cells into the tumor, especially CCR6 positive T regulatory cells, and significantly decreased tumor growth. Antitumor efficacy was further enhanced when the mice were co-treated with an anti-PDL-1 antibody. Collectively these data sets suggest that CCL20 blockade could abrogate anti-PD-L1 resistance in a mouse model of hepatocarcinoma by inhibiting T regulatory recruitment to the tumor (Hepatology. 2019 Jul; 70(1):198-214. doi: 10.1002/hep.30593.).

Specifically, the potential role of the CCR6/CCL20 axis in tumor metastasis was described in the literature. Dellacasagrande and colleagues reported that, in a mouse model of plasmacytoma, tumor cells that disseminated to the liver overexpressed functional CCR6 in comparison with tumor cells of the primary tumor (from s.c. injection of mouse plasmacytoma (MOPC315)). The same authors found that CCR6 was overexpressed in small liver metastases of colon, thyroid and ovarian carcinomas compared with normal liver (Scand J Immunol. 2003 Jun; 57(6):534-44. doi: 10.1046/j.1365-3083.2003.01263.x.).

Furthermore, the present CCR6 modulators may be useful, alone, or in combination in the treatment or prevention of cancers where the expression of CCR6 and/or CCL20 correlates with disease progression and resistance to standard treatment care. Specifically, the correlation of CCR6 expression with disease progression was described in the literature for numerous cancer indications. For example, in renal cell carcinoma CCR6 expression is associated with a lower overall survival (Cancers (Basel). 2019 Dec 30; 12(1). doi: 10.3390/cancers12010089.). In colorectal cancer, tumor expression of CCR6 positively correlates with metastasis and upregulated CCR6 predicts poor survival, shorter disease-free survival (PLoS One. 2014; 9(6):e101137. doi: 10.1371/journal.pone.0101137.), and poorer 5-year overall survival (Biochim Biophys Acta Mol Basis Dis. 2018 Feb; 1864(2):387-397. doi: 10.1016/j.bbadis.2017.10.033.). In ovarian cancer high CCR6 mRNA expression was also associated with a worse prognosis (Cancer Lett. 2020 Mar 1; 472:59-69. doi: 10.1016/j.canlet.2019.12.024.). CCR6 expression was associated with rectal cancer aggressiveness, indeed, high-level expression of CCR6 protein was more common in non-responders to radiotherapy than in responders (Cancer Res Treat. 2018 Oct; 50(4):1203-1213. doi: 10.4143/crt.2017.538.). The expression level of CCR6 in prostate cancer was associated with clinical and pathologic features of more advanced and aggressive disease (J Cancer Res Clin Oncol. 2008 Nov; 134(11):1181-9. doi: 10.1007/s00432-008-0403-5.). In non-small cell lung cancer (NSCLC) high CCR6 expression was associated with shorter disease-free survival and conferred a disease stage-independent 5-fold increased risk for disease recurrence (PLoS One. 2011; 6(9):e24856. doi: 10.1371/journal.pone.0024856.). Hepatocarcinoma patients with increased infiltrated CCR6 positive immune cells in tumor tissues showed a poorer prognosis (Am J Cancer Res. 2017; 7(5):1151-1163.).

Analogous to CCR6, expression of its ligand CCL20 has been reported to correlate with poorer disease outcome for several indications. Specifically, in breast cancer, elevated CCL20 expression significantly correlated with lower overall free survival, lower percent metastasis free survival (Sci Rep. 2017 Aug 29; 7(1):9610. doi: 10.1038/s41598-017-09040-4.), with higher histological grade, higher Ki67 index, and axillary lymph node metastases. Moreover, breast tumor CCL20 expression positively correlated with expression of FOXP3, a marker of T regulatory cells. Patients with axillary lymph node metastases, and concomitant elevation in CCL20 expression and FOXP3-positive T regulatory cells, had the worst overall survival. (Medicine (Baltimore). 2019 Dec; 98(50):e18403. doi: 10.1097/MD.0000000000018403.). In NSCLC higher expression of CCL20 was associated with a lower overall survival (Biomed Pharmacother. 2015 Feb; 69:242-8. doi: 10.1016/j.biopha.2014.12.008.)(Cancer Lett. 2015 Jul 10; 363(1):60-70. doi: 10.1016/j.canlet.2015.04.005.). Analogous to NSCLC, hepatocellular carcinoma patients with high CCL20 expression had poorer overall survival and poorer recurrence-free survival. The same authors described that CCL20 expression was significantly associated with tumor size, tumor number, vascular invasion, tumor differentiation and tumor recurrence (J Gastrointest Surg. 2012 Apr; 16(4):828-36. doi: 10.1007/s11605-011-1775-4.). In addition to CCR6 or CCL20 alone, correlation of CCR6/CCL20 co-expression with disease progression is stated in literature. Indeed, overexpression of both, CCL20 and CCR6, was detected in high-grade glioma tissues as compared to low-grade tissues and increased with ascending tumor World Health Organization (WHO) grades. Particularly glioma patients with CCL20/CCR6 co-expression had the shortest overall survival (Med Oncol. 2012 Dec; 29(5):3491-7. doi: 10.1007/s12032-012-0314-9.).

Besides, CCR6 and/or CCL20 expression correlates with enhance chemotherapeutic resistance and is associated with metastasis. Indeed, CCL20 expression can increase the chemotherapeutic resistance of breast cancer cells (PLoS Biol. 2018 Jul; 16(7):e2005869. doi: 10.1371/journal.pbio.2005869.). Rubie and colleagues describe that in colorectal liver metastases (CRLM) and in human samples of hepatocellular carcinoma (HCC), significant up-regulation of CCL20/CCR6 was detected (RT-PCR). Moreover, CCL20 was significantly overexpression in colorectal liver metastases as compared to the primary HCC, indicating an involvement of the CCL20/CCR6 ligand-receptor pair in the carcinogenesis and progression of hepatic malignancies (World J Gastroenterol. 2006 Nov 7; 12(41):6627-33. doi: 10.3748/wjg.v12.i41.6627.).

The present CCR6 modulators may be useful, alone, or in combination in the treatment or prevention of diseases or disorders where CCR6 and/or CCL20 are expressed or overexpressed in patient samples or cancer cell lines. Specifically, the chemokine receptor CCR6 is described to be expressed in several cancer types or cancer cell lines in the literature. Lu and coworkers describe that CCR6 expression was higher in laryngeal cancer tissues compared with their normal controls. The authors reported that CCR6 was also expressed in commonly used laryngeal cancer cells such as TU212, M4E, M2E and Hep-2 (Biomed Pharmacother. 2017 Jan; 85:486-492 doi: 10.1016/j.biopha.2016.11.055.). Based on gene expression data from malignant melanoma, among the biological networks reported CCR6 gene was described and characterized as a valuable factor involved in immune responses and tumor progression (PLoS One. 2018; 13(1):e0190447. doi: 10.1371/journal.pone.0190447.) Whole exome sequencing in 21 MALT lymphomas of the salivary gland and thyroid revealed that CCR6 was expressed (Haematologica. 2018 Aug; 103(8):1329-1336. doi: 10.3324/haematol.2018.191601.). In samples of adult human T-cell leukemia / lymphoma (ATLL) transcripts of CCR6 were detected, and CCR6 was further found at the protein level using flow cytometric analysis (Leuk Lymphoma. 2006 Oct; 47(10):2163-73. doi: 10.1080/10428190600775599.). In patient-derived prostate cancer samples the gene expression of CCR6 (mRNA) was significantly higher in tumor tissue as compared to adjacent normal tissue (Cancer Res Treat. 2015 Apr; 47(2):306-12. doi: 10.4143/crt.2014.015.). CCR6 expression was detected in commonly used cancer cell lines, indeed, according to Mays and co-workers, in salivary adenoid cystic carcinoma cells SACC-83, among other CC chemokine receptors, CCR6 was expressed using RT-PCR gene analysis (Anticancer Res. 2016 Aug; 36(8):4013-8.). According to Möller and colleagues, in multiple myeloma (MM) cell lines including U266 1970, U-266 1984, U-1958, Karpas 707, LP-1,28 L-363, HL407E and HL407L.3, CCR6 was also expressed (Leukemia. 2003 Jan; 17(1):203-10. doi: 10.1038/sj.leu.2402717.).

Analogous to CCR6, the ligand CCL20 was reported to be expressed in multiple tumor samples and tumor cell lines in the literature. For example, Zhang and co-workers demonstrated that in samples from NSCLC patients, using RT-PCR, CCL20 showed higher expression in tumor samples than in samples of adjacent tissue, this was also verified at the protein level using immunohistochemistry (Biomed Pharmacother. 2015 Feb; 69:242-8. doi: 10.1016/j.biopha.2014.12.008.). Gene expression analysis of cholangiocarcinoma samples and corresponding normal tissue revealed CCL20 to be one of the genes most significantly over-expressed in malignant vs healthy tissue (EXCLI J. 2020; 19:154-166. doi: 10.17179/excli2019-1893.). CCL20 expression was also reported in multiple myeloma (MM) human samples (Cancer Res. 2008 Aug 15; 68(16):6840-50. doi: 10.1158/0008-5472.CAN-08-0402.). Besides, according to Rubies et al., CCL20 mRNA and protein was significantly up-regulated in pancreatic carcinoma (8-fold) as compared to matched normal pancreas in which CCL20 was weakly expressed (J Transl Med. 2010 May 10; 8:45. doi: 10.1186/1479-5876-8-45.)..). CCL20 is also expressed in oral squamous cell carcinoma (IHC staining) and Lee et al. reported that expression is enriched in human CCR6+ regulatory T cells with superior suppressive activity (J Immunol. 2017 Jul 15; 199(2):467-476. doi: 10.4049/jimmunol.1601815.).

In addition to CCR6 or CCL20 alone, the co-expression of both CCR6 and CCL20 is reported for samples of cancer patients and cancer cells lines in literature. Both genes have been described to be expressed in adult T-cell leukemia/lymphoma patient samples (Microarray and IHC protein staining) (Int J Oncol. 2014 Sep; 45(3):1200-8. doi: 10.3892/ijo.2014.2524.) and in CTCL. In the latter, CCL20 and CCR6 were detected at the mRNA and protein levels (Clin Cancer Res. 2011 Dec 15; 17(24):7529-38. doi: 10.1158/1078-0432.CCR-11-1192.). Transcriptomic analysis (nanostring) of samples of hepatocellular carcinoma revealed CCR6 and CCL20 expression. Moreover, a chemotactic gradient between non-tumor and tumor tissues was reported and a recruitment process of T regulatory cells, tumor associated macrophages and natural killer cells involving the CCR6/CCL20 axis suggested (Proc Natl Acad Sci U S A. 2017 Jul 18; 114(29):E5900-E5909. doi: 10.1073/pnas.1706559114.). Similarly, Guo and co-workers reported CCR6 and CCL20 upregulation in hepatocarcinoma lesions compared to healthy tissue as well as CCR6 and CCL20 expression in hepatocarcinoma cell lines (L02, Li-/, Huh-7, SNU-387, Hep3B) (Oncol Rep. 2019 Sep; 42(3):1075-1089. doi: 10.3892/or.2019.7221.). In human colorectal cancer, both CCL20 and CCR6 are expressed according to Nandi et al. (IHC protein staining). Both, CCR6 and CCL20, were found to be highly expressed in samples of NSCLC (protein and mRNA) (Oncol Lett. 2017 Dec; 14(6):8183-8189. doi: 10.3892/ol.2017.7253). Using in situ hybridization, both CCL20 and CCR6 mRNA moieties were strongly expressed in all pancreatic cancer samples analysed. In contrast, in healthy pancreas CCL20 and CCR6 expression was low (Int J Cancer. 1999 May 17; 81(4):650-7. doi: 10.1002/(sici)1097-0215(19990517)81:4<650::aid-ijc23>3.0.co;2-#.). Jin and co-workers examined CCR6 and CCL20 expression in glioblastoma using publicly available datasets. The authors used the GEO dataset GSE2223 to compare the mRNA levels of CCL20 and CCR6, between normal brain and glioblastoma tissues. Again, CCR6 and CCL20 expression levels were significantly higher in glioblastoma tissues than in normal brain tissues (Oncogene. 2018 Jun; 37(23):3070-3087. doi: 10.1038/s41388-018-0182-7.). In addition, Wallace and colleagues observed that in endometrial adenocarcinoma explants and cell lines, expression of CCL20 and its receptor CCR6 were higher compared to non-malignant endometrium (mRNA, RT-PCR) (Mol Cell Endocrinol. 2011 Jan 1; 331(1):129-35. doi: 10.1016/j.mce.2010.08.018.). CCL20/CCR6 axis may play a role in breast cancer, cholangiocarcinoma, and thyroid cancer since expression of CCR6/CCL20 genes and/or proteins was reported in patient derived breast cancer cells (Mol Carcinog. 2016 Jul; 55(7):1175-86. doi: 10.1002/mc.22360.), in HuCCT1 and TFK-1 cholangiocarcinoma cell lines () (Win et al., PMID 32194362) (EXCLI J. 2020; 19:154-166. doi: 10.17179/excli2019-1893.) and thyroid cancer cell lines such as TPC-1, BCPAP, FTC-133, and SW1736 (Tumour Biol. 2016 Apr; 37(4):5569-75. doi: 10.1007/s13277-015-4418-7.). Furthermore, the present CCR6 modulators may be useful, alone, or in combination in the treatment or prevention of cancers where the expression and/or evidence of CCR6/CCL20 axis activity has been reported, or where CCR6+ regulatory T cells have been identified inside the tumor microenvironment.
1) One aspect of the present invention relates to compounds of Formula (I) 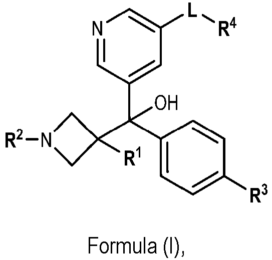 wherein
   - **R¹** represents C₁₋₃-alkyl (especially methyl);
      **R²** represents C₁₋₄-alkyl (especially methyl);
      **R³** represents 2,2,2-trifluoro-ethyl;
      **-L-** represents
         ➢ *-C≡C-C₀₋₂-alkylene-**, optionally mono-substituted with hydroxy (especially -C=C- or -C≡C-C(CH₃)(OH)-), wherein the asterisk (*) denotes the attachment point to the pyridinyl ring and two asterisks (**) denote the attachment point to **R⁴**); or
         ➢ oxadiazol-diyl (notably 1,2,4-oxadiazol-diyl; especially 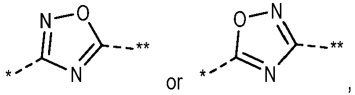 wherein one asterisk (*) denotes the attachment point to the pyridinyl ring and two asterisks (**) denote the attachment point to **R⁴**); and
      **R⁴** represents
         ➢ C₃₋₇-cycloalkyl (especially cyclohexyl);
         ➢ saturated 5- to 8-membered bridged or spiro bicyclic hydrocarbon ring system, wherein one ring carbon atom (especially in said spiro bicyclic hydrocarbon ring system) is optionally replaced with a nitrogen atom (especially such bridged or spiro bicyclic hydrocarbon ring system represents bicyclo[2.2.2]octan-1-yl, bicyclo[1.1.1]pentan-1-yl, or 2-azaspiro[3.3]heptan-6-yl); or
         ➢ 6-membered heteroaryl containing one or two (especially two) ring nitrogen atoms (notably pyrimidinyl; especially pyrimidin-4-yl);
      wherein **R⁴** independently is unsubstituted or mono-substituted with hydroxy, hydroxy-C₁₋₃-alkyl (especially hydroxy-methyl), carbamoyl, C₁₋₃-alkyl-carbonyl-amino (especially acetyl-amino or ethyl-carbonyl-amino), or C₁₋₃-alkyl-carbonyl (especially acetyl)
      [in particular, the group -**L-R⁴** represents 5-(4-hydroxy-cyclohexyl)-1,2,4-oxadiazol-3-yl, 5-(3-hydroxymethyl-bicyclo[1.1.1]pentan-1-yl)-1,2,4-oxadiazol-3-yl, 3-(3-hydroxymethyl-bicyclo[1.1.1]pentan-1-yl)-1,2,4-oxadiazol-5-yl, 2-(4-hydroxy-cyclohexyl)-ethyn-1-yl, 3-hydroxy-3-(pyrimidin-4-yl)-but-1-yn-1-yl, 5-(4-carbamoyl-cyclohexyl)-1,2,4-oxadiazol-3-yl, 5-(4-hydroxy-bicyclo[2.2.2]octan-1-yl)-1,2,4-oxadiazol-3-yl, 5-(3-acetamido-bicyclo[1.1.1]pentan-1-yl)-1,2,4-oxadiazol-3-yl, 5-(2-acetyl-2-azaspiro[3.3]heptan-6-yl)-1,2,4-oxadiazole-3-yl, 5-(4-acetamido-cyclohexyl)-1,2,4-oxadiazole-3-yl, 5-(4-(ethyl-carbonyl-amino)-cyclohexyl)-1,2,4-oxadiazole-3-yl, or 5-(4-carbamoyl-bicyclo[2.2.2]octan-1-yl)-1,2,4-oxadiazol-3-yl]; or
   - **R¹** represents C₁₋₃-alkyl (especially methyl);
      **R²** represents C₁₋₄-alkyl (especially methyl);
      **R³** represents isopropyl; and
      -**L-R⁴** represents 2-(4-hydroxy-cyclohexyl)-ethyn-1-yl, 3-hydroxy-3-(pyrimidin-4-yl)-but-1-yn-1-yl, 5-(4-carbamoyl-cyclohexyl)-1,2,4-oxadiazol-3-yl, 5-(4-hydroxy-bicyclo[2.2.2]octan-1-yl)-1,2,4-oxadiazol-3-yl, 5-(3-acetamido-bicyclo[1.1.1]pentan-1-yl)-1,2,4-oxadiazol-3-yl, 5-(2-acetyl-2-azaspiro[3.3]heptan-6-yl)-1,2,4-oxadiazole-3-yl, 5-(4-acetamido-cyclohexyl)-1,2,4-oxadiazole-3-yl, 5-(4-(ethyl-carbonyl-amino)-cyclohexyl)-1,2,4-oxadiazole-3-yl, or 5-(4-carbamoyl-bicyclo[2.2.2]octan-1-yl)-1,2,4-oxadiazol-3-yl; or
   - **R¹** represents C₁₋₃-alkyl (especially methyl);
      **R²** represents C₁₋₄-alkyl (especially methyl);
      **R³** represents 2,2,2-trifluoro-ethyl; and
      -**L-R⁴** represents 3-(2-hydroxy-1,1-dimethyl-ethyl)-1,2,4-oxadiazol-5-yl.

Definitions provided herein are intended to apply uniformly to the compounds of Formula (I) as defined in any one of embodiments 1) to 9), and, *mutatis mutandis,* throughout the description and the claims unless an otherwise expressly set out definition provides a broader or narrower definition. It is well understood that a definition or preferred definition of a term defines and may replace the respective term independently of (and in combination with) any definition or preferred definition of any or all other terms as defined herein. If not explicitly defined otherwise in the respective embodiment or claim, groups defined herein are unsubstituted.

The term "amino", used alone or in combination, refers to the group -NH₂.

The term "carbonyl", used alone or in combination, refers to the group -C(=O)-.

The term "alkyl", used alone or in combination, refers to a saturated straight or branched hydrocarbon chain group containing one to six carbon atoms. The term "C_{x-y}-alkyl" (x and y each being an integer), refers to an alkyl group as defined before, containing x to y carbon atoms. In case a C_{x-y}-alkyl group is used in combination with another substituent, the term means that said substituent is linked through a C_{x-y}-alkyl group to the rest of the molecule. For example, a C₁₋₄-alkyl group contains from one to four carbon atoms. Examples of C₁₋₄-alkyl group is methyl, ethyl, n-propyl, isopropyl, n-butyl, sec-butyl, tert-butyl, and isobutyl. A preferred example of a C₁₋₄-alkyl as used for the substituent **R²** is methyl. A preferred example of a C₁₋₃-alkyl as used for the substituent **R¹** is methyl.

The term "-C_{x-y}-alkylene-", used alone or in combination, refers to bivalently bound alkyl group as defined before containing x to y carbon atoms. Especially, the points of attachment of any bivalently bound alkyl group are in 1,1-diyl arrangement. In case a -C_{0-y}-alkylene- group is used in combination with another substituent, the term means that either said substituent is directly attached to the rest of the molecule (i.e. the -C₀-alkyl- group represents a direct bond linking said substituent to the rest of the molecule), or it is linked through a -C_{1-y}-alkylene- group to the rest of the molecule. An example of -C_{1-y}-alkylene- group is the -C₁₋₂-alkylene- group encompassing methylene, ethylene, and ethan-1,1-diyl. Examples of the group -C≡C-C₀₋₂-alkylene- are -C=C-, -C≡C-CH₂-, -C≡C-CH₂-CH₂-, and -C=C-CH(CH₃)-; especially -C=C- and -C≡C-CH(CH₃)-. When the group -C≡C-C₀₋₂-alkylene- is referred to as mono-substituted with hydroxy, it is understood that said substitution is only possible when a -C₁₋₂-alkylene- is present. Examples of such mono-substituted -C≡C-C₀₋₂-alkylene- groups are -C=C-CH(OH)-, -C≡C-CH(OH)-CH₂-, -C=C-CH₂-CH(OH)-, -C≡C-CH(CH₂OH)-, and -C=C-C(CH₃)(OH)-; especially -C≡C-C(CH₃)(OH)-. One asterisk (*) in the term "*-C≡C-C₀₋₂-alkylene-**"denotes the attachment point to the pyridinyl ring shown in Formula (I) and two asterisks (**) denote the attachment point to **R⁴**.

The term "amino-carbonyl" or "carbamoyl", used alone or in combination, refers to the group NH₂-C(=O)-.

The term "C₁₋₃-alkyl-carbonyl", used alone or in combination, refers to an alkyl group as define before, wherein one hydrogen atom has been replaced by carbonyl group.

The term "C₁₋₃-alkyl-carbonyl-amino", used alone or in combination, refers to an amino group, wherein one hydrogen atom has been replaced by C₁₋₃-alkyl-carbonyl group as defined before.

The term "hydroxyalkyl" (or hydroxy-alkyl), used alone or in combination, refers to an alkyl group as defined before, wherein one hydrogen atom has been replaced by a hydroxy group. The term "hydroxy-C_{x-y}-alkyl" (x and y each being an integer), used alone or in combination, refers to a hydroxyalkyl group as defined before wherein the alkyl group contains x to y carbon atoms. A hydroxy-C₁₋₃-alkyl group is a hydroxyalkyl group as defined before which contains from one to three carbon atoms. Examples of hydroxy-C₁₋₃-alkyl groups are hydroxy-methyl, 1-hydroxy-ethyl, 2-hydroxy-ethyl, 1-hydroxy-propyl, 2-hydroxy-propyl, 3-hydroxy-propyl, and 1-hydroxy-1-methyl-ethyl.

The term "cycloalkyl", used alone or in combination, refers to a saturated monocyclic hydrocarbon ring containing three to seven carbon atoms (preferably three to six carbon atoms). The term "C_{x-y}-cycloalkyl" (x and y each being an integer), refers to a saturated monocyclic hydrocarbon ring containing x to y carbon atoms. For example, a C₃₋₇-cycloalkyl group contains from three to seven carbon atoms. Examples of C₃₋₇-cycloalkyl groups are cyclopropyl, cyclobutyl, cyclopentyl cyclohexyl, and cycloheptyl; especially cyclohexyl. The above-mentioned groups are unsubstituted or substituted as explicitly defined.

The term "6-membered heteroaryl", used alone or in combination, refers to a 6-membered monocyclic aromatic ring containing one or two (especially two) ring nitrogen atoms. Examples are pyridinyl, pyrimidinyl, pyridazinyl, or pyrazinyl; especially pyrimidinyl. The above-mentioned heteroaryl groups are unsubstituted or substituted as explicitly defined. The term "saturated 5- to 8-membered bridged or spiro bicyclic hydrocarbon ring system" refers to two hydrocarbon rings which have one or two carbon atom(s) in common and wherein the total number of carbon atoms in both rings is an integer from 5 to 8. More particularly,
- the term saturated 5- to 8-membered bridged bicyclic hydrocarbon ring system refers to compounds described by the term "bicyclo[x.y.z]alkyl, wherein the total number of carbon atoms is an integer from 5 to 8, and each one of "x", "y" and "z" is larger than 0 [i.e. the sum of "x", "y" and "z" is from 3 to 6; and the integers "x", "y" and "z" independently indicate the number of carbon atoms in each of the three bridges linked to the two tertiary carbon atoms in descending order (x>y>z)]. Examples for such 5- to 8-membered bridged bicyclic hydrocarbon ring system are bicyclo[1.1.1]pentanyl, bicyclo[2.2.1]heptanyl, bicyclo[2.2.2]octanyl, bicyclo[3.1.1]heptanyl, and bicyclo[3.2.1]octanyl; especially bicyclo[2.2.2]octan-1-yl and bicyclo[1.1.1]pentan-1-yl; and
- the term saturated 5- to 8-membered spiro bicyclic hydrocarbon ring system refers to compounds described by the term "spiro[x.y]alkyl", wherein the total number of carbon atoms is an integer from 5 to 8; [it being understood that the integers "x" and "y" represent the number of carbon atoms in each of the two carbon cycles linked to the one quaternary carbon atom]. For example, the following combinations [x.y] are possible [2.2], [2.3], [3.3], [3.4], and [3.5]. Examples for such 5- to 8-membered spiro bicyclic hydrocarbon ring systems are spiro[2.2]pentanyl, spiro[2.3]hexanyl, spiro[3.3]heptanyl, and spiro[3.4]octanyl; especially spiro[3.3]heptanyl.

A bond drawn as a dotted line shows the point of attachment of the radical drawn to the rest of the molecule. For example, the radical drawn below 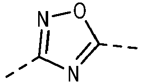 represents 1,2,4-oxadiazol-diyl.

Further embodiments of the invention are presented hereinafter:
2) One embodiment relates to compounds according to embodiment 1), wherein
   - **R¹** represents C₁₋₃-alkyl (especially methyl);
      **R²** represents C₁₋₄-alkyl (especially methyl);
      **R³** represents 2,2,2-trifluoro-ethyl;
      -L- represents
         ➢ *-C≡C-C₀₋₂-alkylene-**, optionally mono-substituted with hydroxy (especially -C=C- or -C≡C-C(CH₃)(OH)-), wherein the asterisk (*) denotes the attachment point to the pyridinyl ring and two asterisks (**) denote the attachment point to **R⁴**); or
         ➢ oxadiazol-diyl (notably 1,2,4-oxadiazol-diyl; especially 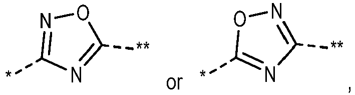 wherein one asterisk (*) denotes the attachment point to the pyridinyl ring and two asterisks (**) denote the attachment point to **R⁴**); and
      **R⁴** represents
         ➢ mono-substituted C₃₋₇-cycloalkyl (especially cyclohexyl), wherein the substituent is selected from hydroxy, carbamoyl, or C₁₋₃-alkyl-carbonyl-amino (especially acetyl-amino or ethyl-carbonyl-amino);
         ➢ saturated 5- to 8-membered bridged or spiro bicyclic hydrocarbon ring system, wherein one ring carbon atom (especially in said spiro bicyclic hydrocarbon ring system) is optionally replaced with a nitrogen atom (especially such bridged or spiro bicyclic hydrocarbon ring system represents bicyclo[2.2.2]octan-1-yl, bicyclo[1.1.1]pentan-1-yl, or 2-azaspiro[3.3]heptan-6-yl), wherein said 5- to 8-membered bridged or spiro bicyclic hydrocarbon ring system independently is mono-substituted with hydroxy, hydroxy-C₁₋₃-alkyl (especially hydroxy-methyl), carbamoyl, C₁₋₃-alkyl-carbonyl-amino (especially acetyl-amino), or C₁₋₃-alkyl-carbonyl (especially acetyl); or
         ➢ unsubstituted 6-membered heteroaryl containing one or two (especially two) ring nitrogen atoms (notably pyrimidinyl; especially pyrimidin-4-yl);
   - **R¹** represents C₁₋₃-alkyl (especially methyl);
      **R²** represents C₁₋₄-alkyl (especially methyl);
      **R³** represents isopropyl; and
      -**L-R⁴** represents 2-(4-hydroxy-cyclohexyl)-ethyn-1-yl, 3-hydroxy-3-(pyrimidin-4-yl)-but-1-yn-1-yl, 5-(4-carbamoyl-cyclohexyl)-1,2,4-oxadiazol-3-yl, 5-(4-hydroxy-bicyclo[2.2.2]octan-1-yl)-1,2,4-oxadiazol-3-yl, 5-(3-acetamido-bicyclo[1.1.1]pentan-1-yl)-1,2,4-oxadiazol-3-yl, 5-(2-acetyl-2-azaspiro[3.3]heptan-6-yl)-1,2,4-oxadiazole-3-yl, 5-(4-acetamido-cyclohexyl)-1,2,4-oxadiazole-3-yl, 5-(4-(ethyl-carbonyl-amino)-cyclohexyl)-1,2,4-oxadiazole-3-yl, or 5-(4-carbamoyl-bicyclo[2.2.2]octan-1-yl)-1,2,4-oxadiazol-3-yl; or
   - **R¹** represents C₁₋₃-alkyl (especially methyl);
      **R²** represents C₁₋₄-alkyl (especially methyl);
      **R³** represents 2,2,2-trifluoro-ethyl; and
      -**L-R⁴** represents 3-(2-hydroxy-1,1-dimethyl-ethyl)-1,2,4-oxadiazol-5-yl.
3) A further embodiment relates to compounds according to embodiment 1), wherein
   - **R¹** represents C₁₋₃-alkyl (especially methyl);
      **R²** represents C₁₋₄-alkyl (especially methyl);
      **R³** represents 2,2,2-trifluoro-ethyl;
      **-L-** represents
         ➢ *-C≡C-C₀₋₂-alkylene-**, optionally mono-substituted with hydroxy (especially -C=C- or -C≡C-C(CH₃)(OH)-), wherein the asterisk (*) denotes the attachment point to the pyridinyl ring and two asterisks (**) denote the attachment point to **R⁴**); and
      **R⁴** represents
         ➢ mono-substituted C₃₋₇-cycloalkyl (especially cyclohexyl), wherein the substituent is selected from hydroxy, carbamoyl, or C₁₋₃-alkyl-carbonyl-amino (especially acetyl-amino or ethyl-carbonyl-amino);
         ➢ or unsubstituted 6-membered heteroaryl containing one or two (especially two) ring nitrogen atoms (notably pyrimidinyl; especially pyrimidin-4-yl);
   - **R¹** represents C₁₋₃-alkyl (especially methyl);
      **R²** represents C₁₋₄-alkyl (especially methyl);
      **R³** represents 2,2,2-trifluoro-ethyl;
      **-L-** represents
         ➢ oxadiazol-diyl (notably 1,2,4-oxadiazol-diyl; especially 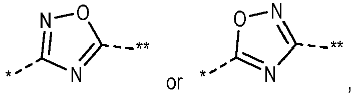 wherein one asterisk (*) denotes the attachment point to the pyridinyl ring and two asterisks (**) denote the attachment point to **R⁴**); and
      **R⁴** represents
         ➢ mono-substituted C₃₋₇-cycloalkyl (especially cyclohexyl), wherein the substituent is selected from hydroxy, carbamoyl, or C₁₋₃-alkyl-carbonyl-amino (especially acetyl-amino or ethyl-carbonyl-amino);
         ➢ or saturated 5- to 8-membered bridged or spiro bicyclic hydrocarbon ring system, wherein one ring carbon atom (especially in said spiro bicyclic hydrocarbon ring system) is optionally replaced with a nitrogen atom (especially such bridged or spiro bicyclic hydrocarbon ring system represents bicyclo[2.2.2]octan-1-yl, bicyclo[1.1.1]pentan-1-yl, or 2-azaspiro[3.3]heptan-6-yl), wherein said 5- to 8-membered bridged or spiro bicyclic hydrocarbon ring system independently is mono-substituted with hydroxy, hydroxy-C₁₋₃-alkyl (especially hydroxy-methyl), carbamoyl, C₁₋₃-alkyl-carbonyl-amino (especially acetyl-amino), or C₁₋₃-alkyl-carbonyl (especially acetyl);
   - **R¹** represents C₁₋₃-alkyl (especially methyl);
      **R²** represents C₁₋₄-alkyl (especially methyl);
      **R³** represents isopropyl; and
      -**L-R⁴** represents 2-(4-hydroxy-cyclohexyl)-ethyn-1-yl, 3-hydroxy-3-(pyrimidin-4-yl)-but-1-yn-1-yl, 5-(4-carbamoyl-cyclohexyl)-1,2,4-oxadiazol-3-yl, 5-(4-hydroxy-bicyclo[2.2.2]octan-1-yl)-1,2,4-oxadiazol-3-yl, 5-(3-acetamido-bicyclo[1.1.1]pentan-1-yl)-1,2,4-oxadiazol-3-yl, 5-(2-acetyl-2-azaspiro[3.3]heptan-6-yl)-1,2,4-oxadiazole-3-yl, 5-(4-acetamido-cyclohexyl)-1,2,4-oxadiazole-3-yl, 5-(4-(ethyl-carbonyl-amino)-cyclohexyl)-1,2,4-oxadiazole-3-yl, or 5-(4-carbamoyl-bicyclo[2.2.2]octan-1-yl)-1,2,4-oxadiazol-3-yl; or
   - **R¹** represents C₁₋₃-alkyl (especially methyl);
      **R²** represents C₁₋₄-alkyl (especially methyl);
      **R³** represents 2,2,2-trifluoro-ethyl; and
      -**L-R⁴** represents 3-(2-hydroxy-1,1-dimethyl-ethyl)-1,2,4-oxadiazol-5-yl.
4) A further embodiment relates to compounds according to embodiment 1), wherein
   - **R¹** represents C₁₋₃-alkyl (especially methyl);
      **R²** represents C₁₋₄-alkyl (especially methyl);
      **R³** represents 2,2,2-trifluoro-ethyl;
      -L- represents
         ➢ *-C≡C-C₀₋₂-alkylene-**, optionally mono-substituted with hydroxy (especially -C=C- or -C≡C-C(CH₃)(OH)-), wherein the asterisk (*) denotes the attachment point to the pyridinyl ring and two asterisks (**) denote the attachment point to **R⁴**); and
      **R⁴** represents
         ➢ mono-substituted C₃₋₇-cycloalkyl (especially cyclohexyl), wherein the substituent is hydroxy; or
         ➢ unsubstituted 6-membered heteroaryl containing one or two (especially two) ring nitrogen atoms (notably pyrimidinyl; especially pyrimidin-4-yl);
   - **R¹** represents C₁₋₃-alkyl (especially methyl);
      **R²** represents C₁₋₄-alkyl (especially methyl);
      **R³** represents 2,2,2-trifluoro-ethyl;
      **-L-** represents
         ➢ oxadiazol-diyl (notably 1,2,4-oxadiazol-diyl; especially 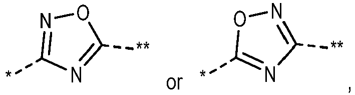 wherein one asterisk (*) denotes the attachment point to the pyridinyl ring and two asterisks (**) denote the attachment point to **R⁴**); and
      **R⁴** represents
         ➢ mono-substituted C₃₋₇-cycloalkyl (especially cyclohexyl), wherein the substituent is selected from hydroxy, carbamoyl, or C₁₋₃-alkyl-carbonyl-amino (especially acetyl-amino or ethyl-carbonyl-amino);
         ➢ or saturated 5- to 8-membered bridged or spiro bicyclic hydrocarbon ring system, wherein one ring carbon atom (especially in said spiro bicyclic hydrocarbon ring system) is optionally replaced with a nitrogen atom (especially such bridged or spiro bicyclic hydrocarbon ring system represents bicyclo[2.2.2]octan-1-yl, bicyclo[1.1.1]pentan-1-yl, or 2-azaspiro[3.3]heptan-6-yl), wherein said 5- to 8-membered bridged or spiro bicyclic hydrocarbon ring system independently is mono-substituted with hydroxy, hydroxy-C₁₋₃-alkyl (especially hydroxy-methyl), carbamoyl, C₁₋₃-alkyl-carbonyl-amino (especially acetyl-amino), or C₁₋₃-alkyl-carbonyl (especially acetyl);
   - **R¹** represents C₁₋₃-alkyl (especially methyl);
      **R²** represents C₁₋₄-alkyl (especially methyl);
      **R³** represents isopropyl; and
      -**L-R⁴** represents 2-(4-hydroxy-cyclohexyl)-ethyn-1-yl, 3-hydroxy-3-(pyrimidin-4-yl)-but-1-yn-1-yl, 5-(4-carbamoyl-cyclohexyl)-1,2,4-oxadiazol-3-yl, 5-(4-hydroxy-bicyclo[2.2.2]octan-1-yl)-1,2,4-oxadiazol-3-yl, 5-(3-acetamido-bicyclo[1.1.1]pentan-1-yl)-1,2,4-oxadiazol-3-yl, 5-(2-acetyl-2-azaspiro[3.3]heptan-6-yl)-1,2,4-oxadiazole-3-yl, 5-(4-acetamido-cyclohexyl)-1,2,4-oxadiazole-3-yl, 5-(4-(ethyl-carbonyl-amino)-cyclohexyl)-1,2,4-oxadiazole-3-yl, or 5-(4-carbamoyl-bicyclo[2.2.2]octan-1-yl)-1,2,4-oxadiazol-3-yl; or
   - **R¹** represents C₁₋₃-alkyl (especially methyl);
      **R²** represents C₁₋₄-alkyl (especially methyl);
      **R³** represents 2,2,2-trifluoro-ethyl; and
      -**L-R⁴** represents 3-(2-hydroxy-1,1-dimethyl-ethyl)-1,2,4-oxadiazol-5-yl.
5) A further embodiment relates to compounds according to embodiment 1),
   - **R¹** represents C₁₋₃-alkyl (especially methyl);
      **R²** represents C₁₋₄-alkyl (especially methyl);
      **R³** represents 2,2,2-trifluoro-ethyl;
      **-L-** represents
         ➢ *-C≡C-C₀₋₂-alkylene-**, optionally mono-substituted with hydroxy (notably -C=C- or -C=C-C(CH₃)(OH)-; especially -C≡C-), wherein the asterisk (*) denotes the attachment point to the pyridinyl ring and two asterisks (**) denote the attachment point to **R⁴**); and
         ➢ **R⁴** represents mono-substituted C₃₋₇-cycloalkyl (especially cyclohexyl), wherein the substituent is hydroxy;
   - **R¹** represents C₁₋₃-alkyl (especially methyl);
      **R²** represents C₁₋₄-alkyl (especially methyl);
      **R³** represents 2,2,2-trifluoro-ethyl;
      -L- represents
         ➢ oxadiazol-diyl (notably 1,2,4-oxadiazol-diyl; especially 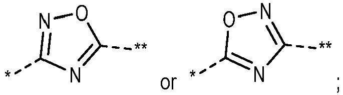 in particular 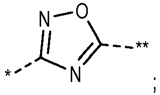 wherein one asterisk (*) denotes the attachment point to the pyridinyl ring and two asterisks (**) denote the attachment point to **R⁴**); and
      **R⁴** represents
         ➢ mono-substituted C₃₋₇-cycloalkyl (especially cyclohexyl), wherein the substituent is selected from hydroxy, carbamoyl, or C₁₋₃-alkyl-carbonyl-amino (especially acetyl-amino or ethyl-carbonyl-amino) [in particular, in case C₃₋₇-cycloalkyl represents cyclohexyl, said substituent is attached in position 4];
         ➢ or saturated 5- to 8-membered bridged bicyclic hydrocarbon ring system (especially such saturated 5- to 8-membered bridged bicyclic hydrocarbon ring system represents bicyclo[2.2.2]octan-1-yl or bicyclo[1.1.1]pentan-1-yl), wherein said saturated 5- to 8-membered bridged bicyclic hydrocarbon ring system independently is mono-substituted with hydroxy, hydroxy-C₁₋₃-alkyl (especially hydroxy-methyl), carbamoyl, or C₁₋₃-alkyl-carbonyl-amino (especially acetyl-amino) [in particular, the substituent is attached to the tertiary carbon atom in said 5- to 8-membered bridged bicyclic hydrocarbon ring system];
         ➢ or saturated 5- to 8-membered spiro bicyclic hydrocarbon ring system, wherein one ring carbon atom is replaced with a nitrogen atom (especially such saturated 5- to 8-membered spiro bicyclic hydrocarbon ring system is 2-azaspiro[3.3]heptan-6-yl); wherein said saturated 5- to 8-membered spiro bicyclic hydrocarbon ring system is mono-substituted with C₁₋₃-alkyl-carbonyl (especially acetyl) [in particular, the substituent is attached to the nitrogen atom];
   - **R¹** represents C₁₋₃-alkyl (especially methyl);
      **R²** represents C₁₋₄-alkyl (especially methyl);
      **R³** represents isopropyl; and
      -**L-R⁴** represents 2-(4-hydroxy-cyclohexyl)-ethyn-1-yl, 3-hydroxy-3-(pyrimidin-4-yl)-but-1-yn-1-yl, 5-(4-carbamoyl-cyclohexyl)-1,2,4-oxadiazol-3-yl, 5-(4-hydroxy-bicyclo[2.2.2]octan-1-yl)-1,2,4-oxadiazol-3-yl, 5-(3-acetamido-bicyclo[1.1.1]pentan-1-yl)-1,2,4-oxadiazol-3-yl, 5-(2-acetyl-2-azaspiro[3.3]heptan-6-yl)-1,2,4-oxadiazole-3-yl, 5-(4-acetamido-cyclohexyl)-1,2,4-oxadiazole-3-yl, 5-(4-(ethyl-carbonyl-amino)-cyclohexyl)-1,2,4-oxadiazole-3-yl, or 5-(4-carbamoyl-bicyclo[2.2.2]octan-1-yl)-1,2,4-oxadiazol-3-yl; or
   - **R¹** represents C₁₋₃-alkyl (especially methyl);
      **R²** represents C₁₋₄-alkyl (especially methyl);
      **R³** represents 2,2,2-trifluoro-ethyl; and
      -**L-R⁴** represents 3-(2-hydroxy-1,1-dimethyl-ethyl)-1,2,4-oxadiazol-5-yl.
6) A further embodiment relates to compounds according to any one of embodiments 1) to 5), wherein **R¹** represents methyl.
7) A further embodiment relates to compounds according to any one of embodiments 1) to 6), wherein **R²** represents methyl.
8) A further embodiment relates to compounds according to embodiment 1), wherein
   - **R¹** represents methyl;
      **R²** represents methyl;
      **R³** represents 2,2,2-trifluoro-ethyl; and
      -**L-R⁴** represents 5-(4-hydroxy-cyclohexyl)-1,2,4-oxadiazol-3-yl, 5-(3-hydroxymethyl-bicyclo[1.1.1]pentan-1-yl)-1,2,4-oxadiazol-3-yl, 3-(3-hydroxymethyl-bicyclo[1.1.1]pentan-1-yl)-1,2,4-oxadiazol-5-yl, 2-(4-hydroxycyclohexyl)-ethyn-1-yl, 3-hydroxy-3-(pyrimidin-4-yl)-but-1-yn-1-yl, 5-(4-carbamoyl-cyclohexyl)-1,2,4-oxadiazol-3-yl, 5-(4-hydroxy-bicyclo[2.2.2]octan-1-yl)-1,2,4-oxadiazol-3-yl, 5-(3-acetamido-bicyclo[1.1.1]pentan-1-yl)-1,2,4-oxadiazol-3-yl, 5-(2-acetyl-2-azaspiro[3.3]heptan-6-yl)-1,2,4-oxadiazole-3-yl, 5-(4-acetamido-cyclohexyl)-1,2,4-oxadiazole-3-yl, 5-(4-(ethyl-carbonyl-amino)-cyclohexyl)-1,2,4-oxadiazole-3-yl, or 5-(4-carbamoyl-bicyclo[2.2.2]octan-1-yl)-1,2,4-oxadiazol-3-yl;
   - **R¹** represents methyl;
      **R²** represents methyl;
      **R³** represents isopropyl; and
      -**L-R⁴** represents 2-(4-hydroxy-cyclohexyl)-ethyn-1-yl, 3-hydroxy-3-(pyrimidin-4-yl)-but-1-yn-1-yl, 5-(4-carbamoyl-cyclohexyl)-1,2,4-oxadiazol-3-yl, 5-(4-hydroxy-bicyclo[2.2.2]octan-1-yl)-1,2,4-oxadiazol-3-yl, 5-(3-acetamido-bicyclo[1.1.1]pentan-1-yl)-1,2,4-oxadiazol-3-yl, 5-(2-acetyl-2-azaspiro[3.3]heptan-6-yl)-1,2,4-oxadiazole-3-yl, 5-(4-acetamido-cyclohexyl)-1,2,4-oxadiazole-3-yl, 5-(4-(ethyl-carbonyl-amino)-cyclohexyl)-1,2,4-oxadiazole-3-yl, or 5-(4-carbamoyl-bicyclo[2.2.2]octan-1-yl)-1,2,4-oxadiazol-3-yl; or
   - **R¹** represents methyl;
      **R²** represents methyl;
      **R³** represents 2,2,2-trifluoro-ethyl; and
      -**L-R⁴** represents 3-(2-hydroxy-1,1-dimethyl-ethyl)-1,2,4-oxadiazol-5-yl.
9) A further embodiment relates to compounds according to any one of embodiments 1) to 8), wherein the asymmetric carbon atom bearing the hydroxy group has the absolute configuration depicted in Formula (II) 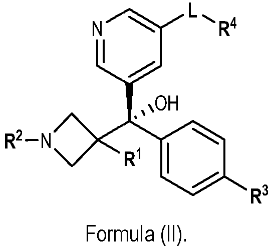
10) Another embodiment relates to compounds according to embodiment 1), which are selected from
   trans-4-[3-(5-{(R)-(1,3-Dimethyl-azetidin-3-yl)-hydroxy-[4-(2,2,2-trifluoro-ethyl)-phenyl]-methyl}-pyridin-3-yl)-[1,2,4]oxadiazol-5-yl]-cyclohexanol;
   cis-4-[3-(5-{(R)-(1,3-Dimethyl-azetidin-3-yl)-hydroxy-[4-(2,2,2-trifluoro-ethyl)-phenyl]-methyl}-pyridin-3-yl)-[1,2,4]oxadiazol-5-yl]-cyclohexanol;
   (R)-(1,3-Dimethyl-azetidin-3-yl)-{5-[5-(3-hydroxymethyl-bicyclo[1.1.1]pent-1-yl)-[1,2,4]oxadiazol-3-yl]-pyridin-3-yl}-[4-(2,2,2-trifluoro-ethyl)-phenyl]-methanol;
   N-[3-(3-{5-[(R)-(1,3-Dimethyl-azetidin-3-yl)-hydroxy-(4-isopropyl-phenyl)-methyl]-pyridin-3-yl}-[1,2,4]oxadiazol-5-yl)-bicyclo[1.1.1]pent-1-yl]-acetamide;
   4-(3-{5-[(R)-(1,3-Dimethyl-azetidin-3-yl)-hydroxy-(4-isopropyl-phenyl)-methyl]-pyridin-3-yl}-[1,2,4]oxadiazol-5-yl)-bicyclo[2.2.2]octan-1-ol;
   2-[5-(5-{(R)-(1,3-Dimethyl-azetidin-3-yl)-hydroxy-[4-(2,2,2-trifluoro-ethyl)-phenyl]-methyl}-pyridin-3-yl)-[1,2,4]oxadiazol-3-yl]-2-methyl-propan-1-ol;
   1-[6-(3-{5-[(R)-(1,3-Dimethyl-azetidin-3-yl)-hydroxy-(4-isopropyl-phenyl)-methyl]-pyridin-3-yl}-[1,2,4]oxadiazol-5-yl)-2-aza-spiro[3.3]hept-2-yl]-ethanone;
   trans-N-[4-(3-{5-[(R)-(1,3-Dimethyl-azetidin-3-yl)-hydroxy-(4-isopropyl-phenyl)-methyl]-pyridin-3-yl}-[1,2,4]oxadiazol-5-yl)-cyclohexyl]-propionamide;
   cis-N-[4-(3-{5-[(R)-(1,3-Dimethyl-azetidin-3-yl)-hydroxy-(4-isopropyl-phenyl)-methyl]-pyridin-3-yl}-[1,2,4]oxadiazol-5-yl)-cyclohexyl]-propionamide;
   cis-N-[4-(3-{5-[(R)-(1,3-Dimethyl-azetidin-3-yl)-hydroxy-(4-isopropyl-phenyl)-methyl]-pyridin-3-yl}-[1,2,4]oxadiazol-5-yl)-cyclohexyl]-acetamide;
   trans-N-[4-(3-{5-[(R)-(1,3-Dimethyl-azetidin-3-yl)-hydroxy-(4-isopropyl-phenyl)-methyl]-pyridin-3-yl}-[1,2,4]oxadiazol-5-yl)-cyclohexyl]-acetamide;
   (R)-(1,3-Dimethyl-azetidin-3-yl)-{5-[3-(3-hydroxymethyl-bicyclo[1.1.1]pent-1-yl)-[1,2,4]oxadiazol-5-yl]-pyridin-3-yl}-[4-(2,2,2-trifluoro-ethyl)-phenyl]-methanol;
   (R)-4-{5-[(R)-(1,3-Dimethyl-azetidin-3-yl)-hydroxy-(4-isopropyl-phenyl)-methyl]-pyridin-3-yl}-2-pyrimidin-4-yl-but-3-yn-2-ol;
   (S)-4-{5-[(R)-(1,3-Dimethyl-azetidin-3-yl)-hydroxy-(4-isopropyl-phenyl)-methyl]-pyridin-3-yl}-2-pyrimidin-4-yl-but-3-yn-2-ol;
   trans-4-{5-[(R)-(1,3-Dimethyl-azetidin-3-yl)-hydroxy-(4-isopropyl-phenyl)-methyl]-pyridin-3-ylethynyl}-cyclohexanol;
   cis-4-{5-[(R)-(1,3-Dimethyl-azetidin-3-yl)-hydroxy-(4-isopropyl-phenyl)-methyl]-pyridin-3-ylethynyl}-cyclohexanol;
   4-(3-{5-[(R)-(1,3-Dimethyl-azetidin-3-yl)-hydroxy-(4-isopropyl-phenyl)-methyl]-pyridin-3-yl}-[1,2,4]oxadiazol-5-yl)-bicyclo[2.2.2]octane-1-carboxylic acid amide;
   cis-4-(3-{5-[(R)-(1,3-Dimethyl-azetidin-3-yl)-hydroxy-(4-isopropyl-phenyl)-methyl]-pyridin-3-yl}-[1,2,4]oxadiazol-5-yl)-cyclohexanecarboxylic acid amide; or
   trans-4-(3-{5-[(R)-(1,3-Dimethyl-azetidin-3-yl)-hydroxy-(4-isopropyl-phenyl)-methyl]-pyridin-3-yl}-[1,2,4]oxadiazol-5-yl)-cyclohexanecarboxylic acid amide.
11) Another embodiment relates to compounds according to embodiment 1), which are selected from
   trans-4-[3-(5-{(R)-(1,3-Dimethyl-azetidin-3-yl)-hydroxy-[4-(2,2,2-trifluoro-ethyl)-phenyl]-methyl}-pyridin-3-yl)-[1,2,4]oxadiazol-5-yl]-cyclohexanol;
   (R)-(1,3-Dimethyl-azetidin-3-yl)-{5-[5-(3-hydroxymethyl-bicyclo[1.1.1]pent-1-yl)-[1,2,4]oxadiazol-3-yl]-pyridin-3-yl}-[4-(2,2,2-trifluoro-ethyl)-phenyl]-methanol;
   N-[3-(3-{5-[(R)-(1,3-Dimethyl-azetidin-3-yl)-hydroxy-(4-isopropyl-phenyl)-methyl]-pyridin-3-yl}-[1,2,4]oxadiazol-5-yl)-bicyclo[1.1.1]pent-1-yl]-acetamide;
   4-(3-{5-[(R)-(1,3-Dimethyl-azetidin-3-yl)-hydroxy-(4-isopropyl-phenyl)-methyl]-pyridin-3-yl}-[1,2,4]oxadiazol-5-yl)-bicyclo[2.2.2]octan-1-ol;
   2-[5-(5-{(R)-(1,3-Dimethyl-azetidin-3-yl)-hydroxy-[4-(2,2,2-trifluoro-ethyl)-phenyl]-methyl}-pyridin-3-yl)-[1,2,4]oxadiazol-3-yl]-2-methyl-propan-1-ol;
   1-[6-(3-{5-[(R)-(1,3-Dimethyl-azetidin-3-yl)-hydroxy-(4-isopropyl-phenyl)-methyl]-pyridin-3-yl}-[1,2,4]oxadiazol-5-yl)-2-aza-spiro[3.3]hept-2-yl]-ethanone;
   trans-N-[4-(3-{5-[(R)-(1,3-Dimethyl-azetidin-3-yl)-hydroxy-(4-isopropyl-phenyl)-methyl]-pyridin-3-yl}-[1,2,4]oxadiazol-5-yl)-cyclohexyl]-propionamide;
   cis-N-[4-(3-{5-[(R)-(1,3-Dimethyl-azetidin-3-yl)-hydroxy-(4-isopropyl-phenyl)-methyl]-pyridin-3-yl}-[1,2,4]oxadiazol-5-yl)-cyclohexyl]-acetamide;
   trans-N-[4-(3-{5-[(R)-(1,3-Dimethyl-azetidin-3-yl)-hydroxy-(4-isopropyl-phenyl)-methyl]-pyridin-3-yl}-[1,2,4]oxadiazol-5-yl)-cyclohexyl]-acetamide;
   (R)-(1,3-Dimethyl-azetidin-3-yl)-{5-[3-(3-hydroxymethyl-bicyclo[1.1.1]pent-1-yl)-[1,2,4]oxadiazol-5-yl]-pyridin-3-yl}-[4-(2,2,2-trifluoro-ethyl)-phenyl]-methanol;
   (R)-4-{5-[(R)-(1,3-Dimethyl-azetidin-3-yl)-hydroxy-(4-isopropyl-phenyl)-methyl]-pyridin-3-yl}-2-pyrimidin-4-yl-but-3-yn-2-ol;
   (S)-4-{5-[(R)-(1,3-Dimethyl-azetidin-3-yl)-hydroxy-(4-isopropyl-phenyl)-methyl]-pyridin-3-yl}-2-pyrimidin-4-yl-but-3-yn-2-ol;
   trans-4-{5-[(R)-(1,3-Dimethyl-azetidin-3-yl)-hydroxy-(4-isopropyl-phenyl)-methyl]-pyridin-3-ylethynyl}-cyclohexanol;
   cis-4-{5-[(R)-(1,3-Dimethyl-azetidin-3-yl)-hydroxy-(4-isopropyl-phenyl)-methyl]-pyridin-3-ylethynyl}-cyclohexanol;
   4-(3-{5-[(R)-(1,3-Dimethyl-azetidin-3-yl)-hydroxy-(4-isopropyl-phenyl)-methyl]-pyridin-3-yl}-[1,2,4]oxadiazol-5-yl)-bicyclo[2.2.2]octane-1-carboxylic acid amide; or
   4-(3-{5-[(R)-(1,3-Dimethyl-azetidin-3-yl)-hydroxy-(4-isopropyl-phenyl)-methyl]-pyridin-3-yl}-[1,2,4]oxadiazol-5-yl)-cyclohexanecarboxylic acid amide.
12) Another embodiment relates to compounds according to embodiment 1), which are selected from
   4-[3-(5-{(R)-(1,3-Dimethyl-azetidin-3-yl)-hydroxy-[4-(2,2,2-trifluoro-ethyl)-phenyl]-methyl}-pyridin-3-yl)-[1,2,4]oxadiazol-5-yl]-bicyclo[2.2.2]octane-1-carboxylic acid amide; or
   N-{4-[3-(5-{(R)-(1,3-Dimethyl-azetidin-3-yl)-hydroxy-[4-(2,2,2-trifluoro-ethyl)-phenyl]-methyl}-pyridin-3-yl)-[1,2,4]oxadiazol-5-yl]-trans-cyclohexyl}-acetamide.

The invention relates to compounds of the Formula (I) as defined in embodiment 1), or to such compounds further limited by the characteristics of any one of embodiments 2) to 9), under consideration of their respective dependencies; to pharmaceutically acceptable salts thereof; and to the use of such compounds as medicaments especially in the treatment of diseases or disorders where CCR6 receptors are involved as described hereinbelow.

The present invention also includes isotopically labelled, especially ²H (deuterium) labelled compounds of Formula (I), which compounds are identical to the compounds of Formula (I) except that one or more atoms have each been replaced by an atom having the same atomic number but an atomic mass different from the atomic mass usually found in nature. Isotopically labelled, especially ²H (deuterium) labelled compounds of Formula (I) and salts thereof are within the scope of the present invention. Substitution of hydrogen with the heavier isotope ²H (deuterium) may lead to greater metabolic stability, resulting e.g. in increased *in-vivo* half-life or reduced dosage requirements, or may lead to reduced inhibition of cytochrome P450 enzymes, resulting e.g. in an improved safety profile. In one embodiment of the invention, the compounds of Formula (I) are not isotopically labelled, or they are labelled only with one or more deuterium atoms. In a sub-embodiment, the compounds of Formula (I) are not isotopically labelled at all. Isotopically labelled compounds of Formula (I) may be prepared in analogy to the methods described hereinafter, but using the appropriate isotopic variation of suitable reagents or starting materials.

Where the plural form is used for compounds, salts, pharmaceutical compositions, diseases and the like, this is intended to mean also a single compound, salt, pharmaceutical composition, disease or the like.

Any reference to compounds of Formula (I) according to embodiments 1) to 12) is to be understood as referring also to the salts (and especially the pharmaceutically acceptable salts) of such compounds, as appropriate and expedient.

The term "pharmaceutically acceptable salts" refers to salts that retain the desired biological activity of the subject compound and exhibit minimal undesired toxicological effects. Such salts include inorganic or organic acid and/or base addition salts depending on the presence of basic and/or acidic groups in the subject compound. For reference see for example "Handbook of Pharmaceutical Salts. Properties, Selection and Use.", P. Heinrich Stahl, Camille G. Wermuth (Eds.), Wiley-VCH, 2008; and "Pharmaceutical Salts and Co-crystals", Johan Wouters and Luc Quéré (Eds.), RSC Publishing, 2012.

The compounds of Formula (I) may encompass compounds with one or more asymmetric centers, such as one or more asymmetric carbon atoms, which may be present in (R)- as well as (S)-configuration. The compounds of Formula (I) may further encompass compounds with one or more double bonds which are allowed to be present in Z- as well as E-configuration and/or compounds with substituents at a ring system which are allowed to be present, relative to each other, in cis- as well as trans-configuration. The compounds of Formula (I) may thus be present as mixtures of stereoisomers or preferably in stereoisomerically enriched form, especially as essentially pure stereoisomers. Mixtures of stereoisomers may be separated in a manner known to a person skilled in the art.

In case a particular compound (or generic structure) is designated as (R)- or (S)-enantiomer, such designation is to be understood as referring to the respective compound (or generic structure) in enriched, especially essentially pure, enantiomeric form. Likewise, in case a specific asymmetric center in a compound is designated as being in (R)- or (S)-configuration or as being in a certain relative configuration, such designation is to be understood as referring to the compound that is in enriched, especially essentially pure, form with regard to the respective configuration of said asymmetric center. In analogy, cis- or trans-designations are to be understood as referring to the respective stereoisomer in enriched, especially essentially pure, form. Likewise, in case a particular compound (or generic structure) is designated as Z- or E-stereoisomer (or in case a specific double bond in a compound is designated as being in Z- or E-configuration), such designation is to be understood as referring to the respective compound (or generic structure) in enriched, especially essentially pure, stereoisomeric form (or to the compound that is in enriched, especially essentially pure, form with regard to the respective configuration of the double bond). The same applies *mutatis mutandis* for compounds designated as being cis- or trans-stereoisomers.

The term "enriched", when used in the context of stereoisomers, is to be understood in the context of the present invention to mean that the respective stereoisomer is present in a ratio of at least 70:30, especially of at least 90:10 (i.e., in a purity of at least 70% by weight, especially of at least 90% by weight), with regard to the respective other stereoisomer / the entirety of the respective other stereoisomers.

The term "essentially pure", when used in the context of stereoisomers, is to be understood in the context of the present invention to mean that the respective stereoisomer is present in a purity of at least 95% by weight, especially of at least 99% by weight, with regard to the respective other stereoisomer / the entirety of the respective other stereoisomers.

The absolute stereochemical configuration of the compounds of Formula (I) and/or intermediates in the synthesis of the compounds of Formula (I), as disclosed hereinbelow or hereinabove, may be determined by common methods in the art such as obtaining a single crystal of said compounds/intermediates and performing an X-ray diffraction analysis thereon, for example by analogy to the methods applied to the compounds/intermediates disclosed in PCT/EP2021/061401.

The compounds of Formula (I) according to embodiments 1) to 12) and their pharmaceutically acceptable salts can be used as medicaments, e.g. in the form of pharmaceutical compositions for enteral (such especially oral) or parenteral administration (including topical application or inhalation).

The production of the pharmaceutical compositions can be effected in a manner which will be familiar to any person skilled in the art (see for example Remington, The Science and Practice of Pharmacy, 21st Edition (2005), Part 5, "Pharmaceutical Manufacturing" [published by Lippincott Williams & Wilkins]) by bringing the described compounds of Formula (I), or their pharmaceutically acceptable salts, optionally in combination with other therapeutically valuable substances, into a galenical administration form together with suitable, non-toxic, inert, therapeutically compatible solid or liquid carrier materials and, if desired, usual pharmaceutical adjuvants.

Whenever the word "between" is used to describe a numerical range, it is to be understood that the end points of the indicated range are explicitly included in the range. For example: if a temperature range is described to be between 40 °C and 80 °C, this means that the end points 40 °C and 80 °C are included in the range; or if a variable is defined as being an integer between 1 and 4, this means that the variable is the integer 1, 2, 3, or 4.

Unless used regarding temperatures, the term "about" (or alternatively the term "around") placed before a numerical value "X" refers in the current application to an interval extending from X minus 10% of X to X plus 10% of X, and preferably to an interval extending from X minus 5% of X to X plus 5% of X. In the particular case of temperatures, the term "about" placed before a temperature "Y" refers in the current application to an interval extending from the temperature Y minus 10 °C to Y plus 10 °C, and preferably to an interval extending from Y minus 5 °C to Y plus 5 °C.

The compounds of Formula (I) as defined hereinabove are useful for the prevention or treatment of various diseases, conditions or disorders ameliorated by modulating CCR6 receptors. Such diseases, conditions, or disorders, where CCR6 receptors are involved may be defined as inflammatory and/or autoimmune diseases, conditions, or disorders; and cancer.

The compounds of Formula (I) as defined hereinabove are useful for the prevention or treatment of of various diseases, conditions, or disorders, ameliorated by modulating CCR6 receptors. Such diseases, conditions, or disorders, where CCR6 receptors are involved may be defined as inflammatory/autoimmune diseases, conditions, or disorders, including rheumatoid arthritis; ankylosing spondylitis; spondyloarthritis; psoriasis; psoriatic arthritis; inflammatory skin disorders such as rosacea; Crohn's disease; ulcerative colitis; inflammatory bowel disease; irritable bowel disease; dry eye disease; multiple sclerosis; systemic lupus erythematosus; Sjögren's disease; autoimmune hepatitis; Primary Sclerosing Cholangitis; Posterior uveitis; allergic conjunctivitis; allergic disease in the gastrointestinal tract; type I diabetes and endometriosis; diseases of the ocular surface in which elevated levels of IL-17A have been recorded such as meibomian gland dysfunction; GVHD; graft-versus host disease; autoimmune keratitis; filamentary keratitis; dry eye syndrome with rheumatic arthritis; dry eye syndrome without systemic disease; Stevens-Johnson syndrome; psoriasis including plaque psoriasis, guttate psoriasis, inverse psoriasis, pustular psoriasis, erythrodermic psoriasis; autoimmune keratitis; filamentary keratitis; autoimmune uveitis; allergic conjunctivitis; asthma; allergic disease of the gastrointestinal tract; T1D; endometriosis; meibomian gland dysfunction; graft-versus host disease; juvenile arthritis; juvenile rheumatoid arthritis; systemic onset rheumatoid arthritis; pauciarticular rheumatoid arthritis; pauciarticular juvenile rheumatoid arthritis; polyarticular rheumatoid arthritis; enteropathic arthritis; juvenile Reiter's Syndrome; ankylosing spondylitis; juvenile ankylosing spondylitis; SEA Syndrome; reactive arthritis (reactive arthropathy); psoriatic arthropathy; juvenile enteropathic arthritis; polymyalgia rheumatica; enteropathic spondylitis; juvenile idiopathic arthritis (JIA); juvenile psoriatic arthritis; juvenile rheumatoid arthritis; systemic onset juvenile rheumatoid arthritis; acute pancreatitis; chronic pancreatitis; giant cell arteritis; atherosclerosis; bone erosion; intraperotoneal abscesses; intraperitoneal abscesses; and/or secondary osteoarthritis from inflammatory diseases.

Further, such diseases, conditions, or disorders, ameliorated by modulating CCR6 receptors may be defined as including cancer such as skin cancer e.g. melanoma (superficial spreading, nodular, lentigo maligna and acral lentiginous melanoma); advanced melanoma; metastatic melanoma; Merkel cell carcinoma; Kaposi sarcoma; basal cell carcinoma; squamous cell carcinoma; and pre-cancerous skin lesions such as actinic keratosis; lung cancer including small cell lung cancer and non-small (SCLC, NSCLC) such as squamous and non-squamous NSCLC; pleuropulmonary blastoma and tracheobronchial tumors; bladder cancer including urinary bladder cancer; urothelial cell carcinoma; mesothelioma; renal carcinomas including renal cell carcinoma (RCC) such as clear cell RCC; papillary RCC; chromophobe RCC; non-clear cell RCC; unclassified RCC; metastatic renal cell carcinoma; metastatic renal clear cell carcinoma; renal parenchymal carcinoma; gastro-intestinal cancers including colorectal cancer; metastatic colorectal cancer; familial adenomatous polyposis (FAP); rectum carcinoma; colon carcinoma; colorectal adenoma; colorectal adenocarcinoma; colorectal cancer liver metastases; hereditary non-polyposis colorectal cancer; esophageal cancer; gastric cancer; advanced gastric cancer; gallbladder cancer; cholangiocarcinoma; hepatocellular carcinoma; pancreatic cancer such as pancreatic adenocarcinoma or pancreatic ductal (adeno)carcinoma; pancreatic neuroendocrine tumors; endometrial cancer; ovarian cancer; prostate cancer including castrate-resistant prostate cancer; brain tumors including brain metastases, malignant gliomas, glioblastoma multiforme, medulloblastoma, meningiomas, astrocytoma; peripheral neuroectodermal tumors; oligoastrocytic tumors; oligodendrogliomas; ependymal tumors; anaplastic astrocytoma; pilocytic astrocytoma; craniopharyngioma; spinal cord tumors; brain stem glioma; central nervous system atypical teratoid/rhabdoid tumor; medulloblastoma; central nervous system germ cell tumors; craniopharyngioma; ependymoma; neuroblastoma; head and neck cancer such as esthesioneuroblastoma; cervical cancer; advanced cervical cancer; breast cancer including normal-like, basal-like, claudin-low, HER2 positive, luminal-A, luminal-B and triple negative breast carcinoma; pregnancy breast cancer and male breast cancer; oral tumors; nasopharyngeal tumors; heart tumors; thoracic cancer; lymphomas such as Hodgkin lymphoma, non-Hodgkin lymphoma, Burkitt lymphoma; primary intra-ocular B-Cell lymphoma; diffuse large B-cell lymphoma; primary mediastinal large B-cell lymphoma; mucosa-associated lymphoid tissue (MALT) lymphoma; gastric MALT lymphoma; cutaneous T-cell lymphoma; primary central nervous system lymphoma; Sézary syndrome and Waldenström macroglobulinemia; leukemia such as acute lymphoblastic leukemia; acute myeloid leukemia ; chronic lymphocytic leukemia; chronic myelogenous leukemia; hairy cell leukemia; chronic myeloid leukemia; adult T-cell leukemia; carcinomas; adenocarcinomas; thyroid carcinoma including papillary thyroid carcinoma and medullary thyroid carcinoma choriocarcinoma; sarcomas including Ewing's sarcoma; bone cancer such as osteosarcoma; high-grade osteosarcoma; rhabdomyosarcoma; Ewing sarcoma; malignant fibrous histiocytoma of the bone; chordoma; soft tissue sarcoma; myeloma; multiple myelomas; labial carcinoma; larynx carcinoma; hypopharynx carcinoma; tongue carcinoma; salivary gland carcinoma; cervix carcinoma; uterine corpus carcinoma; endometrium carcinoma; chorion carcinoma; testis carcinoma; urinary carcinoma; bronchial carcinoma; basalioma; teratoma; retinoblastoma; choroid melanoma; seminoma; chondrosarcoma; myosarcoma; liposarcoma; fibrosarcoma; plasmacytoma; hepatocarcinoma; advanced liver cancer; gastrointestinal stromal tumors; neuroendocrine tumors; bile duct cancer; appendix cancer; gastrointestinal carcinoid tumor; carcinoid tumor; islet cell tumor; small intestine cancer; stomach cancer; adrenocortical carcinoma; parathyroid cancer; paraganglioma; pheochromocytoma; pituitary tumor; penile cancer; renal pelvis and ureter cancer; testicular cancer; urethral cancer; Wilms tumor; extracranial germ cell tumor; extragonadal germ cell tumor; fallopian tube cancer; gestational trophoblastic tumor; primary peritoneal cancer; vaginal cancer; vulvar cancer; hypopharyngeal cancer; laryngeal cancer; papillomatosis cancer; lip and oral cavity cancer; metastatic squamous neck cancer; mouth cancer; nasopharyngeal cancer; oropharyngeal cancer; paranasal sinus and nasal cavity and paranasal sinus cancer; parathyroid cancer; pharyngeal cancer; throat cancer; chronic myeloproliferative neoplasm; Langerhans cell histiocytosis; plasma cell neoplasm; myelodysplastic syndromes; myeloproliferative neoplasm; midline tract carcinoma; virally induced tumors; and/or diseases involving CCR6 and/or CCL20 mediated metastasis, chemotaxis, cell adhesion, trans-endothelial migration, cell proliferation and/or survival.

Notably, such diseases, conditions, or disorders, ameliorated by modulating CCR6 receptors are selected from
- inflammatory/autoimmune diseases, conditions, or disorders, such as rheumatoid arthritis; ankylosing spondylitis; spondyloarthritis; psoriasis; psoriatic arthritis; inflammatory skin disorders e.g. rosacea; Crohn's disease; ulcerative colitis; irritable bowel disease; inflammatory bowel disease; dry eye disease; multiple sclerosis; systemic lupus erythematosus; Sjögren's disease; autoimmune hepatitis; Primary Sclerosing Cholangitis; psoriasis including plaque psoriasis, guttate psoriasis, inverse psoriasis, pustular psoriasis, erythrodermic psoriasis; autoimmune keratitis; filamentary keratitis; autoimmune uveitis; allergic conjunctivitis; asthma; allergic disease of the gastrointestinal tract; type 1 diabetes (T1D); endometriosis; meibomian gland dysfunction; and/or graft-versus host disease; and/or
- cancer such as lymphoma including T cell lymphoma and primary mediastinal B-cell lymphoma; brain cancer including glioma and glioblastoma; breast cancer including triple negative breast cancer; colorectal cancer; hepatocarcinoma; renal cell carcinoma; lung cancer including non-small cell lung cancer and small cell lung cancer; gastric cancer; melanoma including Merkel cell carcinoma, cutaneous squamous cell carcinoma and malignant melanoma; bladder cancer; head and neck cancer including squamous cell head and neck carcinoma; Hodgkin's lymphoma; cervical cancer; endometrial cancer; colon cancer; gastrointestinal stromal tumors; pancreatic cancer; prostatic cancer; leukemia including acute myeloid leukemia; ovarian cancer; oesophageal carcinomas; mesothelioma; neuroblastoma; sarcoma e.g. high-grade osteosarcoma; astrocytoma; myeloma; urothelial cancer including locally advanced and metastatic urothelial cancer; MSI-H or dMMR cancer; rectal cancer; laryngeal cancer; salivary adenocarcinoma; multiple myeloma; cholangiocarcinoma; oral squamous cell carcinoma; thyroid cancer; and/or esophagogastric junction cancer.

Especially, such diseases, conditions, or disorders, ameliorated by modulating CCR6 receptors are selected from
- inflammatory/autoimmune diseases, conditions, or disorders, such as psoriasis; psoriatic arthritis; rheumatoid arthritis; ankylosing spondylitis; spondyloarthritis; inflammatory skin disorders e.g. rosacea; Crohn's disease; ulcerative colitis; irritable bowel disease; inflammatory bowel disease; dry eye disease; multiple sclerosis; systemic lupus erythematosus; Sjögren's disease; autoimmune hepatitis; and/or Primary Sclerosing Cholangitis; In particular, such diseases, conditions, or disorders, are psoriasis, psoriatic arthritis, or inflammatory bowel disease; and especially may be selected from A1) psoriasis or psoriatic arthritis; or A2) inflammatory bowel disease; and/or
- cancer such as lymphoma (e.g. T cell lymphoma); brain cancer (e.g. glioma or glioblastoma); breast cancer; colorectal cancer; hepatocarcinomas; renal cell carcinoma; lung cancer; and/or gastric cancer.

When used for the prevention / prophylaxis or treatment of a cancer, such use includes use of the compounds of Formula (I) of the present invention or the compounds of Formula (I) as disclosed in WO2021219849 (e.g. 2-(3-{5-[(R)-(1,3-Dimethyl-azetidin-3-yl)-hydroxy-(4-isopropyl-phenyl)-methyl]-pyridin-3-yl}-[1,2,4]oxadiazol-5-yl)-propan-2-ol; 1-[4-(3-{5-[(R)-(1,3-Dimethyl-azetidin-3-yl)-hydroxy-(4-isopropyl-phenyl)-methyl]-pyridin-3-yl}-[1,2,4]oxadiazol-5-yl)-piperidin-1-yl]-ethanone; or (S)-4-{5-[(R)-(1,3-Dimethyl-azetidin-3-yl)-hydroxy-(4-isopropyl-phenyl)-methyl]-pyridin-3-yl}-2-(6-methyl-pyrimidin-4-yl)-but-3-yn-2-ol; or a pharmaceutically acceptable salt thereof) as single therapeutic agents and their use in combination with one or more chemotherapy agents and / or radiotherapy and / or targeted therapy (especially in combination with targeted therapy).

The terms "radiotherapy" or "radiation therapy" or "radiation oncology", refer to the medical use of ionizing radiation in the prevention / prophylaxis (adjuvant therapy) and / or treatment of cancer; including external and internal radiotherapy.

The term "targeted therapy" refers to the prevention / prophylaxis (adjuvant therapy) and / or treatment of cancer with one or more anti-neoplastic agents such as small molecules or antibodies which act on specific types of cancer cells or stromal cells. Some targeted therapies block the action of certain enzymes, proteins, or other molecules involved in the growth and spread of cancer cells. Other types of targeted therapies help the immune system kill cancer cells (immunotherapies); or inhibit angiogenesis, the growth and formation of new blood vessels in the tumor; or deliver toxic substances directly to cancer cells and kill them. An example of a targeted therapy which is in particular suitable to be combined with the compounds of Formula (I) of the present invention or with the compounds of Formula (I) as disclosed in WO2021219849 (e.g. 2-(3-{5-[(R)-(1,3-Dimethyl-azetidin-3-yl)-hydroxy-(4-isopropyl-phenyl)-methyl]-pyridin-3-yl}-[1,2,4]oxadiazol-5-yl)-propan-2-ol; 1-[4-(3-{5-[(R)-(1,3-Dimethyl-azetidin-3-yl)-hydroxy-(4-isopropyl-phenyl)-methyl]-pyridin-3-yl}-[1,2,4]oxadiazol-5-yl)-piperidin-1-yl]-ethanone; or (S)-4-{5-[(R)-(1,3-Dimethyl-azetidin-3-yl)-hydroxy-(4-isopropyl-phenyl)-methyl]-pyridin-3-yl}-2-(6-methyl-pyrimidin-4-yl)-but-3-yn-2-ol; or a pharmaceutically acceptable salt thereof) is immunotherapy, especially immunotherapy targeting the programmed cell death receptor 1 (PD-1 receptor) or its ligand PD-L1.

Immunotherapy further refers to (i) an agonist of a stimulatory (including a co-stimulatory) receptor or (ii) an antagonist of an inhibitory (including a co- inhibitory) signal on T cells, both of which result in amplifying antigen-specific T cell responses (often referred to as immune checkpoint regulators). Certain of the stimulatory and inhibitory molecules are members of the immunoglobulin super family (IgSF). One important family of membrane-bound ligands that bind to co-stimulatory or co-inhibitory receptors is the B7 family, which includes B7-1, B7-2, B7-HI (PD-LI), B7-DC (PD-L2), B7-H2 (ICOS-L), B7-H3, B7-H4, B7-H5 (VISTA), and B7-H6. Another family of membrane bound ligands that bind to co-stimulatory or co-inhibitory receptors is the TNF family of molecules that bind to cognate TNF receptor family members, which includes CD40 and CD40L, OX-40, OX-40L, CD70, CD27L, CD30, CD30L, 4-IBBL, CD137 (4-IBB), TRAIL/Apo2-L, TRAILR1/DR4, TRAILR2/DR5, TRAILR3, TRAILR4, OPG, RANK, RANKL, TWEAKR/Fnl4, TWEAK, BAFFR, EDAR, XEDAR, TACI, APRIL, BCMA, LTpR, LIGHT, DcR3, HVEM, VEGI/TLIA, TRAMP/DR3, EDAR, EDAI, XEDAR, EDA2, TNFRI, Lymphotoxin a/TNFp, TNFR2, TNFa, LTPR, Lymphotoxin a 1p2, FAS, FASL, RELT, DR6, TROY, NGFR.

When used in combination with the compounds of Formula (I) of the present invention or with the compounds of Formula (I) as disclosed in WO2021219849 (e.g. 2-(3-{5-[(R)-(1,3-Dimethyl-azetidin-3-yl)-hydroxy-(4-isopropyl-phenyl)-methyl]-pyridin-3-yl}-[1,2,4]oxadiazol-5-yl)-propan-2-ol; 1-[4-(3-{5-[(R)-(1,3-Dimethyl-azetidin-3-yl)-hydroxy-(4-isopropylphenyl)-methyl]-pyridin-3-yl}-[1,2,4]oxadiazol-5-yl)-piperidin-1-yl]-ethanone; or (S)-4-{5-[(R)-(1,3-Dimethyl-azetidin-3-yl)-hydroxy-(4-isopropyl-phenyl)-methyl]-pyridin-3-yl}-2-(6-methyl-pyrimidin-4-yl)-but-3-yn-2-ol; or a pharmaceutically acceptable salt thereof), the term "targeted therapy" especially refers to agents such as:
a) Epidermal growth factor receptor (EGFR) inhibitors or blocking antibodies (for example Gefitinib, Erlotinib, Afatinib, Icotinib, Lapatinib, Panitumumab, Zalutumumab, Nimotuzumab, Matuzumab and Cetuximab) as well as trastuzumab (HERCEPTIN);
b) RAS/RAF/MEK pathway inhibitors (for example Vemurafenib, Sorafenib, Dabrafenib, GDC-0879, PLX-4720, LGX818, RG7304, Trametinib (GSK1120212), Cobimetinib (GDC-0973/XL518), Binimetinib (MEK162, ARRY-162), Selumetinib (AZD6244));
c) Janus kinase (JAK) inhibitors (for example Ruxolitinib, Itacitinib, Momelotinib);
d) Aromatase inhibitors (for example Exemestane, Letrozole, Anastrozole, Vorozole, Formestane, Fadrozole);
e) signal transduction inhibitors (STI). A "signal transduction inhibitor" is an agent that selectively inhibits one or more vital steps in signaling pathways, in the normal function of cancer cells, thereby leading to apoptosis. Suitable STis include but are not limited to: (i) bcr/abl kinase inhibitors such as, for example, STI 571 (GLEEVEC^{®}), Dasatinib; (ii) epidermal growth factor (EGF) receptor inhibitors such as, for example, kinase inhibitors (IRESSA^{®}, SSI-774) and antibodies (Imclone: C225 [Goldstein et al., Clin. Cancer Res., 1:1311-1318 (1995)], and Abgenix: ABX-EGF); (iii) her-2/neu receptor inhibitors such as famesyl transferase inhibitors (FTI) such as, for example, L-744,832 (Kohl et al., Nat. Med., 1(8):792-797 (1995)); (iv) inhibitors of Akt family kinases or the Akt pathway, such as, for example, rapamycin (see, for example, Sekulic et al., Cancer Res., 60:3504-3513 (2000)); (v) cell cycle kinase inhibitors such as, for example, flavopiridol and UCN-O1 (see, for example, Sausville, Curr. Med. Chem. Anti-Cane. Agents, 3:47-56 (2003)); and (vi) phosphatidyl inositol kinase inhibitors such as, for example, LY294002 (see, for example, Vlahos et al., J Biol. Chem., 269:5241-5248 (1994)).
f) Angiogenesis inhibitors, especially VEGF signalling inhibitors such as Bevacuzimab (Avastin), Ramucirumab, Sorafenib or Axitinib;
g) Immune Checkpoint inhibitors (for example: anti-PD1 antibodies such as Pembrolizumab (Lambrolizumab, MK-3475), Nivolumab, Pidilizumab (CT-011), AMP-514/MEDI0680, PDR001, SHR-1210; REGN2810, BGBA317, PF-06801591, MGA-012, TSR042, JS-001, BCD100, IBI-308, BI-754091; fusion proteins targeting PD-1 such as AMP-224; small molecule anti-PD1 agents such as for example compounds disclosed in WO2015/033299, WO2015/044900 and WO2015/034820; anti-PD1L antibodies, such as BMS-936559, atezolizumab (MPDL3280A, RG7446), avelumab (MSB0010718C), durvalumab (MEDI4736); anti-PDL2 antibodies, such as AMP224; anti-CTLA-4 antibodies, such as ipilimumab, tremelimumab; anti-Lymphocyte-activation gene 3 (LAG-3) antibodies, such as Relatlimab (BMS-986016), IMP701, IMP731, MK-4280, ImmuFact IMP321; anti T cell immunoglobulin mucin-3 (TIM-3) antibodies, such as MBG453, TSR-022; anti T cell immunoreceptor with Ig and ITIM domains (TIGIT) antibodies, such as RG6058 (anti-TIGIT, MTIG7192A); anti- Killer-cell immunoglobulin-like receptors (KIR) for example Lirilumab (IPH2102/BMS-986015), antagonists of Galectins (such as Galectin-1, Galectin-9), BTLA;
h) Vaccination approaches (for example dendritic cell vaccination, DNA, peptide or protein vaccination (for example with gp100 peptide or MAGE-A3 peptide) as well as recombinant viruses;
i)Re-introduction of patient derived or allogenic (non-self) cancer cells genetically modified to secrete immunomodulatory factors such as granulocyte monocyte colony stimulating factor (GMCSF) gene-transfected tumor cell vaccine (GVAX) or Fms-related tyrosine kinase 3 (Flt-3) ligand gene-transfected tumor cell vaccine (FVAX),or Toll like receptor enhanced GM-CSF tumor based vaccine (TEGVAX);
j) T-cell based adoptive immunotherapies, including chimeric antigen receptor (CAR) engineered T-cells (for example CTL019);
k) Cytokine or immunocytokine based therapy (for example Interferon alpha, interferon beta, interferon gamma, interleukin 2, interleukin 6, interleukin 10, interleukin 15, TGF-β);
l) Toll-like receptor (TLR) agonists (for example resiquimod, imiquimod, motolimod, glucopyranosyl lipid A, CpG oligodesoxynucleotides);
m) Thalidomide analogues (for example Lenalidomide, Pomalidomide);
n) Activators of T-cell co-stimulatory receptors (for example anti-CD137/4-1BB antibodies, such as BMS-663513 (urelumab), Utomilumab (PF-05082566); anti-OX40/CD134 (Tumor necrosis factor receptor superfamily, member 4) (such as RG7888 (MOXR0916), 9B12; MEDI6469, GSK3174998, MEDI6383, MEDI0562), anti OX40-Ligand/CD252; anti-glucocorticoid-induced TNFR family related gene (GITR) (such as TRX518, MEDI1873, MK-4166, BMS-986156, BMS-986153), anti-CD40 (TNF receptor superfamily member 5) antibodies (such as Dacetuzumab (SGN-40), HCD122, CP-870,893, RG7876, ADC-1013, APX005M, SEA-CD40); anti-CD40-Ligand antibodies (such as BG9588); anti-CD27 antibodies such as Varlilumab; anti-CD28 antibodies; anti-ICOS antibodies;
o) Molecules binding a tumor specific antigen as well as a T-cell surface marker such as bispecific antibodies or antibody fragments, antibody mimetic proteins such as designed ankyrin repeat proteins (DARPINS), bispecific T-cell engager (BITE, for example AMG103, AMG330);
p) Antibodies or small molecular weight inhibitors targeting colony-stimulating factor-1 receptor (CSF-1R) (for example Emactuzumab (RG7155), Cabiralizumab (FPA-008), PLX3397);
q) Agents targeting immune cell check points on natural killer cells such as antibodies against Killer-cell immunoglobulin-like receptors (KIR) for example Lirilumab (IPH2102/BMS-986015);
r) Agents targeting the Adenosine receptors or the ectonucleases CD39 and CD73 that convert adenosin triphosphate (ATP) to Adenosine, such as MEDI9447 (anti-CD73 antibody), PBF-509; CPI-444 (Adenosine A2a receptor antagonist);
s) antagonists to chemokine receptors including CCR2 or CCR4;
t) modulators of the complement system v) agents that deplete or inhibit T regulatory cells (e.g., using an anti-CD25 monoclonal antibody (e.g., daclizumab) or by ex vivo anti-CD25 bead depletion) or reverse/prevent T cell anergy or exhaustion.

When used in combination with the compounds of Formula (I) of the present invention or with the compounds of Formula (I) as disclosed in WO2021219849 (e.g. 2-(3-{5-[(R)-(1,3-Dimethyl-azetidin-3-yl)-hydroxy-(4-isopropyl-phenyl)-methyl]-pyridin-3-yl}-[1,2,4]oxadiazol-5-yl)-propan-2-ol; 1-[4-(3-{5-[(R)-(1,3-Dimethyl-azetidin-3-yl)-hydroxy-(4-isopropylphenyl)-methyl]-pyridin-3-yl}-[1,2,4]oxadiazol-5-yl)-piperidin-1-yl]-ethanone; or (S)-4-{5-[(R)-(1,3-Dimethyl-azetidin-3-yl)-hydroxy-(4-isopropyl-phenyl)-methyl]-pyridin-3-yl}-2-(6-methyl-pyrimidin-4-yl)-but-3-yn-2-ol; or a pharmaceutically acceptable salt thereof), immune checkpoint inhibitors, and especially those targeting the PD-1 receptor or its ligand PD-L1, are preferred.

The term "chemotherapy" refers to the treatment of cancer with one or more cytotoxic anti-neoplastic agents ("cytotoxic chemotherapy agents"). Chemotherapy is often used in conjunction with other cancer treatments, such as radiation therapy or surgery. The term especially refers to conventional chemotherapeutic agents which act by killing cells that divide rapidly, one of the main properties of most cancer cells. Chemotherapy may use one drug at a time (single-agent chemotherapy) or several drugs at once (combination chemotherapy or polychemotherapy). Chemotherapy using drugs that convert to cytotoxic activity only upon light exposure is called photochemotherapy or photodynamic therapy.

The term "cytotoxic chemotherapy agent" or "chemotherapy agent" as used herein refers to an active anti-neoplastic agent inducing apoptosis or necrotic cell death.

When used in combination with the compounds of Formula (I) of the present invention or with the compounds of Formula (I) as disclosed in WO2021219849 (e.g. 2-(3-{5-[(R)-(1,3-Dimethyl-azetidin-3-yl)-hydroxy-(4-isopropyl-phenyl)-methyl]-pyridin-3-yl}-[1,2,4]oxadiazol-5-yl)-propan-2-ol; 1-[4-(3-{5-[(R)-(1,3-Dimethyl-azetidin-3-yl)-hydroxy-(4-isopropylphenyl)-methyl]-pyridin-3-yl}-[1,2,4]oxadiazol-5-yl)-piperidin-1-yl]-ethanone; or (S)-4-{5-[(R)-(1,3-Dimethyl-azetidin-3-yl)-hydroxy-(4-isopropyl-phenyl)-methyl]-pyridin-3-yl}-2-(6-methyl-pyrimidin-4-yl)-but-3-yn-2-ol; or a pharmaceutically acceptable salt thereof), the term especially refers to conventional cytotoxic chemotherapy agents such as: 1) alkylating agents (including, without limitation, nitrogen mustards, ethylenimine derivatives, alkyl sulfonates, nitrosoureas and triazenes) such as uracil mustard, mechlorethamine, chlorambucil, cyclophosphamide, ifosfamide, streptozocin, carmustine, lomustine, melphalan, busulfan, procarbazine, dacarbazine, temozolomide, pipobroman, triethylene-melamine, triethylenethiophosphoramine, thiotepa or altretamine; in particular temozolomide); 2) platinum drugs (for example cisplatin, carboplatin or oxaliplatin); 3) antimetabolite drugs (for example 5-fluorouracil, floxuridine, pentostatine, capecitabine, 6-mercaptopurine, methotrexate, gemcitabine, cytarabine, fludarabine or pemetrexed); 4) anti-tumor antibiotics (for example daunorubicin, doxorubicin, epirubicin, idarubicin, actinomycin-D, bleomycin, mitomycin-C or mitoxantrone); 5) mitotic inhibitors (for example paclitaxel, docetaxel, ixabepilone, vinblastine, vincristine, vinorelbine, vindesine or estramustine); or 6) topoisomerase inhibitors (for example etoposide, teniposide, topotecan, irinotecan, diflomotecan or elomotecan). Also suitable are cytotoxic agents such as biological response modifiers; growth inhibitors; antihormonal therapeutic agents; leucovorin; tegafur; and haematopoietic growth factors. When used in combination with the compounds of Formula (I) of the present invention or with the compounds of Formula (I) as disclosed in WO2021219849 (e.g. 2-(3-{5-[(R)-(1,3-Dimethyl-azetidin-3-yl)-hydroxy-(4-isopropyl-phenyl)-methyl]-pyridin-3-yl}-[1,2,4]oxadiazol-5-yl)-propan-2-ol; 1-[4-(3-{5-[(R)-(1,3-Dimethyl-azetidin-3-yl)-hydroxy-(4-isopropylphenyl)-methyl]-pyridin-3-yl}-[1,2,4]oxadiazol-5-yl)-piperidin-1-yl]-ethanone; or (S)-4-{5-[(R)-(1,3-Dimethyl-azetidin-3-yl)-hydroxy-(4-isopropyl-phenyl)-methyl]-pyridin-3-yl}-2-(6-methyl-pyrimidin-4-yl)-but-3-yn-2-ol; or a pharmaceutically acceptable salt thereof), preferred cytotoxic chemotherapy agents are the above-mentioned alkylating agents (notably fotemustine, cyclophosphamide, ifosfamide, carmustine, dacarbazine and prodrugs thereof such as especially temozolomide or pharmaceutically acceptable salts of these compounds; in particular temozolomide); mitotic inhibitors (notably paclitaxel, docetaxel, ixabepilone; or pharmaceutically acceptable salts of these compounds; in particular paclitaxel); platinum drugs (notably cisplatin, oxaliplatin and carboplatin); as well etoposide and gemcitabine.

For avoidance of any doubt, if compounds are described as useful for the prevention or treatment of certain diseases, conditions or disorders, such compounds are likewise suitable for use in the preparation of a medicament for the prevention or treatment of said diseases.

The present invention also relates to compounds for use in a method for the prevention or treatment of diseases, conditions or disorders mentioned hereinabove and/or hereinbelow comprising administering to a subject a pharmaceutically active amount of a compound as described hereinabove or/and hereinbelow either alone or in combination with other pharmacologically active compounds and/or therapies.

In a preferred embodiment of the present invention, the administered amount of a compound of Formula (I) is comprised between 1 mg and 1000 mg per day, particularly between 5 mg and 500 mg per day, more particularly between 25 mg and 400 mg per day, especially between 50 mg and 200 mg per day.

The meaning of the term "prevention" may also be understood as "prophylaxis".

### Preparation of compounds of Formula (I)

A further aspect of the invention is a process for the preparation of compounds of Formula (I). Compounds according to Formula (I) of the present invention can be prepared from commercially available or well-known starting materials according to the methods described in the experimental part; by analogous methods; or may be prepared by appropriate modifications of the general synthetic routes disclosed in PCT/EP2021/061401. The compounds obtained may also be converted into salts, especially pharmaceutically acceptable salts thereof in a manner known *per se*.

### Experimental section

### Abbreviations (as used herein and in the description above):

- Ac: acetyl
- anal: analytical
- anh: anhydrous
- aq.: aqueous
- Boc: tert.-butyloxycarbonyl
- CC: column chromatography
- CDI: 1,1'-carbonyldiimidazole
- DEA: diethylamine
- DCM: dichloromethane
- DMF: N,N-dimethylformamide
- EA: ethyl acetate
- Et: ethyl
- eq: equivalent
- FLIPR: fluorescent imaging plate reader
- Fluo-8-AM: acetyloxymethyl 2-[N-[2-(acetyloxymethoxy)-2-oxoethyl]-4-[3-(acetyloxymethoxy)-6-oxoxanthen-9-yl]-2-[2-[2-[bis[2-(acetyloxymethoxy)-2-oxoethyl]amino]phenoxy]ethoxy]anilino]acetate
- g: gram(s)
- HEK: human embryonic kidney
- Hex: hexyl
- h: hour(s)
- Hep: heptane
- HPLC: high performance liquid chromatography
- HV: high vacuum
- LC-MS: liquid chromatography mass spectrometry
- M: molarity [mol / L]
- Me: methyl
- mg: milligram(s)
- min: minute(s)
- mL: millilitre
- nBu: n-butyl
- org: organic
- prep: preparative
- iPr: isopropyl
- PyBOP: (benzotriazol-1-yloxy)tripyrrolidinophosphonium hexafluorophosphate
- rpm: rotations per minute
- RT: room temperature
- rxn: reaction
- sat: saturated
- SFC: supercritical fluid chromatography
- soln: solution
- t: time
- T3P^{®}: propanephosphonic acid anhydride
- tBu: tert-butyl
- TEA: triethylamine
- t_{R}: retention time
- THF: tetrahydrofuran

### I. Chemistry

The following Examples illustrate the preparation of biologically active compounds of the invention but do not at all limit the scope thereof.

General: All temperatures are stated in degrees Celsius (°C). Unless otherwise indicated, the reactions take place at RT under an argon atmosphere and are run in a round-bottomed flask or sealable tube equipped with a magnetic stir bar.

### Characterization methods used:

The LC-MS retention times have been obtained using the following elution conditions:

### I) LC-MS (A):

Zorbax RRHD SB-Aq, 1.8 µm, 2.1x50mm column thermostated at 40°C. The two elution solvents were as follows: solvent A= water + 0.04%TFA; solvent B = MeCN. The eluent flow rate was 0.8 mL/min and the characteristics of the eluting mixture proportion in function of the time t from start of the elution are summarized in the table below (a linear gradient being used between two consecutive time points):

| t (min) | 0 | 1.2 | 1.9 | 2.1 |
|---|---|---|---|---|
| Solvent A (%) | 95 | 5 | 5 | 95 |
| Solvent B (%) | 5 | 95 | 95 | 5 |

The chiral HPLC/SFC retention times have been obtained using the following elution conditions:

### I) Chiral HPLC (A):

CHIRALPAK AD-H, 5 µm, 4.6x250mm column thermostated at 25°C was used. The two elution solvents were as follows: solvent A= Hep; solvent B = EtOH. The eluent flow rate was 0.8mL/min, the isocratic solvent proportion was 80% (A) / 20% (B).

### II) Chiral SFC (B):

CHIRALCEL OD-H, 5 µm, 4.6x250mm column thermostated at 40°C was used. The two elution solvents were as follows: solvent A= CO₂; solvent B = MeOH. The eluent flow rate was 4mL/min, the isocratic solvent proportion was 90% (A) / 10% (B).

### III) Chiral SFC (C):

CHIRALPAK AD-H, 5 µm, 4.6x250mm column thermostated at 40°C was used. The two elution solvents were as follows: solvent A= CO₂; solvent B = EtOH. The eluent flow rate was 4mL/min and the isocratic solvent proportion was 80% (A) / 20% (B).

### IV) Chiral SFC (D):

CHIRALPAK IC, 5 µm, 4.6x250mm column thermostated at 40°C was used. The two elution solvents were as follows: solvent A= CO₂; solvent B = iPrOH+0.1%DEA. The eluent flow rate was 4mL/min and the isocratic solvent proportion was 85% (A) / 15% (B).

### V) Chiral SFC (E):

CHIRALPAK IB, 5 µm, 4.6x250mm column thermostated at 40°C was used. The two elution solvents were as follows: solvent A= CO₂; solvent B = MeOH+0.1%DEA. The eluent flow rate was 4mL/min and the isocratic solvent proportion was 75% (A) / 25% (B).

### Purification methods used:

### Preparative LC-MS methods used:

The purifications by preparative LC-MS have been performed using the conditions described hereafter.

### I) Prep LC-MS (I):

A X-Bridge column (Waters C18, 10µm OBD, 30x75 mm) was used. The two elution solvents were as follows: solvent A = water + 0.5% formic acid; solvent B = MeCN. The eluent flow rate was 75 mL/min and the characteristics of the eluting mixture proportion in function of the time t from start of the elution are summarized in the tables below (a linear gradient being used between two consecutive time points):

| t (min) | 0 | 0.01 | 4.0 | 6.0 | 6.2 | 6.6 |
|---|---|---|---|---|---|---|
| Solvent A (%) | 80 | 80 | 5 | 5 | 80 | 80 |
| Solvent B (%) | 20 | 20 | 95 | 95 | 20 | 20 |

### II) Prep LC-MS (II):

A X-Bridge column (Waters C18, 10µm OBD, 30x75 mm) was used. The two elution solvents were as follows: solvent A = water + 0.5% NH₄OH (25%); solvent B = MeCN. The eluent flow rate was 75 mL/min and the characteristics of the eluting mixture proportion in function of the time t from start of the elution are summarized in the tables below (a linear gradient being used between two consecutive time points):

| t (min) | 0 | 0.01 | 4.0 | 6.0 | 6.2 | 6.6 |
|---|---|---|---|---|---|---|
| Solvent A (%) | 80 | 80 | 5 | 5 | 80 | 80 |
| Solvent B (%) | 20 | 20 | 95 | 95 | 20 | 20 |

### III) Prep LC-MS (III):

A X-Bridge column (Waters C18, 10µm OBD, 30x75 mm) was used. The two elution solvents were as follows: solvent A = water + 0.5% NH₄OH (25%); solvent B = MeCN. The eluent flow rate was 75 mL/min and the characteristics of the eluting mixture proportion in function of the time t from start of the elution are summarized in the tables below (a linear gradient being used between two consecutive time points):

| t (min) | 0 | 0.01 | 4.0 | 6.0 | 6.2 | 6.6 |
|---|---|---|---|---|---|---|
| Solvent A (%) | 90 | 90 | 5 | 5 | 90 | 90 |
| Solvent B (%) | 10 | 10 | 95 | 95 | 10 | 10 |

### IV) Prep LC-MS (IV):

X-Bridge column (Waters C18, 10µm OBD, 30x75 mm) was used. The two elution solvents were as follows: solvent A = water + 0.5% NH₄OH (25%); solvent B = MeCN. The eluent flow rate was 75 mL/min and the characteristics of the eluting mixture proportion in function of the time t from start of the elution are summarized in the tables below (a linear gradient being used between two consecutive time points):

| t (min) | 0 | 0.01 | 3.5 | 6.0 | 6.2 | 6.6 |
|---|---|---|---|---|---|---|
| Solvent A (%) | 50 | 50 | 5 | 5 | 50 | 50 |
| Solvent B (%) | 50 | 50 | 95 | 95 | 50 | 50 |

### V) Prep LC-MS (V):

An Agilent column (Zorbax SB-Aq, 5µm OBD, 30x75 mm) was used. The two elution solvents were as follows: solvent A = water + 0.5% formic acid; solvent B = MeCN. The eluent flow rate was 75 mL/min and the characteristics of the eluting mixture proportion in function of the time t from start of the elution are summarized in the tables below (a linear gradient being used between two consecutive time points):

| t (min) | 0 | 0.01 | 4.0 | 6.0 | 6.2 | 6.6 |
|---|---|---|---|---|---|---|
| Solvent A (%) | 80 | 80 | 5 | 5 | 80 | 80 |
| Solvent B (%) | 20 | 20 | 95 | 95 | 20 | 20 |

### VI) Prep LC-MS (VI):

An Agilent column (Zorbax SB-Aq, 5µm OBD, 30x75 mm) was used. The two elution solvents were as follows: solvent A = water + 0.5% formic acid; solvent B = MeCN. The eluent flow rate was 75 mL/min and the characteristics of the eluting mixture proportion in function of the time t from start of the elution are summarized in the tables below (a linear gradient being used between two consecutive time points):

| t (min) | 0 | 0.01 | 3 | 4.0 | 6.0 | 6.2 | 6.6 |
|---|---|---|---|---|---|---|---|
| Solvent A (%) | 95 | 95 | 50 | 5 | 5 | 95 | 95 |
| Solvent B (%) | 5 | 5 | 50 | 95 | 95 | 5 | 5 |

### Vli) Prep LC-MS (VII):

An Agilent column (Zorbax SB-Aq, 5µm OBD, 30x75 mm) was used. The two elution solvents were as follows: solvent A = water + 0.5% formic acid; solvent B = MeCN. The eluent flow rate was 75 mL/min and the characteristics of the eluting mixture proportion in function of the time t from start of the elution are summarized in the tables below (a linear gradient being used between two consecutive time points):

| t (min) | 0 | 0.01 | 3.5 | 6.0 | 6.2 | 6.6 |
|---|---|---|---|---|---|---|
| Solvent A (%) | 70 | 70 | 5 | 5 | 70 | 70 |
| Solvent B (%) | 30 | 30 | 95 | 95 | 30 | 30 |

### VIII) Prep LC-MS (VIII):

A Zorbax column (SB-AQ, 7µm OBD, 50x150 mm) was used. The two elution solvents were as follows: solvent A = MeCN; solvent B = water + 0.5% Formic acid (25%). The characteristics of the eluting mixture proportion in function of the time t from start of the elution are summarized in the tables below (a linear gradient being used between two consecutive time points):

| t (min) | 0 | 0.3 | 0.8 | 7.5 | 7.7 | 9.5 | 10.0 | 11.5 | 12.0 |
|---|---|---|---|---|---|---|---|---|---|
| Flow (mL/min) | 75 | 75 | 150 | 150 | 150 | 150 | 150 | 150 | 75 |
| Solvent A (%) | 40 | 40 | 40 | 75 | 95 | 95 | 40 | 40 | 40 |
| Solvent B (%) | 60 | 60 | 60 | 25 | 5 | 5 | 60 | 60 | 60 |

### IX) Prep LC-MS (IX):

An Agilent column (Zorbax SB-Aq, 5µm OBD, 30x75 mm) was used. The two elution solvents were as follows: solvent A = water + 0.5% formic acid; solvent B = MeCN. The eluent flow rate was 75 mL/min and the characteristics of the eluting mixture proportion in function of the time t from start of the elution are summarized in the tables below (a linear gradient being used between two consecutive time points):

| t (min) | 0 | 0.01 | 4.0 | 6.0 | 6.2 | 6.6 |
|---|---|---|---|---|---|---|
| Solvent A (%) | 90 | 90 | 5 | 5 | 90 | 90 |
| Solvent B (%) | 10 | 10 | 95 | 95 | 10 | 10 |

### X) Prep LC-MS (X):

A X-Bridge column (Waters C18, 10µm OBD, 50x150 mm) was used. The two elution solvents were as follows: solvent A = water + 0.5% NH₄OH (25%); solvent B = MeCN. The eluent flow rate was 75 mL/min and the characteristics of the eluting mixture proportion in function of the time t from start of the elution are summarized in the tables below (a linear gradient being used between two consecutive time points):

| t (min) | 0 | 0.01 | 3 | 4.0 | 6.0 | 6.2 | 6.6 |
|---|---|---|---|---|---|---|---|
| Solvent A (%) | 95 | 95 | 50 | 5 | 5 | 95 | 95 |
| Solvent B (%) | 5 | 5 | 50 | 95 | 95 | 5 | 5 |

### XI) Prep LC-MS (XI):

X-Bridge column (Waters C18, 10µm OBD, 30x75 mm) was used. The two elution solvents were as follows: solvent A = water + 0.5% NH₄OH (25%); solvent B = MeCN. The eluent flow rate was 75 mL/min and the characteristics of the eluting mixture proportion in function of the time t from start of the elution are summarized in the tables below (a linear gradient being used between two consecutive time points):

| t (min) | 0 | 0.01 | 3.5 | 6.0 | 6.2 | 6.6 |
|---|---|---|---|---|---|---|
| Solvent A (%) | 70 | 70 | 5 | 5 | 70 | 70 |
| Solvent B (%) | 30 | 30 | 95 | 95 | 30 | 30 |

### Preparative chiral SFC and HPLC methods used:

The purifications by preparative chiral SFC and HPLC have been performed using the conditions described hereafter.

### I) Prep chiral HPLC (I):

A ChiralPak AD-H (5µm, 30x250mm) column thermostated at 25°C was used. The elution solvent was Hep/EtOH 80/20, run at a flow rate of 34mL/min.

### II) Prep chiral SFC (II):

A ChiralCel OD-H (5µm, 30x250mm) column thermostated at 40°C was used. The elution solvent was CO₂/MeOH 90/10, run at a flow rate of 160mL/min.

### III) Prep chiral SFC (III):

A ChiralPak AD-H column (5µm, 30x250mm) thermostated at 40°C was used. The elution solvent was CO₂/EtOH 80/20, run at a flow rate of 160mL/min.

### IV) Prep chiral SFC (IV):

A ChiralPak IC column (5µm, 30x250mm) thermostated at 40°C was used. The elution solvent was CO₂/(iPrOH+0.1%DEA) 85/15, run at a flow rate of 160mL/min.

### V) Prep chiral SFC (V)

A ChiralPak IB column (5µm, 30x250mm) thermostated at 40°C was used. The elution solvent was CO₂/MeOH 75/25, run at a flow rate of 160mL/min.

### Preparation of intermediate of Formula A2

### A2.1: 3-(Methoxy-methyl-carbamoyl)-3-methyl-azetidine-1-carboxylic acid tert-butyl ester

To a suspension of 1-Boc-3-methylazetidine-3-carboxylic acid (20g) and N,O-dimethyl hydroxylamine hydrochloride (9.72g) in DCM (270mL) were sequentially added dropwise, DIPEA (54mL) and T3P^{®} (50% in DCM, 55.5mL) while maintaining the temperature at RT with a water bath. The resulting solution was stirred at RT for 1h and quenched with half sat. aq. NaHCO₃. The aq. phase was further extracted with DCM and the combined org. phases were dried over MgSO₄ and concentrated in vacuo. The residue was dried under HV to give the title compound as off-white solid (22.4g). LC-MS (A): t_{R} = 0.79min; [M+H]+: 259.13.

### Preparation of intermediate of Formula A4

### A4.1: 3-Methyl-3-[4-(2,2,2-trifluoro-ethyl)-benzoyl]-azetidine-1-carboxylic acid tert-butyl ester

To a soln of 1-bromo-4-(2,2,2-trifluoroethyl)benzene (1.36g) in anh Et₂O (12mL) under argon and cooled to -78°C was added dropwise tBuLi (1.6M in pentane, 5.17mL) while maintaining the internal temperature below -70°C. The resulting mixture was stirred at -78°C for 5 min and a soln of Weinreb amide A2.1 (1.2g) in anh THF (12mL) was added dropwise while keeping the internal temperature below -70°C. The rxn mixture was stirred for 15min, quenched with water and extracted with EA. The combined org phases were washed with brine, dried over MgSO₄ and evaporated to dryness. The resulting crude material was purified by CC using Sfär Silica D prepacked cartridges from Biotage^{®} and eluting with Hep/EA to give the title compound as white powder (1.445g). LC-MS (A): t_{R} = 1.05min; [M+H]+: 358.07.

### A4.2: 3-(4-Isopropyl-benzoyl)-3-methyl-azetidine-1-carboxylic acid tert-butyl ester

To a soln of 1-bromo-4-isopropylbenzene (10.4g) in anh THF (100mL) under argon and cooled to -78°C was added dropwise nBuLi (2.5M in hexane, 21.2mL) while maintaining the internal temperature below -70°C. The resulting mixture was stirred at -78°C for 20 min. A soln of Weinreb amide A2.1 (10g) in anhydrous THF (50mL) was added dropwise while keeping the internal temperature below -70°C. The resulting soln was allowed to warm to RT and was stirred until completion of the rxn. The rxn mixture was quenched with water and extracted with DCM. The combined org. phases were washed with brine, dried over MgSO₄, and evaporated to dryness. The resulting crude material was purified by CC using Sfär Silica D prepacked cartridges from Biotage^{®} and eluting with Hep/EA to give the title compound as yellow resin (11.7g). LC-MS (A): t_{R} = 1.09min; [M+H]+: 318.31.

### Preparation of intermediates of Formula A6

To a soln of ketone of Formula A4 (1eq) and 5-bromo nicotinonitrile (1.1 to 1.3eq) in anh THF (2.5 to 5.5mL/mmol) under argon and cooled to -78°C was added dropwise HexLi (2.3M in hexane, 1.3 to 1.4eq) (except for intermediate of Formula A6.1 where HexLi was replaced by nBuLi, 2.5M in hexanes) while maintaining the internal temperature below -70°C. The resulting soln was stirred below -70°C until completion of the rxn, quenched with water and extracted with EA. The combined org. phases were washed with brine, dried over MgSO₄, and concentrated in vacuo. The resulting crude was purified using Sfär KP-Amino D and/or Sfär Silica D prepacked cartridges from Biotage^{®} and eluting with Hep/EA. When necessary an additional purification by prep LC-MS using the conditions listed in the table below was performed.

For intermediate of Formula A6.1, a consecutive purification by prep chiral HPLC (I) afforded the title compound as first eluting enantiomer (chiral HPLC (A): t_{R} = 6.76min).

For intermediate of Formula A6.2, a consecutive purification by prep chiral SFC (II) afforded the title compound as second eluting enantiomer (chiral HPLC (B): t_{R} = 2.21min).

| **A6** | **Name** | **Reactant A4** | **Prep LC-MS** | **t_{R} [min]** | **MS-data m/z [M+H]⁺** |
|---|---|---|---|---|---|
| A6.1 | 3-{(R)-(5-Cyano-pyridin-3-yl)-hydroxy-[4-(2,2,2-trifluoro-ethyl)-phenyl]-methyl}-3-methyl-azetidine-1-carboxylic acid tert-butyl ester | A4.1 | (I) | 1.02 | 462.14 |
| A6.2 | 3-[(R)-(5-Cyano-pyridin-3-yl)-hydroxy-(4-isopropylphenyl)-methyl]-3-methyl-azetidine-1-carboxylic acid tert-butyl ester | A4.2 | (VIII) | 1.06 | 422.16 |

### Preparation of intermediates of Formula A7

To a soln of intermediate of Formula A6 (1eq) and hydroxylamine hydrochloride (1.5eq) in DMSO (5.4 mL/mmol) was added dropwise TEA (2eq). The rxn mixture was stirred for 4.5h to 18h at RT and partitioned between EA and water. The org phase was washed with water and brine, dried over MgSO₄, and evaporated to dryness to give the crude title compound.

| **A7** | **Name** | **Reactant A6** | **t_{R} [min]** | **MS-data m/z [M+H]⁺** |
|---|---|---|---|---|
| A7.1 | tert-butyl (R)-3-(hydroxy(5-(N'-hydroxycarbamimidoyl)pyridin-3-yl)(4-(2,2,2-trifluoroethyl)phenyl)methyl)-3-methylazetidine-1-carboxylate | A6.1 | 0.79 | 495.14 |
| A7.2 | tert-butyl (R)-3-(hydroxy(5-(N'-hydroxycarbamimidoyl)pyridin-3-yl)(4-isopropylphenyl)methyl)-3-methylazetidine-1-carboxylate | A6.2 | 0.81 | 455.10 |

### Preparation of intermediates of Formula A8

### A8.1: 3-Hydroxymethyl-bicyclo[1.1.1]pentane-1-carboxylic acid

To a soln of 3-(hydroxymethyl)bicyclo[1.1.1]pentane-1-carbonitrile (300mg) in EtOH (4.6mL) was added water (1mL) and aq. NaOH (10.8M, 1.3mL). The rxn mixture was heated to 75°C and stirred for 1h30. After cooling to RT, it was acidified with aq. HCl (1M) and extracted with EA. The combined org phases were dried over MgSO₄ and evaporated to dryness to give the crude title compound as white solid (268mg). ¹H NMR (500 MHz, DMSO) *δ*: 12.24 (s, 1 H), 4.54 (t, *J =* 5.6 Hz, 1 H), 3.37 (d, *J* = 5.5 Hz, 2 H), 1.77-1.84 (m, 6 H).

### A8.2: trans-4-Propionylamino-cyclohexanecarboxylic acid

### A8.2.1: trans-4-Amino-cyclohexanecarboxylic acid methyl ester

Methyl trans-4-(tert-butoxycarbonylamino)cyclohexanecarboxylate (500 mg) was treated with a solution of HCl in dioxane (4 M, 5 mL). The solution was stirred at RT for 30 min before the solvent was evaporated to give the desired product as a white solid (380 mg). LC-MS (A): t_{R} = 0.34min; [M+H]⁺: 158.15.

### A8.2.2: trans-4-Propionylamino-cyclohexanecarboxylic acid methyl ester

To a suspension of intermediate of Formula A8.2.1 (190 mg) in THF (2 mL) was added propionic anhydride (0.14 mL) and TEA (0.41 mL) and the mixture was stirred at 60 °C for 2 h. The solvent was evaporated, and the residue was purified by prep LC-MS (X) to give the desired product as a white solid (85 mg). LC-MS (A): t_{R} = 0.62min; [M+H]⁺: 214.26.

### A8.2.3 trans-4-Propionylamino-cyclohexanecarboxylic acid

To a suspension of intermediate of Formula A8.2.2 (84 mg) in MeOH (3.5 mL) was added lithium hydroxide monohydrate (31 mg) and the mixture was stirred at 65 °C for 2.5 h. After addition of more lithium hydroxide monohydrate (15 mg), the mixture was stirred at 65 °C for 16 h. The reaction was filtered and concentrated under vacuum. The crude was purified by prep LC-MS (VI) to give the desired product as a white powder (66 mg). LC-MS (A): t_{R} = 0.49min; [M+H]⁺: 200.31.

### A8.3 3-Acetamidobicyclo[1.1.1]pentane-1-carboxylic acid

To a suspension of methyl 3- aminobicyclo[1.1.1]pentane-1-carboxylate (200 mg) in THF (2 mL) was added acetic anhydride (0.12 mL) and TEA (0.47 mL), the reaction mixture was heated to 60 °C and stirred for 1 h. The reaction was diluted with MeCN and water and directly purified by prep LC-MS (X + VI) to give the desired product as a white solid (119 mg). Hydrolysis of the methyl ester seemed to have happened during the purification by prep LC-MS under basic conditions (X). LC-MS (A): t_{R} = 0.38min; [M+H]⁺: 169.99.

### Preparation of intermediates of Formula A9

### Procedure A:

A mixture of intermediate of Formula A7 (1eq), carboxylic acid of Formula A8 (1.5eq), PyBOP (1.5 to 3eq) and TEA (3eq) in DMF (7.5 to 8mL/mmol) was heated to 80°C and stirred for 20h. The rxn mixture was cooled to RT and filtered. The filtrate was directly purified by prep LC-MS using the conditions listed in the table below to afford the title compound.

### Procedure B:

A solution of carboxylic acid of Formula A8 (1.5eq) and CDI (1.5eq) in DMSO (4mL/mmol A7) was stirred at RT for 30 min. The mixture was then transferred to a solution of intermediate of Formula A7 (1eq) in DMSO (1.5mL/mmol). The mixture was stirred at RT for another 30 min, before it was heated to 85°C and stirred for 8 h. The reaction mixture was diluted with MeCN, filtered through as syringe filter and directly purified by prep LC-MS (see method in the table below)

| **A9** | **Name** | **Reactant A7** | **Reactant A8** | **Proced ure** | **Prep LC-MS** | **t_{R} [min]** | **MS-data m/z [M+H]⁺** |
|---|---|---|---|---|---|---|---|
| A9.1 | trans-3-{(R)-Hydroxy-{5-[5-(4-hydroxycyclohexyl)-[1,2,4]oxadiazol-3-yl]-pyridin-3-yl}-[4-(2,2,2-trifluoro-ethyl)-phenyl]-methyl}-3-methyl-azetidine-1-carboxylic acid tert-butyl ester | A7.1 | trans-4-Hydroxy cyclo hexane carboxylic acid | A | (II) | 1.00 | 603.19 |
| A9.2 | 3-{(R)-Hydroxy-{5-[5-(3-hydroxymethyl-bicyclo[1.1.1]pent-1-yl)-[1,2,4]oxadiazol-3-yl]-pyridin-3-yl}-[4-(2,2,2-trifluoro-ethyl)-phenyl]-methyl}-3-methyl-azetidine-1-carboxylic acid tert-butyl ester | A7.1 | A8.1 | A | (II) | 1.01 | 601.16 |
| A9.3 | 3-((R)-Hydroxy-(4-isopropyl-phenyl)-{5-[5-(4-methoxycarbonyl-bicyclo[2.2.2]oct-1-yl)-[1,2,4]oxadiazol-3-yl]-pyridin-3-yl}-methyl)-3-methyl-azetidine-1-carboxylic acid tert-butyl ester | A7.2 | 4-(methoxyca rbonyl)bicyc lo[2.2.2]octa ne-1-carboxylic acid | B | (IV) | 1.21 | 631.35 |
| A9.4 | 3-[(R)-{5-[5-(3-Acetylamino-bicyclo[1.1.1]pent-1-yl)-[1,2,4]oxadiazol-3-yl]-pyridin-3-yl}-hydroxy-(4-isopropyl-phenyl)-methyl]-3-methyl-azetidine-1-carboxylic acid tert-butyl ester | A7.2 | A8.3 | B | (V) | 1.05 | 588.35 |
| A9.5 | 3-[(R)-Hydroxy-{5-[5-(4-hydroxy-bicyclo[2.2.2]oct-1-yl)-[1,2,4]oxadiazol-3-yl]-pyridin-3-yl}-(4-isopropylphenyl)-methyl]-3-methyl-azetidine-1-carboxylic acid tert-butyl ester | A7.2 | 4-hydroxy bicyclo[2.2. 2]octane-1-carboxylic acid | A | (VII) | 1.07 | 589.37 |
| A9.6 | 3-[(R)-{5-[5-(2-Acetyl-2-aza-spiro[3.3]hept-6-yl)-[1,2,4]oxadiazol-3-yl]-pyridin-3-yl}-hydroxy-(4-isopropyl-phenyl)-methyl]-3-methyl-azetidine-1-carboxylic acid tert-butyl ester | A7.2 | 2-acetyl-2-azaspiro[3.3 ]heptane-6-carboxylic acid | B | (III) | 1.10 | 602.32 |
| A9.7 | 3-((R)-Hydroxy-(4-isopropyl-phenyl)-{5-[5-(trans-4-propionylamino-cyclohexyl)-[1,2,4]oxadiazol-3-yl]-pyridin-3-yl}-methyl)-3-methyl-azetidine-1-carboxylic acid tert-butyl ester | A7.2 | A8.2 | B | (II) | 1.09 | 618.36 |
| A9.8 | 3-[(R)-{5-[5-(4-Acetylamino-cyclohexyl)-[1,2,4]oxadiazol-3-yl]-pyridin-3-yl}-hydroxy-(4-isopropyl-phenyl)-methyl]-3-methyl-azetidine-1-carboxylic acid tert-butyl ester | A7.2 | 4-acetamidoc yclohexane-1-carboxylic acid | B | (VII) | 1.07 | 604.46 |
| A9.9 | 3-((R)-Hydroxy-(4-isopropyl-phenyl)-{5-[5-(4-methoxycarbonyl-cyclohexyl)-[1,2,4]oxadiazol-3-yl]-pyridin-3-yl}-methyl)-3-methyl-azetidine-1-carboxylic acid tert-butyl ester | A7.2 | trans-4-(Methoxyca rbonyl)cyclo hexanecarb oxylic acid | B | (XI) | 1.17 | 605.33 |

### Preparation of intermediates of Formula A10

To a soln of intermediate of Formula A9 (1eq) in dioxane (5 to 8mL/mmol) was added HCl (4M in dioxane, 8.9eq) and the rxn mixture was stirred at RT for 18h. When necessary to reach completion of the reaction, additional amounts of HCl (4M in dioxane) were added. The rxn mixture was evaporated to dryness to give the crude hydrochloride salt.

| **A10** | **Name** | **Reactant A9** | **t_{R} [min]** | **MS-data m/z [M+H]⁺** |
|---|---|---|---|---|
| A10.1 | trans-4-[3-(5-{(R)-Hydroxy-(3-methyl-azetidin-3-yl)-[4-(2,2,2-trifluoro-ethyl)-phenyl]-methyl}-pyridin-3-yl)-[1,2,4]oxadiazol-5-yl]-cyclohexanol | A9.1 | 0.69 | 503.10 |
| A10.2 | (R)-{5-[5-(3-Hydroxymethyl-bicyclo[1.1.1]pent-1-yl)-[1,2,4]oxadiazol-3-yl]-pyridin-3-yl}-(3-methyl-azetidin-3-yl)-[4-(2,2,2-trifluoro-ethyl)-phenyl]-methanol | A9.2 | 0.70 | 501.09 |
| A10.3 | 4-(3-{5-[(R)-Hydroxy-(4-isopropyl-phenyl)-(3-methyl-azetidin-3-yl)-methyl]-pyridin-3-yl}-[1,2,4]oxadiazol-5-yl)-bicyclo[2.2.2]octane-1-carboxylic acid methyl ester | A9.3 | 0.86 | 531.30 |
| A10.4 | N-[3-(3-{5-[(R)-Hydroxy-(4-isopropyl-phenyl)-(3-methyl-azetidin-3-yl)-methyl]-pyridin-3-yl}-[1,2,4]oxadiazol-5-yl)-bicyclo[1.1.1]pent-1-yl]-acetamide | A9.4 | 0.72 | 488.34 |
| A10.5 | 4-(3-{5-[(R)-Hydroxy-(4-isopropyl-phenyl)-(3-methyl-azetidin-3-yl)-methyl]-pyridin-3-yl}-[1,2,4]oxadiazol-5-yl)-bicyclo[2.2.2]octan-1-ol | A9.5 | 0.73 | 489.34 |
| A10.6 | 1-[6-(3-{5-[(R)-Hydroxy-(4-isopropyl-phenyl)-(3-methyl-azetidin-3-yl)-methyl]-pyridin-3-yl}-[1,2,4]oxadiazol-5-yl)-2-aza-spiro[3.3]hept-2-yl]-ethanone | A9.6 | 0.74 | 502.35 |
| A10.7 | trans-N-[4-(3-{5-[(R)-Hydroxy-(4-isopropyl-phenyl)-(3-methyl-azetidin-3-yl)-methyl]-pyridin-3-yl}-[1,2,4]oxadiazol-5-yl)-cyclohexyl]-propionamide | A9.7 | 0.77 | 518.39 |
| A10.8 | N-[4-(3-{5-[(R)-Hydroxy-(4-isopropyl-phenyl)-(3-methyl-azetidin-3-yl)-methyl]-pyridin-3-yl}-[1,2,4]oxadiazol-5-yl)-cyclohexyl]-acetamide | A9.8 | 0.73 | 504.43 |
| A10.9 | 4-(3-{5-[(R)-Hydroxy-(4-isopropyl-phenyl)-(3-methyl-azetidin-3-yl)-methyl]-pyridin-3-yl}-[1,2,4]oxadiazol-5-yl)-cyclohexanecarboxylic acid methyl ester | A9.9 | 0.85 | 505.32 |

### Preparation of intermediates of Formula B1

### B1.1: 5-{(R)-(1-tert-Butoxycarbonyl-3-methyl-azetidin-3-yl)-hydroxy-[4-(2,2,2-trifluoro-ethyl)-phenyl]-methyl}-nicotinic acid

To a suspension of intermediate of Formula A6.1 (450 mg) in EtOH (5.75 mL) was added aq. NaOH (1 M, 5.75 mL). The mixture was stirred at 80 °C for 4 h. The reaction was concentrated under vacuum (to half the volume), diluted with water and treated with aq. citric acid (10%). A pH of ~3 was reached, the mixture was extracted with EA (3 times) and the combined organic layers were dried over MgSO₄, filtered and concentrated under vacuum. The residue was suspended in EA and filtered. The filtrate was concentrated to dryness to give the desired product as a white solid (540 mg). LC-MS (A): t_{R} = 0.88min; [M+H]⁺: 480.95.

### Preparation of intermediates of Formula B2

### B2.1: N',3-dihydroxy-2,2-dimethylpropanimidamide

To a solution of 3-hydroxy-2,2-dimethylpropanenitrile (200 mg) in EtOH (14 mL) were added hydroxylamine hydrochloride (425 mg) and K₂CO₃ (1.12 g). The mixture was heated to 85 °C and stirred for 21 h. The reaction was filtered and washed with EtOH. The combined filtrates were concentrated under vacuum, suspended in n-heptane, then concentrated again to give the title compound as a white, slightly sticky solid (280 mg). LC-MS (A): t_{R} = 0.20min; [M+H]⁺: 133.37.

### B2.2: N'-hydroxy-3-(hydroxymethyl)bicyclo[1.1.1]pentane-1-carboximidamide

To a solution of 3-(hydroxymethyl)bicyclo[1.1.1]pentane-1-carbonitrile (250 mg) in EtOH (4.3 mL) were added hydroxylamine hydrochloride (325 mg) and K₂CO₃ (1.07 g). The reaction was heated to 80 °C and stirred for 16 h. After cooling to RT, the reaction mixture was filtered, concentrated and dried under HV to afford the desired product as a white, sticky foam (324 mg). LC-MS (A): t_{R} = 0.22min; [M+H]⁺: 157.19.

### Preparation of intermediates of Formula B3

### B3.1: 3-{(R)-Hydroxy-{5-[3-(2-hydroxy-1,1-dimethyl-ethyl)-[1,2,4]oxadiazol-5-yl]-pyridin-3-yl}-[4-(2,2,2-trifluoro-ethyl)-phenyl]-methyl}-3-methyl-azetidine-1-carboxylic acid tert-butyl ester

To a solution of intermediate of Formula B1.1 (50 mg) in DMF (1 mL) were added DIPEA (40 mg) and PyBOP (83 mg). After stirring at RT for 15 min, a solution of intermediate of Formula B2.1 (28 mg) in DMF (0.3 mL) and K₃PO₄ (90 mg) were added. The mixture was heated at 85 °C for 16 h. The reaction mixture was diluted with MeCN and directly purified by prep LC-MS (VI) to give the desired product as a white solid (34 mg). LC-MS (A): t_{R} = 1.01min; [M+H]⁺: 577.14.

### B3.2: 3-{(R)-Hydroxy-{5-[3-(3-hydroxymethyl-bicyclo[1.1.1]pent-1-yl)-[1,2,4]oxadiazol-5-yl]-pyridin-3-yl}-[4-(2,2,2-trifluoro-ethyl)-phenyl]-methyl}-3-methyl-azetidine-1-carboxylic acid tert-butyl ester

To a solution of intermediate of Formula B1.1 (50 mg) in DMF (1 mL) were added DIPEA (40 mg) and PyBOP (83 mg). After stirring at RT for 20 min, a solution of intermediate of Formula B2.2 (33 mg) in DMF (0.7 mL) and K₃PO₄ (90 mg) were added. The mixture was heated at 85 °C for 16 h. The reaction mixture was diluted with MeCN and directly purified by prep LC-MS (III) + (I) to give the desired product as a white solid (15 mg). LC-MS (A): t_{R} = 0.99min; [M+H]⁺: 601.26.

### Preparation of intermediates of Formula B4

### B4.1: 2-[5-(5-{(R)-Hydroxy-(3-methyl-azetidin-3-yl)-[4-(2,2,2-trifluoro-ethyl)-phenyl]-methyl}-pyridin-3-yl)-[1,2,4]oxadiazol-3-yl]-2-methyl-propan-1-ol

Intermediate of Formula B3.1 (34 mg) was treated with a solution of HCl in dioxane (4 M, 1.0 mL). After stirring at RT for 1 h, the reaction was concentrated to dryness to give the desired product as a yellow foam (39 mg). LC-MS (A): t_{R} = 0.69min; [M+H]⁺: 477.28.

### B4.2: (R)-{5-[3-(3-Hydroxymethyl-bicyclo[1.1.1]pent-1-yl)-[1,2,4]oxadiazol-5-yl]-pyridin-3-yl}-(3-methyl-azetidin-3-yl)-[4-(2,2,2-trifluoro-ethyl)-phenyl]-methanol

Intermediate of Formula B3.2 (15 mg) was treated with a solution of HCl in dioxane (4 M, 0.3 mL). After stirring at RT for 1 h, the reaction was concentrated to dryness to give the desired product as a white solid (14 mg). LC-MS (A): t_{R} = 0.69min; [M+H]⁺: 501.07.

### Preparation of intermediate of Formula C1

### C1.1: 3-[(R)-(5-Bromo-pyridin-3-yl)-hydroxy-(4-isopropyl-phenyl)-methyl]-3-methyl-azetidine-1-carboxylic acid tert-butyl ester

The title compound was synthesized starting from intermediate of Formula A4.2 and 3,5-dibromo-pyridine, and following the procedure described for intermediates of Formula A6. A consecutive chiral separation using prep chiral SFC (III) afforded the title compound as first eluting enantiomer. LC-MS (A): t_{R} = 1.12min; [M+H]⁺: 475.09; chiral SFC (C): t_{R} = 1.41min.

### Preparation of intermediate of Formula C2

### C2.1: (R)-(5-Bromo-pyridin-3-yl)-(4-isopropyl-phenyl)-(3-methyl-azetidin-3-yl)-methanol

The title compound was synthesized starting from intermediate of Formula C1.1 and following the procedure described for intermediates A10. LC-MS (A): t_{R} = 0.74min; [M+H]⁺: 375.02.

### Preparation of intermediate of Formula C3

### C3.1: (R)-(5-Bromo-pyridin-3-yl)-(1,3-dimethyl-azetidin-3-yl)-(4-isopropyl-phenyl)-methanol

The title compound was synthesized starting from intermediate of Formula C2.1 and following the procedure described for Examples 1 to 9 (Procedure A). LC-MS (A): t_{R} = 0.75min; [M+H]⁺: 389.06.

### Preparation of intermediate of Formula C4

### C4.1: (R)- or (S)-2-Pyrimidin-4-yl-but-3-yn-2-ol

### C4.1.1: Pyrimidine-4-carboxylic acid methoxy-methyl-amide

To a suspension of pyrimidine-4-carboxylic acid (500 mg) and in DCM (50mL) were sequentially added N,O-dimethyl hydroxylamine hydrochloride (413mg) DIPEA (2.37mL) and T3P^{®} (50% in DCM, 1.93mL). The resulting solution was stirred at RT for 18h and quenched with sat. aq. NaHCO₃. The aq. phase was further extracted with DCM and the combined org. phases were washed with brine, dried over MgSO₄ and concentrated in vacuo. The resulting crude material was purified by CC using Sfär Silica D prepacked cartridges from Biotage^{®} and eluting with DCM/MeOH to give the title compound as colorless resin (500mg). LC-MS (A): t_{R} = 0.41min; [M+H]⁺: 168.08.

### C4.1.2: 1-Pyrimidin-4-yl-ethanone

To a soln of intermediate of Formula C4.1.1 (500mg) in anh THF (7.5mL) under argon and cooled to -78°C was added dropwise MeMgBr (3M in Et₂O, 2 mL). The rxn mixture was stirred at RT for 15 min, quenched with half sat. aq. NH₄Cl and extracted with DCM. The combined org phases were over MgSO₄ and evaporated to dryness. The resulting crude material was purified by CC using Sfär Silica D prepacked cartridges from Biotage^{®} and eluting with DCM/MeOH to give the title compound as off-white solid (260mg). LC-MS (A): t_{R} = 0.46min; ¹H NMR (400 MHz, CDCl₃) *δ*: 9.39 (s, 1 H), 9.00 (d, *J* = 4.6 Hz, 1 H), 7.92 (d, *J* = 4.7 Hz, 1 H), 2.75 (s, 3 H).

### C4.1.3: (R)- or (S)-2-Pyrimidin-4-yl-but-3-yn-2-ol

To a soln of trimethylsilylacetylene (267mg) in anh THF (3.5mL) under argon and cooled to 0°C was added dropwise HexLi (2.3M in hexanes, 1.16mL) while maintaining the temperature below 5°C. The rxn mixture was stirred at 0°C for 1h and intermediate of Formula C4.1.2 (260mg) in anh THF (1.5mL) was added dropwise at 0°C. The rxn mixture was stirred for 19.5h while allowing temperature to reach slowly RT. It was quenched by addition of MeOH (5mL) and K₂CO₃ (294mg) was added. After stirring at RT for 30 min, the rxn mixture was filtered, diluted with MeOH and water, and purified by prep LC-MS (III). A consecutive chiral separation using prep chiral SFC (IV) afforded the title compound as first eluting enantiomer. LC-MS (A): t_{R} = 0.44 min; [M+H]⁺: 149.14; chiral SFC (D): t_{R} = 1.42 min. The second eluting enantiomer had the following retention time: chiral SFC (D): t_{R} = 1.81 min.

### Preparation of examples

To a soln of intermediate of Formula A10 or B4 (1eq) in anh dioxane (18 to 25 mL/mmol) was added DIPEA (2 to 3 eq), formaldehyde (37% soln in H₂O, 1.5 to 3.5eq) and NaBH(OAc)₃ (1.5 to 2.6 eq). The rxn mixture was stirred for 15 min to 18h at RT, quenched with aq. NaOH (1M) and extracted with EA. The combined org. phases were dried over MgSO₄ and concentrated in vacuo. The resulting crude was purified by prep LC-MS using the conditions listed in the table below.

Example 8 was additionally purified by prep chiral SFC (V) to obtain a single cis or trans isomer. The compound of Example 8 was isolated as the second eluting isomer (chiral SFC (E): t_{R} = 2.23 min). The first eluting isomer had a t_{R} value of 1.84 min.

| **Example N°** | **Name** | **Reactant A10 or B4** | **Prep LC-MS** | **t_{R} [min]** | **MS-data m/z [M+H]⁺** |
|---|---|---|---|---|---|
| **1** | trans-4-[3-(5-{(R)-(1,3-Dimethyl-azetidin-3-yl)-hydroxy-[4-(2,2,2-trifluoro-ethyl)-phenyl]-methyl}-pyridin-3-yl)-[1,2,4]oxadiazol-5-yl]-cyclohexanol | A10.1 | (III) | 0.71 | 517.11 |
| **2** | (R)-(1,3-Dimethyl-azetidin-3-yl)-{5-[5-(3-hydroxymethyl-bicyclo[1.1.1]pent-1-yl)-[1,2,4]oxadiazol-3-yl]-pyridin-3-yl}-[4-(2,2,2-trifluoro-ethyl)-phenyl]-methanol | A10.2 | (III) | 0.71 | 515.09 |
| **3** | N-[3-(3-{5-[(R)-(1,3-Dimethyl-azetidin-3-yl)-hydroxy-(4-isopropyl-phenyl)-methyl]-pyridin-3-yl}-[1,2,4]oxadiazol-5-yl)-bicyclo[1.1.1]pent-1-yl]-acetamide | A10.4 | (II) | 0.74 | 502.36 |
| **4** | 4-(3-{5-[(R)-(1,3-Dimethyl-azetidin-3-yl)-hydroxy-(4-isopropyl-phenyl)-methyl]-pyridin-3-yl}-[1,2,4]oxadiazol-5-yl)-bicyclo[2.2.2]octan-1-ol | A10.5 | (II) + (VI) | 0.75 | 503.41 |
| **5** | 2-[5-(5-{(R)-(1,3-Dimethyl-azetidin-3-yl)-hydroxy-[4-(2,2,2-trifluoro-ethyl)-phenyl]-methyl}-pyridin-3-yl)-[1,2,4]oxadiazol-3-yl]-2-methyl-propan-1-ol | B4.1 | (III) | 0.70 | 491.30 |
| **6** | 1-[6-(3-{5-[(R)-(1,3-Dimethyl-azetidin-3-yl)-hydroxy-(4-isopropyl-phenyl)-methyl]-pyridin-3-yl}-[1,2,4]oxadiazol-5-yl)-2-aza-spiro[3.3]hept-2-yl]-ethanone | A10.6 | (III) | 0.75 | 516.17 |
| **7** | trans-N-[4-(3-{5-[(R)-(1,3-Dimethyl-azetidin-3-yl)-hydroxy-(4-isopropyl-phenyl)-methyl]-pyridin-3-yl}-[1,2,4]oxadiazol-5-yl)-cyclohexyl]-propionamide | A10.7 | (II) | 0.79 | 532.42 |
| **8** | cis- or trans-N-[4-(3-{5-[(R)-(1,3-Dimethyl-azetidin-3-yl)-hydroxy-(4-isopropyl-phenyl)-methyl]-pyridin-3-yl}-[1,2,4]oxadiazol-5-yl)-cyclohexyl]-acetamide | A10.8 | (III) | 0.76 | 518.33 |
| **9** | (R)-(1,3-Dimethyl-azetidin-3-yl)-{5-[3-(3-hydroxymethyl-bicyclo[1.1.1]pent-1-yl)-[1,2,4]oxadiazol-5-yl]-pyridin-3-yl}-[4-(2,2,2-trifluoro-ethyl)-phenyl]-methanol | B4.2 | (VI) + (III) | 0.70 | 515.07 |

A mixture of intermediate of Formula C3.1 (1eq), alkyne of Formula C4 (1.3eq), Cul (0.025eq), tetrakis (triphenylphosphine)palladium(0) (0.1 to 0.4eq) and pyrrolidine (3.5 to 5eq) in anh THF (9.7 to 15.6 mL/mmol) was flushed with argon, heated at 80°C and stirred for 30 min to 1.5h. The rxn mixture was cooled to RT, diluted with MeOH and water, filtered and purified by prep LC-MS using the conditions as indicated in the table below. 4-Ethynylcyclohexan-1-ol was commercially available and was received as 3:7 cis/trans isomeric mixture. After cross-coupling the cis/trans isomers could be separated by prep LC-MS.

| **Example N°** | **Name** | **Reactant C4** | **Prep LC-MS** | **t_{R} [min]** | **MS-data m/z [M+H]⁺** |
|---|---|---|---|---|---|
| **10** | (R)- or (S)-4-{5-[(R)-(1,3-Dimethyl-azetidin-3-yl)-hydroxy-(4-isopropyl-phenyl)-methyl]-pyridin-3-yl}-2-pyrimidin-4-yl-but-3-yn-2-ol | C4.1 | (IX) + (III) | 0.70 | 457.10 |
| **11** | trans-4-{5-[(R)-(1,3-Dimethyl-azetidin-3-yl)-hydroxy-(4-isopropyl-phenyl)-methyl]-pyridin-3-ylethynyl}-cyclohexanol | 4-Ethynylcycl ohexan-1-ol | (II) | 0.72 | 433.03 |
| **12** | cis-4-{5-[(R)-(1,3-Dimethyl-azetidin-3-yl)-hydroxy-(4-isopropyl-phenyl)-methyl]-pyridin-3-ylethynyl}-cyclohexanol | 4-Ethynylcycl ohexan-1-ol | (II) | 0.74 | 433.04 |

### Preparation of Example 13: 4-(3-{5-[(R)-(1,3-Dimethyl-azetidin-3-yl)-hydroxy-(4-isopropyl-phenyl)-methyl]-pyridin-3-yl}-[1,2,4]oxadiazol-5-yl)-bicyclo[2.2.2]octane-1-carboxylic acid amide

### 13.1: 4-(3-{5-[(R)-(1,3-Dimethyl-azetidin-3-yl)-hydroxy-(4-isopropyl-phenyl)-methyl]-pyridin-3-yl}-[1,2,4]oxadiazol-5-yl)-bicyclo[2.2.2]octane-1-carboxylic acid methyl ester

To a suspension of intermediate of Formula A10.3 (350 mg) in dioxane (3.5 mL) was added DIPEA (0.17 mL), aq. formaldehyde (37 wt.-%, 0.13 mL) and NaBH(OAc)₃ (163 mg). The yellow suspension was stirred at RT for 30 min. The mixture was then treated with aq. NaHCO₃ and extracted with EA twice. The combined organic layers were dried over MgSO₄, filtered and concentrated under vacuum to give a yellow oil, which was purified by prep LC-MS (IV) to give the desired product as a white powder (230 mg). LC-MS (A): t_{R} = 0.89min; [M+H]⁺: 545.15.

### 13.2: 4-(3-{5-[(R)-(1,3-Dimethyl-azetidin-3-yl)-hydroxy-(4-isopropyl-phenyl)-methyl]-pyridin-3-yl}-[1,2,4]oxadiazol-5-yl)-bicyclo[2.2.2]octane-1-carboxylic acid

To a solution of intermediate of Formula A13.1 (230 mg) in MeOH (5 mL) was added LiOH.H₂O (35mg) and the mixture was stirred at 65°C for 2 h. Two more additions of LiOH.H₂O (30 and 15 mg, respectively) and further stirring at 65°C for 5 h and 1 h, respectively, were necessary to obtain complete conversion. After cooling to RT, the mixture was filtered and the solvent evaporated. The residue was purified by prep LC-MS (VI) to give the desired product as a white powder (166 mg). LC-MS (A): t_{R} = 0.80min; [M+H]⁺: 531.28.

### 13.3: 4-(3-{5-[(R)-(1,3-Dimethyl-azetidin-3-yl)-hydroxy-(4-isopropyl-phenyl)-methyl]-pyridin-3-yl}-[1,2,4]oxadiazol-5-yl)-bicyclo[2.2.2]octane-1-carboxylic acid amide

To a solution of intermediate of Formula 13.2 (25 mg) and HATU (17 mg) in DMF (1 mL) was added DIPEA (20 mg). After stirring at RT for 5 min, a solution of ammonium chloride (2.8 mg) in DMF (1 mL) was added and the reaction was stirred at RT for 1 h. The mixture was diluted with MeCN and water and directly purified by prep LC-MS (III) to give the desired product as a white powder (7.5 mg). LC-MS (A): t_{R} = 0.74min; [M+H]⁺: 530.11.

### Preparation of Example 14: 4-(3-{5-[(R)-(1,3-Dimethyl-azetidin-3-yl)-hydroxy-(4-isopropyl-phenyl)-methyl]-pyridin-3-yl}-[1,2,4]oxadiazol-5-yl)-cyclohexanecarboxylic acid amide

### 14.1: trans-4-(3-{5-[(R)-(1,3-Dimethyl-azetidin-3-yl)-hydroxy-(4-isopropyl-phenyl)-methyl]-pyridin-3-yl}-[1,2,4]oxadiazol-5-yl)-cyclohexanecarboxylic acid methyl ester

To a suspension of intermediate of Formula A10.9 (45 mg) in dioxane (0.5 mL) was added DIPEA (21 mg), aq. formaldehyde (37 wt.-%, 24 mg) and NaBH(OAc)₃ (27 mg). The yellow suspension was stirred at RT for 30 min. The mixture was then treated with aq. NaHCO₃ and extracted with EA twice. The combined organic layers were dried over MgSO₄, filtered and concentrated under vacuum to give a yellow oil, which was purified by prep LC-MS (XI) to give the desired product as a white powder (28 mg). LC-MS (A): t_{R} = 0.84min; [M+H]⁺: 519.29.

### 14.2: 4-(3-{5-[(R)-(1,3-Dimethyl-azetidin-3-yl)-hydroxy-(4-isopropyl-phenyl)-methyl]-pyridin-3-yl}-[1,2,4]oxadiazol-5-yl)-cyclohexanecarboxylic acid

To a suspension of intermediate of Formula 14.1 (28 mg) in MeOH (0.5 mL) was added LiOH·H₂O (4.5 mg). The mixture was stirred at RT for 16 h. More lithium hydroxide monohydrate (4.5 mg) was added and the mixture was stirred at 85 °C for 4 h. The reaction was filtered, and the solvent was evaporated , the crude was purified by prep LC-MS (VI) to give the desired product as a white powder (21 mg). A mixture of cis and trans isomer was obtained, due to partial isomerisation. LC-MS (A): t_{R} = 0.76min; [M+H]⁺: 505.33.

### 14.3: 4-(3-{5-[(R)-(1,3-Dimethyl-azetidin-3-yl)-hydroxy-(4-isopropyl-phenyl)-methyl]-pyridin-3-yl}-[1,2,4]oxadiazol-5-yl)-cyclohexanecarboxylic acid amide

To a solution of intermediate of Formula 14.2 (21 mg) and HATU (15 mg) in DMF (1 mL) was added DIPEA (17 mg). After stirring at RT for 5 min, a solution of ammonium chloride (2.5 mg) in DMF (1 mL) was added and the reaction was stirred at RT for 1 h. The mixture was diluted with MeCN and water and directly purified by prep LC-MS (III) to give the desired product as a white powder (10 mg). LC-MS (A): t_{R} = 0.72min; [M+H]⁺: 504.36.

### Preparation of Example 15: 4-[3-(5-{(R)-(1,3-Dimethyl-azetidin-3-yl)-hydroxy-[4-(2,2,2-trifluoro-ethyl)-phenyl]-methyl}-pyridin-3-yl)-[1,2,4]oxadiazol-5-yl]-bicyclo[2.2.2]octane-1-carboxylic acid amide

15.1: 5-{(R)-Hydroxy-(3-methyl-azetidin-3-yl)-[4-(2,2,2-trifluoro-ethyl)-phenyl]-methyl}-nicotinonitrile as HCl salt The title compound was synthesized starting from intermediate of Formula A6.1 and following the procedure described for intermediate of Formula B4.1. LC-MS (A): t_{R} = 0.67 min; [M+H]⁺: 361.99.15.2: 5-{(R)-(1,3-Dimethyl-azetidin-3-yl)-hydroxy-[4-(2,2,2-trifluoro-ethyl)-phenyl]-methyl}-nicotinonitrile
The title compound was synthesized starting from intermediate of Formula 15.1 and following the procedure described for intermediates of Formula 14.1. LC-MS (A): t_{R} = 0.68 min; [M+H]⁺: 376.04.

15.3: 5-{(R)-(1,3-Dimethyl-azetidin-3-yl)-hydroxy-[4-(2,2,2-trifluoro-ethyl)-phenyl]-methyl}-N-hydroxy-nicotinamidine The title compound was synthesized starting from intermediate of Formula 15.2 and following the procedure described for intermediates of Formula A7. LC-MS (A): t_{R} = 0.52 min; [M+H]⁺: 409.06.

### 15.4: 4-Carbamoyl-bicyclo[2.2.2]octane-1-carboxylic acid methyl ester

The title compound was synthesized starting from 4-(methoxycarbonyl)bicyclo[2.2.2]octane-1-carboxylic acid and following the procedure described for intermediates of Formula 14.3. LC-MS (A): t_{R} = 0.59 min; [M+H]⁺: 212.13.

### 15.5: 4-Carbamoyl-bicyclo[2.2.2]octane-1-carboxylic acid as lithium salt

To a solution of intermediate 15.4 (175 mg) in THF (2.4 mL), MeOH (0.8 mL), and water (0.8 mL) was added LiOH·H₂O (105 mg) and the reaction mixture was stirred at 50°C for 1 h. Evaporation to dryness gave the crude as white powder (238 mg). LC-MS (A): t_{R} = 0.43 min; [M+H]⁺: 198.18.

### 15.6: 4-[3-(5-{(R)-(1,3-Dimethyl-azetidin-3-yl)-hydroxy-[4-(2,2,2-trifluoro-ethyl)-phenyl]-methyl}-pyridin-3-yl)-[1,2,4]oxadiazol-5-yl]-bicyclo[2.2.2]octane-1-carboxylic acid amide

To a solution of intermediate 15.3 (30 mg) in DMF (1.4 mL) were added intermediate 15.5 (21 mg), DIPEA (40 mg) and HATU (48 mg). After stirring at RT for 40 min, the reaction mixture was heated at 60 °C overnight. The reaction mixture was diluted with EtOAc and washed with 1M NaOH. The org. phase was dried over MgSO₄ and concentrated in vacuo. The crude was purified by prep LC-MS (III) to give the desired product as a white solid (18 mg). LC-MS (A): t_{R} = 0.72 min; [M+H]⁺: 570.12.

### Preparation of Example 16: N-{4-[3-(5-{(R)-(1,3-Dimethyl-azetidin-3-yl)-hydroxy-[4-(2,2,2-trifluoro-ethyl)-phenyl]-methyl}-pyridin-3-yl)-[1,2,4]oxadiazol-5-yl]-trans-cyclohexyl}-acetamide

### 16.1: {4-[3-(5-{(R)-(1,3-Dimethyl-azetidin-3-yl)-hydroxy-[4-(2,2,2-trifluoro-ethyl)-phenyl]-methyl}-pyridin-3-yl)-[1,2,4]oxadiazol-5-yl]-cyclohexyl}-carbamic acid tert-butyl ester

The title compound was synthesized starting from Boc-trans-4-aminocyclohexanecarboxylic acid and intermediate 15.3 and following the procedure described for intermediates of Formula 15.6. LC-MS (A): t_{R} = 0.86 min; [M+H]⁺: 616.16. 16.2: (R)-{5-[5-(trans-4-Amino-cyclohexyl)-[1,2,4]oxadiazol-3-yl]-pyridin-3-yl}-(1,3-dimethyl-azetidin-3-yl)-[4-(2,2,2-trifluoro-ethyl)-phenyl]-methanol

The title compound was synthesized starting from intermediate of Formula A16.1 and following the procedure described for intermediates of Formula B4.1. LC-MS (A): t_{R} = 0.58 min; [M+H]⁺: 516.09.

### 16.3: N-{4-[3-(5-{(R)-(1,3-Dimethyl-azetidin-3-yl)-hydroxy-[4-(2,2,2-trifluoro-ethyl)-phenyl]-methyl}-pyridin-3-yl)-[1,2,4]oxadiazol-5-yl]-trans-cyclohexyl}-acetamide

To a solution of intermediate 16.2 (12 mg) and triethylamine (13 µL) in CH₂Cl₂ was added acetyl chloride (6 µL) at 0°C and the reaction mixture was stirred for 30 min at 0°C and 1 h at rt. The reaction mixture was diluted with CH₂Cl₂ and the org. phase was washed with sat. aq. NaHCO₃ and concentrated in vacuo. The crude was dissolved in MeOH, K₂CO₃ (37 mg) was added and the mixture was stirred overnight at rt. The org. phase was washed with sat. aq. NaHCO₃ and brine, dried over MgSO₄ and concentrated in vacuo to give the title compound as yellowish solid (9 mg). LC-MS (A): t_{R} = 0.73 min; [M+H]⁺: 558.09.

### II. Biological Assays

FLIPR assay: The bioactivity of compounds is tested in a fluorometric imaging plate reader (FLIPR: Molecular Devices) using engineered HEK-293 cells expressing the human CCR6 (GenBank: AY242126). Frozen cells are plated on Poly-L-Lysine precoated 384-well plates 2 days prior to bioassay in DMEM medium supplemented with 10% FCS and 1% Penicillin-Streptomycin. At the day of bioassay, cell supernatant is discarded and cells are dye loaded for 30minutes at room temperature in the dark with Fluo-8-AM (Focus Biomolecules) in Hanks Balanced Salt Solution (Gibco), buffered with 20mM Hepes at pH 6.75 and supplemented with 0.05 % BSA. This buffer, but lacking the dye, is also used for washing and compound dilution steps (assay buffer). Cells are washed free of excess dye with a wash-station (Biotek), leaving 40 microliter of assay buffer at the end. Cells were incubated for 15minutes at room temperature in the dark, before adding compounds. Stock solutions of test compounds are made up at a concentration of 10mM in DMSO, and serially diluted first in DMSO and then transferred in assay buffer to concentrations required for inhibition dose response curves. After a 45minute incubation period in assay buffer at room temperature, 10 microliters of each compound dilution are transferred from a compound plate to the plate containing the recombinant cells in the FLIPR instrument according to the manufacturer's instructions. After cells and compounds were preincubated for 30minutes at room temperature in the dark, 10 microliter agonist CCL20 (Peprotech) at a final concentration of 10 nM is added, again using the FLIPR instrument. Changes in fluorescence are monitored before and after addition of the test compounds and agonist. Emission peak values above base level after CCL20 addition are exported after base line subtraction. The calculated IC₅₀ values may fluctuate depending on the daily assay performance. Fluctuations of this kind are known to those skilled in the art. In the case where IC₅₀ values have been determined several times for the same compound, mean values are given. Data are shown in the table below.

| **Example N°** | **FLIPR IC₅₀ (nM)** | **Example N°** | **FLIPR IC₅₀ (nM)** | **Example N°** | **FLIPR IC₅₀ (nM)** | **Example N°** | **FLIPR IC₅₀ (nM)** |
|---|---|---|---|---|---|---|---|
| **1** | 83.0 | **5** | 144 | **9** | 146 | **13** | 37.5 |
| **2** | 140 | **6** | 111 | **10** | 71.5 | **14** | 46.3 |
| **3** | 55.7 | **7** | 78.8 | **11** | 28.7 | **15** | 65.3 |
| **4** | 23.9 | **8** | 52.2 | **12** | 25.4 | **16** | 226 |

## Claims

1. A compound of Formula (I) 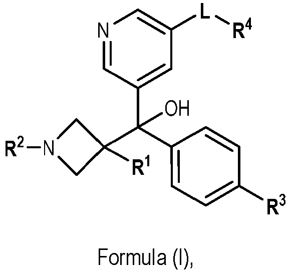 wherein
• **R¹** represents C₁₋₃-alkyl;
**R²** represents C₁₋₄-alkyl;
**R³** represents 2,2,2-trifluoro-ethyl;
**-L-** represents
➢ *-C≡C-C₀₋₂-alkylene-**, optionally mono-substituted with hydroxy; wherein the asterisk (*) denotes the attachment point to the pyridinyl ring and two asterisks (**) denote the attachment point to R⁴; or
➢ oxadiazol-diyl; and
**R⁴** represents
➢ C₃₋₇-cycloalkyl;
➢ saturated 5- to 8-membered bridged or spiro bicyclic hydrocarbon ring system, wherein one ring carbon atom is optionally replaced with a nitrogen atom; or
➢ 6-membered heteroaryl containing one or two ring nitrogen atoms;
wherein R⁴ independently is unsubstituted or mono-substituted with hydroxy, hydroxy-C₁₋₃-alkyl, carbamoyl, C₁₋₃-alkyl-carbonyl-amino, or C₁₋₃-alkyl-carbonyl; or
• **R¹** represents C₁₋₃-alkyl;
**R²** represents C₁₋₄-alkyl;
**R³** represents isopropyl; and
**-L-R⁴** represents 2-(4-hydroxy-cyclohexyl)-ethyn-1-yl, 3-hydroxy-3-(pyrimidin-4-yl)-but-1-yn-1-yl, 5-(4-carbamoyl-cyclohexyl)-1,2,4-oxadiazol-3-yl, 5-(4-hydroxy-bicyclo[2.2.2]octan-1-yl)-1,2,4-oxadiazol-3-yl, 5-(3-acetamido-bicyclo[1.1.1]pentan-1-yl)-1,2,4-oxadiazol-3-yl, 5-(2-acetyl-2-azaspiro[3.3]heptan-6-yl)-1,2,4-oxadiazol-3-yl, 5-(4-acetamido-cyclohexyl)-1,2,4-oxadiazol-3-yl, 5-(4-(ethyl-carbonyl-amino)-cyclohexyl)-1,2,4-oxadiazol-3-yl, or 5-(4-carbamoyl-bicyclo[2.2.2]octan-1-yl)-1,2,4-oxadiazol-3-yl; or
• **R¹** represents C₁₋₃-alkyl;
**R²** represents C₁₋₄-alkyl;
**R³** represents 2,2,2-trifluoro-ethyl; and
**-L-R⁴** represents 3-(2-hydroxy-1,1-dimethyl-ethyl)-1,2,4-oxadiazol-5-yl;
or a pharmaceutically acceptable salt thereof.

2. A compound according to claim 1, wherein
• **R¹** represents C₁₋₃-alkyl;
**R²** represents C₁₋₄-alkyl;
**R³** represents 2,2,2-trifluoro-ethyl;
**-L-** represents
➢ *-C≡C-C₀₋₂-alkylene-**, optionally mono-substituted with hydroxy; wherein the asterisk (*) denotes the attachment point to the pyridinyl ring and two asterisks (**) denote the attachment point to **R⁴;** or
➢ oxadiazol-diyl; and
**R⁴** represents
➢ mono-substituted C₃₋₇-cycloalkyl, wherein the substituent is selected from hydroxy, carbamoyl, and C₁₋₃-alkyl-carbonyl-amino;
➢ saturated 5- to 8-membered bridged or spiro bicyclic hydrocarbon ring system, wherein one ring carbon atom is optionally replaced with a nitrogen atom, wherein said 5- to 8-membered bridged or spiro bicyclic hydrocarbon ring system independently is mono-substituted with hydroxy, hydroxy-C₁₋₃-alkyl, carbamoyl, C₁₋₃-alkyl-carbonyl-amino, or C₁₋₃-alkyl-carbonyl; or
➢ unsubstituted 6-membered heteroaryl containing one or two ring nitrogen atoms; or
• **R¹** represents C₁₋₃-alkyl;
**R²** represents C₁₋₄-alkyl;
**R³** represents isopropyl; and
**-L-R⁴** represents 2-(4-hydroxy-cyclohexyl)-ethyn-1-yl, 3-hydroxy-3-(pyrimidin-4-yl)-but-1-yn-1-yl, 5-(4-carbamoyl-cyclohexyl)-1,2,4-oxadiazol-3-yl, 5-(4-hydroxy-bicyclo[2.2.2]octan-1-yl)-1,2,4-oxadiazol-3-yl, 5-(3-acetamido-bicyclo[1.1.1]pentan-1-yl)-1,2,4-oxadiazol-3-yl, 5-(2-acetyl-2-azaspiro[3.3]heptan-6-yl)-1,2,4-oxadiazol-3-yl, 5-(4-acetamido-cyclohexyl)-1,2,4-oxadiazol-3-yl, 5-(4-(ethyl-carbonyl-amino)-cyclohexyl)-1,2,4-oxadiazol-3-yl, or 5-(4-carbamoyl-bicyclo[2.2.2]octan-1-yl)-1,2,4-oxadiazol-3-yl; or
• **R¹** represents C₁₋₃-alkyl;
**R²** represents C₁₋₄-alkyl;
**R³** represents 2,2,2-trifluoro-ethyl; and
**-L-R⁴** represents 3-(2-hydroxy-1,1-dimethyl-ethyl)-1,2,4-oxadiazol-5-yl;
or a pharmaceutically acceptable salt thereof.

3. A compound according to claim 1, wherein
• **R¹** represents C₁₋₃-alkyl;
**R²** represents C₁₋₄-alkyl;
**R³** represents 2,2,2-trifluoro-ethyl;
**-L-** represents
➢ *-C≡C-C₀₋₂-alkylene-**, optionally mono-substituted with hydroxy; wherein the asterisk (*) denotes the attachment point to the pyridinyl ring and two asterisks (**) denote the attachment point to **R⁴**);and
**R⁴** represents
➢ mono-substituted C₃₋₇-cycloalkyl, wherein the substituent is hydroxy; or
➢ unsubstituted 6-membered heteroaryl containing one or two ring nitrogen atoms; or
• **R¹** represents C₁₋₃-alkyl;
**R²** represents C₁₋₄-alkyl;
**R³** represents 2,2,2-trifluoro-ethyl;
**-L-** represents
➢ oxadiazol-diyl; and
**R⁴** represents
➢ mono-substituted C₃₋₇-cycloalkyl, wherein the substituent is selected from hydroxy, carbamoyl, and C₁₋₃-alkyl-carbonyl-amino; or
➢ saturated 5- to 8-membered bridged or spiro bicyclic hydrocarbon ring system, wherein one ring carbon atom is optionally replaced with a nitrogen atom, wherein said 5- to 8-membered bridged or spiro bicyclic hydrocarbon ring system independently is mono-substituted with hydroxy, hydroxy-C₁₋₃-alkyl, carbamoyl, C₁₋₃-alkyl-carbonyl-amino, or C₁₋₃-alkyl-carbonyl; or
• **R¹** represents C₁₋₃-alkyl;
**R²** represents C₁₋₄-alkyl;
**R³** represents isopropyl; and
**-L-R⁴** represents 2-(4-hydroxy-cyclohexyl)-ethyn-1-yl, 3-hydroxy-3-(pyrimidin-4-yl)-but-1-yn-1-yl, 5-(4-carbamoyl-cyclohexyl)-1,2,4-oxadiazol-3-yl, 5-(4-hydroxy-bicyclo[2.2.2]octan-1-yl)-1,2,4-oxadiazol-3-yl, 5-(3-acetamido-bicyclo[1.1.1]pentan-1-yl)-1,2,4-oxadiazol-3-yl, 5-(2-acetyl-2-azaspiro[3.3]heptan-6-yl)-1,2,4-oxadiazol-3-yl, 5-(4-acetamido-cyclohexyl)-1,2,4-oxadiazol-3-yl, 5-(4-(ethyl-carbonyl-amino)-cyclohexyl)-1,2,4-oxadiazol-3-yl, or 5-(4-carbamoyl-bicyclo[2.2.2]octan-1-yl)-1,2,4-oxadiazol-3-yl; or
• **R¹** represents C₁₋₃-alkyl;
**R²** represents C₁₋₄-alkyl;
**R³** represents 2,2,2-trifluoro-ethyl; and
**-L-R⁴** represents 3-(2-hydroxy-1,1-dimethyl-ethyl)-1,2,4-oxadiazol-5-yl;
or a pharmaceutically acceptable salt thereof.

4. A compound according to any one of claims 1 to 3, wherein **R¹** represents methyl; or a pharmaceutically acceptable salt thereof.

5. A compound according to any one of claims 1 to 4, wherein **R²** represents methyl; or a pharmaceutically acceptable salt thereof.

6. A compound according to claim 1, wherein
• **R¹** represents methyl;
**R²** represents methyl;
**R³** represents 2,2,2-trifluoro-ethyl; and
**-L-R⁴** represents 5-(4-hydroxy-cyclohexyl)-1,2,4-oxadiazol-3-yl, 5-(3-hydroxymethyl-bicyclo[1.1.1]pentan-1-yl)-1,2,4-oxadiazol-3-yl, 3-(3-hydroxymethyl-bicyclo[1.1.1]pentan-1-yl)-1,2,4-oxadiazol-5-yl, 2-(4-hydroxycyclohexyl)-ethyn-1-yl, 3-hydroxy-3-(pyrimidin-4-yl)-but-1-yn-1-yl, 5-(4-carbamoyl-cyclohexyl)-1,2,4-oxadiazol-3-yl, 5-(4-hydroxy-bicyclo[2.2.2]octan-1-yl)-1,2,4-oxadiazol-3-yl, 5-(3-acetamido-bicyclo[1.1.1]pentan-1-yl)-1,2,4-oxadiazol-3-yl, 5-(2-acetyl-2-azaspiro[3.3]heptan-6-yl)-1,2,4-oxadiazol-3-yl, 5-(4-acetamido-cyclohexyl)-1,2,4-oxadiazol-3-yl, 5-(4-(ethyl-carbonyl-amino)-cyclohexyl)-1,2,4-oxadiazol-3-yl, or 5-(4-carbamoyl-bicyclo[2.2.2]octan-1-yl)-1,2,4-oxadiazol-3-yl; or
• **R¹** represents methyl;
**R²** represents methyl;
**R³** represents isopropyl; and
**-L-R⁴** represents 2-(4-hydroxy-cyclohexyl)-ethyn-1-yl, 3-hydroxy-3-(pyrimidin-4-yl)-but-1-yn-1-yl, 5-(4-carbamoyl-cyclohexyl)-1,2,4-oxadiazol-3-yl, 5-(4-hydroxy-bicyclo[2.2.2]octan-1-yl)-1,2,4-oxadiazol-3-yl, 5-(3-acetamido-bicyclo[1.1.1]pentan-1-yl)-1,2,4-oxadiazol-3-yl, 5-(2-acetyl-2-azaspiro[3.3]heptan-6-yl)-1,2,4-oxadiazol-3-yl, 5-(4-acetamido-cyclohexyl)-1,2,4-oxadiazol-3-yl, 5-(4-(ethyl-carbonyl-amino)-cyclohexyl)-1,2,4-oxadiazol-3-yl, or 5-(4-carbamoyl-bicyclo[2.2.2]octan-1-yl)-1,2,4-oxadiazol-3-yl; or
• **R¹** represents methyl;
**R²** represents methyl;
**R³** represents 2,2,2-trifluoro-ethyl; and
**-L-R⁴** represents 3-(2-hydroxy-1,1-dimethyl-ethyl)-1,2,4-oxadiazol-5-yl;
or a pharmaceutically acceptable salt thereof.

7. A compound according to claim 1, wherein
**R¹** represents methyl;
**R²** represents methyl;
**R³** represents isopropyl; and
**-L-R⁴** represents 2-(4-hydroxy-cyclohexyl)-ethyn-1-yl, 3-hydroxy-3-(pyrimidin-4-yl)-but-1-yn-1-yl, 5-(4-carbamoyl-cyclohexyl)-1,2,4-oxadiazol-3-yl, 5-(4-hydroxy-bicyclo[2.2.2]octan-1-yl)-1,2,4-oxadiazol-3-yl, 5-(3-acetamido-bicyclo[1.1.1]pentan-1-yl)-1,2,4-oxadiazol-3-yl, 5-(2-acetyl-2-azaspiro[3.3]heptan-6-yl)-1,2,4-oxadiazol-3-yl, 5-(4-acetamido-cyclohexyl)-1,2,4-oxadiazol-3-yl, 5-(4-(ethyl-carbonyl-amino)-cyclohexyl)-1,2,4-oxadiazol-3-yl, or 5-(4-carbamoyl-bicyclo[2.2.2]octan-1-yl)-1,2,4-oxadiazol-3-yl;
or a pharmaceutically acceptable salt thereof.

8. A compound according to claim 1, wherein
**R¹** represents methyl;
**R²** represents methyl;
**R³** represents isopropyl; and
**-L-R⁴** represents 5-(4-acetamido-cyclohexyl)-1,2,4-oxadiazol-3-yl;
or a pharmaceutically acceptable salt thereof.

9. A compound according to any one of claims 1 to 8, wherein the asymmetric carbon atom bearing the hydroxy group has the absolute configuration depicted in Formula (II) 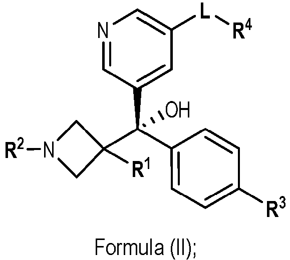 or a pharmaceutically acceptable salt thereof.

10. A compound according to claim 1, which is
trans-4-[3-(5-{(R)-(1,3-Dimethyl-azetidin-3-yl)-hydroxy-[4-(2,2,2-trifluoro-ethyl)-phenyl]-methyl}-pyridin-3-yl)-[1,2,4]oxadiazol-5-yl]-cyclohexanol;
cis-4-[3-(5-{(R)-(1,3-Dimethyl-azetidin-3-yl)-hydroxy-[4-(2,2,2-trifluoro-ethyl)-phenyl]-methyl}-pyridin-3-yl)-[1,2,4]oxadiazol-5-yl]-cyclohexanol;
(R)-(1,3-Dimethyl-azetidin-3-yl)-{5-[5-(3-hydroxymethyl-bicyclo[1.1.1]pent-1-yl)-[1,2,4]oxadiazol-3-yl]-pyridin-3-yl}-[4-(2,2,2-trifluoro-ethyl)-phenyl]-methanol;
N-[3-(3-{5-[(R)-(1,3-Dimethyl-azetidin-3-yl)-hydroxy-(4-isopropyl-phenyl)-methyl]-pyridin-3-yl}-[1,2,4]oxadiazol-5-yl)-bicyclo[1.1.1]pent-1-yl]-acetamide;
4-(3-{5-[(R)-(1,3-Dimethyl-azetidin-3-yl)-hydroxy-(4-isopropyl-phenyl)-methyl]-pyridin-3-yl}-[1,2,4]oxadiazol-5-yl)-bicyclo[2.2.2]octan-1-ol;
2-[5-(5-{(R)-(1,3-Dimethyl-azetidin-3-yl)-hydroxy-[4-(2,2,2-trifluoro-ethyl)-phenyl]-methyl}-pyridin-3-yl)-[1,2,4]oxadiazol-3-yl]-2-methyl-propan-1-ol;
1-[6-(3-{5-[(R)-(1,3-Dimethyl-azetidin-3-yl)-hydroxy-(4-isopropyl-phenyl)-methyl]-pyridin-3-yl}-[1,2,4]oxadiazol-5-yl)-2-aza-spiro[3.3]hept-2-yl]-ethanone;
trans-N-[4-(3-{5-[(R)-(1,3-Dimethyl-azetidin-3-yl)-hydroxy-(4-isopropyl-phenyl)-methyl]-pyridin-3-yl}-[1,2,4]oxadiazol-5-yl)-cyclohexyl]-propionamide;
cis-N-[4-(3-{5-[(R)-(1,3-Dimethyl-azetidin-3-yl)-hydroxy-(4-isopropyl-phenyl)-methyl]-pyridin-3-yl}-[1,2,4]oxadiazol-5-yl)-cyclohexyl]-propionamide;
cis-N-[4-(3-{5-[(R)-(1,3-Dimethyl-azetidin-3-yl)-hydroxy-(4-isopropyl-phenyl)-methyl]-pyridin-3-yl}-[1,2,4]oxadiazol-5-yl)-cyclohexyl]-acetamide;
trans-N-[4-(3-{5-[(R)-(1,3-Dimethyl-azetidin-3-yl)-hydroxy-(4-isopropyl-phenyl)-methyl]-pyridin-3-yl}-[1,2,4]oxadiazol-5-yl)-cyclohexyl]-acetamide;
(R)-(1,3-Dimethyl-azetidin-3-yl)-{5-[3-(3-hydroxymethyl-bicyclo[1.1.1]pent-1-yl)-[1,2,4]oxadiazol-5-yl]-pyridin-3-yl}-[4-(2,2,2-trifluoro-ethyl)-phenyl]-methanol;
(R)-4-{5-[(R)-(1,3-Dimethyl-azetidin-3-yl)-hydroxy-(4-isopropyl-phenyl)-methyl]-pyridin-3-yl}-2-pyrimidin-4-yl-but-3-yn-2-ol;
(S)-4-{5-[(R)-(1,3-Dimethyl-azetidin-3-yl)-hydroxy-(4-isopropyl-phenyl)-methyl]-pyridin-3-yl}-2-pyrimidin-4-yl-but-3-yn-2-ol;
trans-4-{5-[(R)-(1,3-Dimethyl-azetidin-3-yl)-hydroxy-(4-isopropyl-phenyl)-methyl]-pyridin-3-ylethynyl}-cyclohexanol;
cis-4-{5-[(R)-(1,3-Dimethyl-azetidin-3-yl)-hydroxy-(4-isopropyl-phenyl)-methyl]-pyridin-3-ylethynyl}-cyclohexanol; 4-(3-{5-[(R)-(1,3-Dimethyl-azetidin-3-yl)-hydroxy-(4-isopropyl-phenyl)-methyl]-pyridin-3-yl}-[1,2,4]oxadiazol-5-yl)-
bicyclo[2.2.2]octane-1-carboxylic acid amide;
cis-4-(3-{5-[(R)-(1,3-Dimethyl-azetidin-3-yl)-hydroxy-(4-isopropyl-phenyl)-methyl]-pyridin-3-yl}-[1,2,4]oxadiazol-5-yl)-cyclohexanecarboxylic acid amide; or
trans-4-(3-{5-[(R)-(1,3-Dimethyl-azetidin-3-yl)-hydroxy-(4-isopropyl-phenyl)-methyl]-pyridin-3-yl}-[1,2,4]oxadiazol-5-yl)-cyclohexanecarboxylic acid amide;
or a pharmaceutically acceptable salt thereof.

11. A compound according to claim 1, which is
cis-N-[4-(3-{5-[(R)-(1,3-Dimethyl-azetidin-3-yl)-hydroxy-(4-isopropyl-phenyl)-methyl]-pyridin-3-yl}-[1,2,4]oxadiazol-5-yl)-cyclohexyl]-acetamide; or a pharmaceutically acceptable salt thereof.

12. A compound according to claim 1, which is
trans-N-[4-(3-{5-[(R)-(1,3-Dimethyl-azetidin-3-yl)-hydroxy-(4-isopropyl-phenyl)-methyl]-pyridin-3-yl}-[1,2,4]oxadiazol-5-yl)-cyclohexyl]-acetamide; or a pharmaceutically acceptable salt thereof.

13. A pharmaceutical composition comprising a compound according to any one of claims 1 to 12, or a pharmaceutically acceptable salt thereof, further comprising at least one pharmaceutically acceptable carrier.

14. A compound according to any one of claims 1 to 12, or a pharmaceutically acceptable salt thereof, for use as a medicament.

15. A compound according to any one of claims 1 to 12, or a pharmaceutically acceptable salt thereof, for use in the prevention or treatment of inflammatory/autoimmune diseases, conditions, or disorders.

16. A compound according to any one of claims 1 to 12, or a pharmaceutically acceptable salt thereof, for use in the prevention or treatment of cancer.

17. A compound according to any one of claims 1 to 12, or a pharmaceutically acceptable salt thereof, for use according to claim 15, in the prevention or treatment of rheumatoid arthritis; ankylosing spondylitis; spondyloarthritis; psoriasis; psoriatic arthritis; inflammatory skin disorders; rosacea; Crohn's disease; ulcerative colitis; irritable bowel disease; inflammatory bowel disease; dry eye disease; multiple sclerosis; systemic lupus erythematosus; Sjögren's disease; autoimmune hepatitis; primary sclerosing cholangitis; psoriasis; plaque psoriasis; guttate psoriasis; inverse psoriasis; pustular psoriasis; erythrodermic psoriasis; autoimmune keratitis; filamentary keratitis; autoimmune uveitis; allergic conjunctivitis; asthma; allergic disease of the gastrointestinal tract; type 1 diabetes; endometriosis; meibomian gland dysfunction; or graft-versus host disease.

18. A compound according to any one of claims 1 to 12, or a pharmaceutically acceptable salt thereof, for use according to claim 16, in the prevention or treatment of lymphoma; T cell lymphoma; primary mediastinal B-cell lymphoma; brain cancer; glioma; glioblastoma; breast cancer; triple negative breast cancer; colorectal cancer; hepatocarcinoma; renal cell carcinoma; lung cancer; non-small cell lung cancer; small cell lung cancer; gastric cancer; melanoma; Merkel cell carcinoma; cutaneous squamous cell carcinoma; malignant melanoma; bladder cancer; head and neck cancer; squamous cell head and neck carcinoma; Hodgkin's lymphoma; cervical cancer; endometrial cancer; colon cancer; gastrointestinal stromal tumors; pancreatic cancer; prostatic cancer; leukemia; acute myeloid leukemia; ovarian cancer; oesophageal carcinomas; mesothelioma; neuroblastoma; sarcoma; high-grade osteosarcoma; astrocytoma; myeloma; urothelial cancer; locally advanced and metastatic urothelial cancer; MSI-H or dMMR cancer; rectal cancer; laryngeal cancer; salivary adenocarcinoma; multiple myeloma; cholangiocarcinoma; oral squamous cell carcinoma; thyroid cancer; or esophagogastric junction cancer.

19. A compound according to any one of claims 1 to 12, or a pharmaceutically acceptable salt thereof, for use according to claim 15, in the prevention or treatment of inflammatory/autoimmune diseases/disorders selected from psoriasis; psoriatic arthritis; rheumatoid arthritis; ankylosing spondylitis; spondyloarthritis; inflammatory skin disorders including rosacea; Crohn's disease; ulcerative colitis; irritable bowel disease; dry eye disease; multiple sclerosis; systemic lupus erythematosus; Sjögren's disease; autoimmune hepatitis; and primary sclerosing cholangitis.

20. A compound according to any one of claims 1 to 12, or a pharmaceutically acceptable salt thereof, for use according to claim 16, in the prevention or treatment of cancer selected from lymphoma including T cell lymphoma; brain cancer including glioma or glioblastoma; breast cancer; colorectal cancer; hepatocarcinomas; renal cell carcinoma; lung cancer; and gastric cancer.

## Patentansprüche

1. Verbindung von Formel (I) 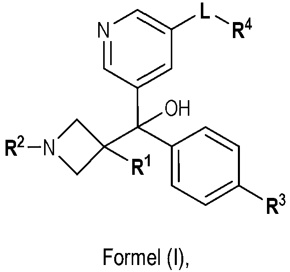 wobei
• **R¹** C₁₋₃-Alkyl repräsentiert;
**R²** C₁₋₄-Alkyl repräsentiert;
**R³** 2,2,2-Trifluorethyl repräsentiert;
**-L-** Folgendes repräsentiert:
➢ *-C≡C-C₀₋₂-Alkylen-**, optional mit Hydroxy monosubstituiert; wobei das Sternchen (*) den Anlagerungspunkt an den Pyridinylring bezeichnet und zwei Sternchen (**) den Anlagerungspunkt an **R⁴** bezeichnen; oder
> Oxadiazol-diyl; und
**R⁴** Folgendes repräsentiert:
> C₃₋₇-Cycloalkyl;
> gesättigtes 5- bis 8-gliedriges verbrücktes oder spiro-bicyclisches Kohlenwasserstoffringsystem, wobei ein Ringkohlenstoffatom optional durch ein Stickstoffatom ersetzt ist; oder
> 6-gliedriges Heteroaryl, das ein oder zwei Ringstickstoffatome enthält;
wobei **R⁴** unabhängig unsubstituiert oder mit Hydroxy, Hydroxy-C₁₋₃-alkyl, Carbamoyl, C₁₋₃-Alkyl-carbonyl-amino oder C₁₋₃-Alkyl-carbonyl monosubstitiert ist; oder
• **R¹** C₁₋₃-Alkyl repräsentiert;
**R²** C₁₋₄-Alkyl repräsentiert;
**R³** Isopropyl repräsentiert; und
**-L-R⁴** 2-(4-Hydroxy-cyclohexyl)-ethyn-1-yl, 3-Hydroxy-3-(pyrimidin-4-yl)-but-1-yn-1-yl, 5-(4-Carbamoyl-cyclohexyl)-1,2,4-oxadiazol-3-yl, 5-(4-Hydroxy-bicyclo[2.2.2]octan-1-yl)-1,2,4-oxadiazol-3-yl, 5-(3-Acetamido-bicyclo[1.1.1]pentan-1-yl)-1,2,4-oxadiazol-3-yl, 5-(2-Acetyl-2-azaspiro[3.3]heptan-6-yl)-1,2,4-oxadiazol-3-yl, 5-(4-Acetamido-cyclohexyl)-1,2,4-oxadiazol-3-yl, 5-(4-(Ethyl-carbonyl-amino)-cyclohexyl)-1,2,4-oxadiazol-3-yl oder 5-(4-Carbamoyl-bicyclo[2.2.2]octan-1-yl)-1,2,4-oxadiazol-3-yl repräsentiert; oder
• **R¹** C₁₋₃-Alkyl repräsentiert;
**R²** C₁₋₄-Alkyl repräsentiert;
**R³** 2,2,2-Trifluorethyl repräsentiert; und
**-L-R⁴** 3-(2-Hydroxy-1,1-dimethyl-ethyl)-1,2,4-oxadiazol-5-yl repräsentiert;
oder ein pharmazeutisch akzeptables Salz davon.

2. Verbindung nach Anspruch 1, wobei
• **R¹** C₁₋₃-Alkyl repräsentiert;
**R²** C₁₋₄-Alkyl repräsentiert;
**R³** 2,2,2-Trifluorethyl repräsentiert;
**-L-** Folgendes repräsentiert:
> *-C≡C-C₀₋₂-Alkylen-**, optional mit Hydroxy monosubstituiert; wobei das Sternchen (*) den Anlagerungspunkt an den Pyridinylring bezeichnet und zwei Sternchen (**) den Anlagerungspunkt an R⁴ bezeichnen; oder
> Oxadiazol-diyl; und
**R⁴** Folgendes repräsentiert:
> monosubstituiertes C₃₋₇-Cycloalkyl, wobei der Substituent aus Hydroxy, Carbamoyl und C₁₋₃-Alkylcarbonylamino ausgewählt ist;
> gesättigtes 5- bis 8-gliedriges verbrücktes oder spiro-bicyclisches Kohlenwasserstoffringsystem, wobei ein Ringkohlenstoffatom optional durch ein Stickstoffatom ersetzt ist, wobei das genannte 5-bis 8-gliedrige verbrückte oder spiro-bicyclische Kohlenwasserstoffringsystem unabhängig mit Hydroxy, Hydroxy-C₁₋₃-alkyl, Carbamoyl, C₁₋₃-Alkylcarbonylamino oder C₁₋₃-Alkylcarbonyl substituiert ist; oder
> unsubstituiertes 6-gliedriges Heteroaryl, das ein oder zwei Ringstickstoffatome enthält; oder
• **R¹** C₁₋₃-Alkyl repräsentiert;
**R²** C₁₋₄-Alkyl repräsentiert;
**R³** Isopropyl repräsentiert; und
**-L-R⁴** 2-(4-Hydroxy-cyclohexyl)-ethyn-1-yl, 3-Hydroxy-3-(pyrimidin-4-yl)-but-1-yn-1-yl, 5-(4-Carbamoyl-cyclohexyl)-1,2,4-oxadiazol-3-yl, 5-(4-Hydroxy-bicyclo[2.2.2]octan-1-yl)-1,2,4-oxadiazol-3-yl, 5-(3-Acetamido-bicyclo[1.1.1]pentan-1-yl)-1,2,4-oxadiazol-3-yl, 5-(2-Acetyl-2-azaspiro[3.3]heptan-6-yl)-1,2,4-oxadiazol-3-yl, 5-(4-acetamido-cyclohexyl)-1,2,4-oxadiazol-3-yl, 5-(4-(Ethyl-carbonyl-amino)-cyclohexyl)-1,2,4-oxadiazol-3-yl oder 5-(4-carbamoyl-bicyclo[2.2.2]octan-1-yl)-1,2,4-oxadiazol-3-yl repräsentiert; oder
• **R¹** C₁₋₃-Alkyl repräsentiert;
**R²** C₁₋₄-Alkyl repräsentiert;
**R³** 2,2,2-Trifluorethyl repräsentiert; und
**-L-R⁴** 3-(2-Hydroxy-1,1-dimethyl-ethyl)-1,2,4-oxadiazol-5-yl repräsentiert;
oder ein pharmazeutisch akzeptables Salz davon.

3. Verbindung nach Anspruch 1, wobei
• **R¹** C₁₋₃-Alkyl repräsentiert;
**R²** C₁₋₄-Alkyl repräsentiert;
**R³** 2,2,2-Trifluorethyl repräsentiert;
**-L-** Folgendes repräsentiert:
> *-C≡C-C₀₋₂-Alkylen-**, optional mit Hydroxy monosubstituiert; wobei das Sternchen (*) den Anlagerungspunkt an den Pyridinylring bezeichnet und zwei Sternchen (**) den Anlagerungspunkt an **R⁴** bezeichnen); und
**R⁴** Folgendes repräsentiert:
> monosubstituiertes C₃₋₇-Cycloalkyl, wobei der Substituent Hydroxy ist; oder
> unsubstituiertes 6-gliedriges Heteroaryl mit einem oder zwei Ringstickstoffatomen; oder
• **R¹** C₁₋₃-Alkyl repräsentiert;
**R²** C₁₋₄-Alkyl repräsentiert;
**R³** 2,2,2-Trifluorethyl repräsentiert;
**-L-** Folgendes repräsentiert:
> Oxadiazol-diyl; und
**R⁴** Folgendes repräsentiert:
> monosubstituiertes C₃₋₇-Cycloalkyl, wobei der Substituent aus Hydroxy, Carbamoyl und C₁₋₃-Alkylcarbonylamino ausgewählt ist; oder
> gesättigtes 5- bis 8-gliedriges verbrücktes oder spiro-bicyclisches Kohlenwasserstoffringsystem, wobei ein Ringkohlenstoffatom optional durch ein Stickstoffatom ersetzt ist, wobei das 5- bis 8-gliedrige verbrückte oder spiro-bicyclische Kohlenwasserstoffringsystem unabhängig monosubstituiert ist mit Hydroxy, Hydroxy-C₁₋₃-alkyl, Carbamoyl, C₁₋₃-Alkylcarbonylamino oder C₁₋₃-Alkylcarbonyl; oder
• **R¹** C₁₋₃-Alkyl repräsentiert;
**R²** C₁₋₄-Alkyl repräsentiert;
**R³** Isopropyl repräsentiert; und
**-L-R⁴** 2-(4-Hydroxy-cyclohexyl)-ethyn-1-yl, 3-Hydroxy-3-(pyrimidin-4-yl)-but-1-yn-1-yl, 5-(4-Carbamoyl-cyclohexyl)-1,2,4-oxadiazol-3-yl, 5-(4-Hydroxy-bicyclo[2.2.2]octan-1-yl)-1,2,4-oxadiazol-3-yl, 5-(3-Acetamido-bicyclo[1.1.1]pentan-1-yl)-1,2,4-oxadiazol-3-yl, 5-(2-Acetyl-2-azaspiro[3.3]heptan-6-yl)-1,2,4-oxadiazol-3-yl, 5-(4-Acetamido-cyclohexyl)-1,2,4-oxadiazol-3-yl, 5-(4-(Ethyl-carbonyl-amino)-cyclohexyl)-1,2,4-oxadiazol-3-yl oder 5-(4-Carbamoyl-bicyclo[2.2.2]octan-1-yl)-1,2,4-oxadiazol-3-yl repräsentiert; oder
• **R¹** C₁₋₃-Alkyl repräsentiert;
**R²** C₁₋₄-Alkyl repräsentiert;
**R³** 2,2,2-Trifluorethyl repräsentiert; und
**-L-R⁴** 3-(2-Hydroxy-1,1-dimethyl-ethyl)-1,2,4-oxadiazol-5-yl repräsentiert;
oder ein pharmazeutisch akzeptables Salz davon.

4. Verbindung nach einem der Ansprüche 1 bis 3, wobei R¹ Methyl repräsentiert; oder ein pharmazeutisch akzeptables Salz davon.

5. Verbindung nach einem der Ansprüche 1 bis 4, wobei R² Methyl repräsentiert; oder ein pharmazeutisch akzeptables Salz davon.

6. Verbindung nach Anspruch 1, wobei
• **R¹** Methyl repräsentiert;
**R²** Methyl repräsentiert;
**R³** 2,2,2-Trifluorethyl repräsentiert; und
**-L-R⁴** 5-(4-Hydroxy-cyclohexyl)-1,2,4-oxadiazol-3-yl, 5-(3-Hydroxymethyl-bicyclo[1.1.1]pentan-1-yl)-1,2,4-oxadiazol-3-yl, 3-(3-Hydroxymethyl-bicyclo[1.1.1]pentan-1-yl)-1,2,4-oxadiazol-5-yl, 2-(4-Hydroxy-cyclohexyl)-ethyn-1-yl, 3-Hydroxy-3-(pyrimidin-4-yl)-but-1-yn-1-yl, 5-(4-Carbamoyl-cyclohexyl)-1,2,4-oxadiazol-3-yl, 5-(4-Hydroxy-bicyclo[2.2.2]octan-1-yl)-1,2,4-oxadiazol-3-yl, 5-(3-Acetamido-bicyclo[1.1.1]pentan-1-yl)-1,2,4-oxadiazol-3-yl, 5-(2-Acetyl-2-azaspiro[3.3]heptan-6-yl)-1,2,4-oxadiazol-3-yl, 5-(4-Acetamido-cyclohexyl)-1,2,4-oxadiazol-3-yl, 5-(4-(Ethyl-carbonyl-amino)-cyclohexyl)-1,2,4-oxadiazol-3-yl oder 5-(4-Carbamoyl-bicyclo[2.2.2]octan-1-yl)-1,2,4-oxadiazol-3-yl repräsentiert; oder
• **R¹** Methyl repräsentiert;
**R²** Methyl repräsentiert;
**R³** Isopropyl repräsentiert; und
**-L-R⁴** 2-(4-Hydroxy-cyclohexyl)-ethyn-1-yl, 3-Hydroxy-3-(pyrimidin-4-yl)-but-1-yn-1-yl, 5-(4-Carbamoyl-cyclohexyl)-1,2,4-oxadiazol-3-yl, 5-(4-Hydroxy-bicyclo[2.2.2]octan-1-yl)-1,2,4-oxadiazol-3-yl, 5-(3-Acetamido-bicyclo[1.1.1]pentan-1-yl)-1,2,4-oxadiazol-3-yl, 5-(2-Acetyl-2-azaspiro[3.3]heptan-6-yl)-1,2,4-oxadiazol-3-yl, 5-(4-Acetamido-cyclohexyl)-1,2,4-oxadiazol-3-yl, 5-(4-(Ethyl-carbonyl-amino)-cyclohexyl)-1,2,4-oxadiazol-3-yl oder 5-(4-Carbamoyl-bicyclo[2.2.2]octan-1-yl)-1,2,4-oxadiazol-3-yl repräsentiert; oder
• **R¹** Methyl repräsentiert;
**R²** Methyl repräsentiert;
**R³** 2,2,2-Trifluorethyl repräsentiert; und
**-L-R⁴** 3-(2-Hydroxy-1,1-dimethyl-ethyl)-1,2,4-oxadiazol-5-yl repräsentiert;
oder ein pharmazeutisch akzeptables Salz davon.

7. Verbindung nach Anspruch 1, wobei
**R¹** Methyl repräsentiert;
**R²** Methyl repräsentiert;
**R³** Isopropyl repräsentiert; und
**-L-R⁴** 2-(4-Hydroxy-cyclohexyl)-ethyn-1-yl, 3-Hydroxy-3-(pyrimidin-4-yl)-but-1-yn-1-yl, 5-(4-Carbamoyl-cyclohexyl)-1,2,4-oxadiazol-3-yl, 5-(4-Hydroxy-bicyclo[2.2.2]octan-1-yl)-1,2,4-oxadiazol-3-yl, 5-(3-Acetamido-bicyclo[1.1.1]pentan-1-yl)-1,2,4-oxadiazol-3-yl, 5-(2-Acetyl-2-azaspiro[3.3]heptan-6-yl)-1,2,4-oxadiazol-3-yl, 5-(4-Acetamido-cyclohexyl)-1,2,4-oxadiazol-3-yl, 5-(4-(Ethyl-carbonyl-amino)-cyclohexyl)-1,2,4-oxadiazol-3-yl oder 5-(4-Carbamoyl-bicyclo[2.2.2]octan-1-yl)-1,2,4-oxadiazol-3-yl repräsentiert;
oder ein pharmazeutisch akzeptables Salz davon.

8. Verbindung nach Anspruch 1, wobei
**R¹** Methyl repräsentiert;
**R²** Methyl repräsentiert;
**R³** Isopropyl repräsentiert; und
**-L-R⁴** 5-(4-Acetamido-cyclohexyl)-1,2,4-oxadiazol-3-yl repräsentiert;
oder ein pharmazeutisch akzeptables Salz davon.

9. Verbindung nach einem der Ansprüche 1 bis 8, wobei das asymmetrische Kohlenstoffatom, das die Hydroxygruppe trägt, die in Formel (II) dargestellte absolute Konfiguration aufweist 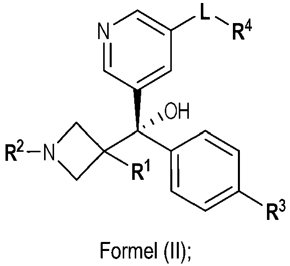 oder ein pharmazeutisch akzeptables Salz davon.

10. Verbindung nach Anspruch 1, die wie folgt ist:
trans-4-[3-(5-{(R)-(1,3-Dimethyl-azetidin-3-yl)-hydroxy-[4-(2,2,2-trifluorethyl)-phenyl]-methyl}-pyridin-3-yl)-[1,2,4]oxadiazol-5-yl]-cyclohexanol;
cis-4-[3-(5-{(R)-(1,3-Dimethyl-azetidin-3-yl)-hydroxy-[4-(2,2,2-trifluorethyl)-phenyl]-methyl}-pyridin-3-yl)-[1,2,4]oxadiazol-5-yl]-cyclohexanol;
(R)-(1,3-Dimethyl-azetidin-3-yl)-{5-[5-(3-hydroxymethyl-bicyclo[1.1.1]pent-1-yl)-[1,2,4]oxadiazol-3-yl]-pyridin-3-yl}-[4-(2,2,2-trifluorethyl)-phenyl]-methanol;
N-[3-(3-{5-[(R)-(1,3-Dimethyl-azetidin-3-yl)-hydroxy-(4-isopropyl-phenyl)-methyl]-pyridin-3-yl}-[1,2,4]oxadiazol-5-yl)-bicyclo[1.1.1]pent-1-yl]-acetamid;
4-(3-{5-[(R)-(1,3-Dimethyl-azetidin-3-yl)-hydroxy-(4-isopropyl-phenyl)-methyl]-pyridin-3-yl}-[1,2,4]oxadiazol-5-yl)-bicyclo[2.2.2]octan-1-ol;
2-[5-(5-{(R)-(1,3-Dimethyl-azetidin-3-yl)-hydroxy-[4-(2,2,2-trifluorethyl)-phenyl]-methyl}-pyridin-3-yl)-[1,2,4]oxadiazol-3-yl]-2-methyl-propan-1-ol;
1-[6-(3-{5-[(R)-(1,3-Dimethyl-azetidin-3-yl)-hydroxy-(4-isopropyl-phenyl)-methyl]-pyridin-3-yl}-[1,2,4]oxadiazol-5-yl)-2-aza-spiro[3.3]hept-2-yl]-ethanon;
trans-N-[4-(3-{5-[(R)-(1,3-Dimethyl-azetidin-3-yl)-hydroxy-(4-isopropyl-phenyl)-methyl]-pyridin-3-yl}-[1,2,4]oxadiazol-5-yl)-cyclohexyl]-propionamid;
cis-N-[4-(3-{5-[(R)-(1,3-Dimethyl-azetidin-3-yl)-hydroxy-(4-isopropyl-phenyl)-methyl]-pyridin-3-yl}-[1,2,4]oxadiazol-5-yl)-cyclohexyl]-propionamid;
cis-N-[4-(3-{5-[(R)-(1,3-Dimethyl-azetidin-3-yl)-hydroxy-(4-isopropyl-phenyl)-methyl]-pyridin-3-yl}-[1,2,4]oxadiazol-5-yl)-cyclohexyl]-acetamid;
trans-N-[4-(3-{5-[(R)-(1,3-Dimethyl-azetidin-3-yl)-hydroxy-(4-isopropyl-phenyl)-methyl]-pyridin-3-yl}-[1,2,4]oxadiazol-5-yl)-cyclohexyl]-acetamid;
(R)-(1,3-Dimethyl-azetidin-3-yl)-{5-[3-(3-hydroxymethyl-bicyclo[1.1.1]pent-1-yl)-[1,2,4]oxadiazol-5-yl]-pyridin-3-yl}-[4-(2,2,2-trifluorethyl)-phenyl]-methanol;
(R)-4-{5-[(R)-(1,3-Dimethyl-azetidin-3-yl)-hydroxy-(4-isopropyl-phenyl)-methyl]-pyridin-3-yl}-2-pyrimidin-4-yl-but-3-yn-2-ol;
(S)-4-{5-[(R)-(1,3-Dimethyl-azetidin-3-yl)-hydroxy-(4-isopropyl-phenyl)-methyl]-pyridin-3-yl}-2-pyrimidin-4-yl-but-3-yn-2-ol;
trans-4-{5-[(R)-(1,3-Dimethyl-azetidin-3-yl)-hydroxy-(4-isopropyl-phenyl)-methyl]-pyridin-3-ylethynyl}-cyclohexanol; cis-4-{5-[(R)-(1,3-Dimethyl-azetidin-3-yl)-hydroxy-(4-isopropyl-phenyl)-methyl]-pyridin-3-ylethynyl}-cyclohexanol;
4-(3-{5-[(R)-(1,3-Dimethyl-azetidin-3-yl)-hydroxy-(4-isopropyl-phenyl)-methyl]-pyridin-3-yl}-[1,2,4]oxadiazol-5-yl)-bicyclo[2.2.2]octane-1-carbonsäureamid;
cis-4-(3-{5-[(R)-(1,3-Dimethyl-azetidin-3-yl)-hydroxy-(4-isopropyl-phenyl)-methyl]-pyridin-3-yl}-[1,2,4]oxadiazol-5-yl)-cyclohexancarbonsäureamid; oder
trans-4-(3-{5-[(R)-(1,3-Dimethyl-azetidin-3-yl)-hydroxy-(4-isopropyl-phenyl)-methyl]-pyridin-3-yl}-[1,2,4]oxadiazol-5-yl)-cyclohexancarbonsäureamid;
oder ein pharmazeutisch akzeptables Salz davon.

11. Verbindung nach Anspruch 1, die wie folgt ist:
cis-N-[4-(3-{5-[(R)-(1,3-Dimethyl-azetidin-3-yl)-hydroxy-(4-isopropyl-phenyl)-methyl]-pyridin-3-yl}-[1,2,4]oxadiazol-5-yl)-cyclohexyl]-acetamid; oder ein pharmazeutisch akzeptables Salz davon.

12. Verbindung nach Anspruch 1, die wie folgt ist:
trans-N-[4-(3-{5-[(R)-(1,3-Dimethyl-azetidin-3-yl)-hydroxy-(4-isopropyl-phenyl)-methyl]-pyridin-3-yl}-[1,2,4]oxadiazol-5-yl)-cyclohexyl]-acetamid; oder ein pharmazeutisch akzeptables Salz davon.

13. Pharmazeutische Zusammensetzung, die eine Verbindung nach einem der Ansprüche 1 bis 12 oder ein pharmazeutisch akzeptables Salz davon umfasst, die ferner mindestens einen pharmazeutisch akzeptables Träger umfasst.

14. Verbindung nach einem der Ansprüche 1 bis 12 oder ein pharmazeutisch akzeptables Salz davon zur Verwendung als Arzneimittel.

15. Verbindung nach einem der Ansprüche 1 bis 12 oder ein pharmazeutisch akzeptables Salz davon zur Verwendung bei der Vorbeugung oder Behandlung von entzündlichen/autoimmunen Erkrankungen, Zuständen oder Störungen.

16. Verbindung nach einem der Ansprüche 1 bis 12 oder ein pharmazeutisch akzeptables Salz davon zur Verwendung bei der Vorbeugung oder Behandlung von Krebs.

17. Verbindung nach einem der Ansprüche 1 bis 12 oder ein pharmazeutisch akzeptables Salz davon zur Verwendung nach Anspruch 15 zur Vorbeugung oder Behandlung von rheumatoider Arthritis, ankylosierender Spondylitis, Spondyloarthritis, Psoriasis, psoriatischer Arthritis, entzündlichen Hauterkrankungen, Rosazea, Morbus Crohn, Colitis ulcerosa, Reizdarmsyndrom, entzündlichen Darmerkrankungen, Syndrom des trockenen Auges, Multipler Sklerose, systemischem Lupus erythematodes, Sjögren-Syndrom, autoimmuner Hepatitis, primär sklerosierender Cholangitis, Psoriasis, Plaque-Psoriasis, Psoriasis guttata, inverser Psoriasis; pustulöser Psoriasis; erythrodermischer Psoriasis; Autoimmunkeratitis; filamentärer Keratitis; Autoimmunuveitis; allergischer Konjunktivitis; Asthma; allergischen Erkrankung des Magen-Darm-Trakts; Typ-1-Diabetes; Endometriose; Meibomdrüsen-Dysfunktion oder Graft-versus-Host-Reaktion.

18. Verbindung nach einem der Ansprüche 1 bis 12 oder ein pharmazeutisch akzeptables Salz davon zur Verwendung nach Anspruch 16 zur Vorbeugung oder Behandlung von Lymphomen; T-Zell-Lymphom; primärem mediastinalem B-Zell-Lymphom; Hirntumor; Gliom; Glioblastom; Brustkrebs; dreifach negativem Brustkrebs; Darmkrebs; Leberkarzinom; Nierenzellkarzinom; Lungenkrebs; nicht-kleinzelligem Lungenkrebs; kleinzelligem Lungenkrebs; Magenkrebs; Melanom; Merkelzellkarzinom; kutanem Plattenepithelkarzinom; malignem Melanom; Blasenkrebs; Kopf-Hals-Krebs; Plattenepithelkarzinom des Kopfes und Halses; Hodgkin-Lymphom; Gebärmutterhalskrebs; Endometriumkarzinom; Dickdarmkrebs; gastrointestinalen Stromatumoren; Bauchspeicheldrüsenkrebs; Prostatakrebs; Leukämie; akuter myeloischer Leukämie; Eierstockkrebs; Ösophaguskarzinomen; Mesotheliom; Neuroblastom; Sarkom; hochgradigem Osteosarkom; Astrozytom; Myelom; Urothelkarzinom; lokal fortgeschrittenem und metastasiertem Urothelkarzinom; MSI-H- oder dMMR-Krebs; Rektumkarzinom; Kehlkopfkrebs; Adenokarzinom der Speicheldrüse; multiplem Myelom; Cholangiokarzinom; Plattenepithelkarzinom der Mundhöhle; Schilddrüsenkrebs; oder Krebs der ösophagogastralen Übergangszone.

19. Verbindung nach einem der Ansprüche 1 bis 12 oder ein pharmazeutisch akzeptables Salz davon zur Verwendung nach Anspruch 15 zur Vorbeugung oder Behandlung von entzündlichen/autoimmunen Erkrankungen/Störungen, ausgewählt aus Psoriasis, Psoriasis-Arthritis, rheumatoider Arthritis, Spondylitis ankylosans, Spondyloarthritis, entzündlichen Hauterkrankungen einschließlich Rosacea, Morbus Crohn, Colitis ulcerosa, Reizdarmsyndrom, Syndrom des trockenen Auges, Multiple Sklerose, systemischer Lupus erythematodes, Sjögren-Syndrom, Autoimmunhepatitis und primär sklerosierende Cholangitis.

20. Verbindung nach einem der Ansprüche 1 bis 12 oder ein pharmazeutisch akzeptables Salz davon zur Verwendung nach Anspruch 16 zur Vorbeugung oder Behandlung von Krebs, ausgewählt aus Lymphomen, einschließlich T-Zell-Lymphomen; Hirntumoren, einschließlich Gliomen oder Glioblastomen; Brustkrebs; Darmkrebs; Leberkarzinomen; Nierenzellkarzinomen; Lungenkrebs und Magenkrebs.

## Revendications

1. Composé de Formule (I) 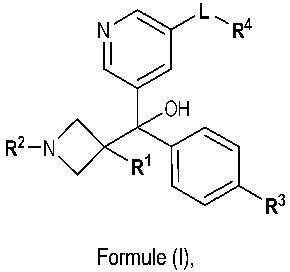 où
• **R¹** représente un C₁₋₃-alkyle ;
**R²** représente un C₁₋₄-alkyle ;
**R³** représente un 2,2,2-trifluoro-éthyle ;
**-L-** représente
➢ un *-C≡C-C₀₋₂-alkylène-** éventuellement monosubstitué par un hydroxy ; où l'astérisque (*) indique le point d'attache au noyau pyridinyle et les deux astérisques (**) indiquent le point d'attache à **R⁴** ; ou
> un oxadiazol-diyle ; et
**R⁴** représente
➢ un C₃₋₇-Cycloalkyle ;
➢ un système hydrocarboné fermé saturé bicyclique ponté ou spiro à 5 à 8 chaînons dans lequel un atome de carbone du cycle est éventuellement remplacé par un atome d'azote ; ou
➢ un hétéroaryle à 6 chaînons contenant un ou deux atomes d'azote sur le cycle ;
où **R⁴** est indépendamment non substitué ou monosubstitué par un hydroxy, hydroxy-C₁₋₃-alkyle, carbamoyle, C₁₋₃-alkyl-carbonyl-amino ou C₁₋₃-alkyl-carbonyle ; ou
• **R¹** représente un C₁₋₃-alkyle ;
**R²** représente un C₁₋₄-alkyle ;
**R³** représente un isopropyle ; et
**-L-R⁴** représente un 2-(4-hydroxy-cyclohexyl)-éthyn-1-yle, 3-hydroxy-3-(pyrimidin-4-yl)-but-1-yn-1-yle, 5-(4-carbamoyl-cyclohexyl)-1,2,4-oxadiazol-3-yle, 5-(4-hydroxy-bicyclo[2.2.2]octan-1-yl)-1,2,4-oxadiazol-3-yle, 5-(3-acétamido-bicyclo[1.1.1]pentan-1-yl)-1,2,4-oxadiazol-3-yle, 5-(2-acétyl-2-azaspiro[3.3]heptan-6-yl)-1,2,4-oxadiazol-3-yle, 5-(4-acétamido-cyclohexyl)-1,2,4-oxadiazol-3-yle, 5-(4-(éthyl-carbonyl-amino)-cyclohexyl)-1,2,4-oxadiazol-3-yle ou 5-(4-carbamoyl-bicyclo[2.2.2]octan-1-yl)-1,2,4-oxadiazol-3-yle ; ou
• **R¹** représente un C₁₋₃-alkyle ;
**R²** représente un C₁₋₄-alkyle ;
**R³** représente un 2,2,2-trifluoro-éthyle ; et
**-L-R⁴** représente un 3-(2-hydroxy-1,1-diméthyl-éthyl)-1,2,4-oxadiazol-5-yle ;
ou sel pharmaceutiquement acceptable de celui-ci.

2. Composé selon la revendication 1, où
• **R¹** représente un C₁₋₃-alkyle ;
**R²** représente un C₁₋₄-alkyle ;
**R³** représente un 2,2,2-trifluoro-éthyle ;
**-L-** représente
➢ un *-C≡C-C₀₋₂-alkylène-* * éventuellement monosubstitué par un hydroxy ; où l'astérisque (*) indique le point d'attache au noyau pyridinyle et les deux astérisques (**) indiquent le point d'attache à **R⁴ ;** ou
> un oxadiazol-diyle ; et
**R⁴** représente
> un C₃₋₇-cycloalkyle monosubstitué, où le substituant est sélectionné parmi un hydroxy, carbamoyle, et C₁₋₃-alkyl-carbonyl-amino ;
➢ un système hydrocarboné fermé saturé bicyclique ponté ou spiro à 5 à 8 chaînons dans lequel un atome de carbone du cycle est éventuellement remplacé par un atome d'azote, ledit système hydrocarboné fermé bicyclique ponté ou spiro à 5 à 8 chaînons étant indépendamment monosubstitué par un hydroxy, hydroxy-C₁₋₃-alkyle, carbamoyle, C₁₋₃-alkyl-carbonyl-amino ou C₁₋₃-alkyl-carbonyle ; ou
➢ un hétéroaryle à 6 chaînons non substitué contenant un ou deux atomes d'azote sur le cycle ; ou
• **R¹** représente un C₁₋₃-alkyle ;
**R²** représente un C₁₋₄-alkyle ;
**R³** représente un isopropyle ; et
**-L-R⁴** représente un 2-(4-hydroxy-cyclohexyl)-éthyn-1-yle, 3-hydroxy-3-(pyrimidin-4-yl)-but-1-yn-1-yle, 5-(4-carbamoyl-cyclohexyl)-1,2,4-oxadiazol-3-yle, 5-(4-hydroxy-bicyclo[2.2.2]octan-1-yl)-1,2,4-oxadiazol-3-yle, 5-(3-acétamido-bicyclo[1.1.1]pentan-1-yl)-1,2,4-oxadiazol-3-yle, 5-(2-acétyl-2-azaspiro[3.3]heptan-6-yl)-1,2,4-oxadiazol-3-yle, 5-(4-acétamido-cyclohexyl)-1,2,4-oxadiazol-3-yle, 5-(4-(éthyl-carbonyl-amino)-cyclohexyl)-1,2,4-oxadiazol-3-yle ou 5-(4-carbamoyl-bicyclo[2.2.2]octan-1-yl)-1,2,4-oxadiazol-3-yle ; ou
• **R¹** représente un C₁₋₃-alkyle ;
**R²** représente un C₁₋₄-alkyle ;
**R³** représente un 2,2,2-trifluoro-éthyle ; et
**-L-R⁴** représente un 3-(2-hydroxy-1,1-diméthyl-éthyl)-1,2,4-oxadiazol-5-yle ;
ou sel pharmaceutiquement acceptable de celui-ci.

3. Composé selon la revendication 1, où
• **R¹** représente un C₁₋₃-alkyle ;
**R²** représente un C₁₋₄-alkyle ;
**R³** représente un 2,2,2-trifluoro-éthyle ;
**-L-** représente
> *-C≡C-C₀₋₂-alkylène-* * éventuellement monosubstitué par un hydroxy ; où l'astérisque (*) indique le point d'attache au noyau pyridinyle et les deux astérisques (**) indiquent le point d'attache à **R⁴** ; et
**R⁴** représente
> un C₃₋₇-cycloalkyle monosubstitué, où le substituant est un hydroxy ; ou
➢ un hétéroaryle à 6 chaînons non substitué contenant un ou deux atomes d'azote sur le cycle ; ou
• **R¹** représente un C₁₋₃-alkyle ;
**R²** représente un C₁₋₄-alkyle ;
**R³** représente un 2,2,2-trifluoro-éthyle ;
**-L-** représente
> un oxadiazol-diyle ; et
**R⁴** représente
> un C₃₋₇-cycloalkyle monosubstitué, où le substituant est sélectionné parmi un hydroxy, carbamoyle, et C₁₋₃-alkyl-carbonyl-amino ; ou
➢ un système hydrocarboné fermé saturé bicyclique ponté ou spiro à 5 à 8 chaînons dans lequel un atome de carbone du cycle est éventuellement remplacé par un atome d'azote, ledit système hydrocarboné fermé bicyclique ponté ou spiro à 5 à 8 chaînons étant indépendamment monosubstitué par un hydroxy, hydroxy-C₁₋₃-alkyle, carbamoyle, C₁₋₃-alkyl-carbonyl-amino ou C₁₋₃-alkyl-carbonyle ; ou
• **R¹** représente un C₁₋₃-alkyle ;
**R²** représente un C₁₋₄-alkyle ;
**R³** représente un isopropyle ; et
**-L-R⁴** représente un 2-(4-hydroxy-cyclohexyl)-éthyn-1-yle, 3-hydroxy-3-(pyrimidin-4-yl)-but-1-yn-1-yle, 5-(4-carbamoyl-cyclohexyl)-1,2,4-oxadiazol-3-yle, 5-(4-hydroxy-bicyclo[2.2.2]octan-1-yl)-1,2,4-oxadiazol-3-yle, 5-(3-acétamido-bicyclo[1.1.1]pentan-1-yl)-1,2,4-oxadiazol-3-yle, 5-(2-acétyl-2-azaspiro[3.3]heptan-6-yl)-1,2,4-oxadiazol-3-yle, 5-(4-acétamido-cyclohexyl)-1,2,4-oxadiazol-3-yle, 5-(4-(éthyl-carbonyl-amino)-cyclohexyl)-1,2,4-oxadiazol-3-yle ou 5-(4-carbamoyl-bicyclo[2.2.2]octan-1-yl)-1,2,4-oxadiazol-3-yle ; ou
• **R¹** représente un C₁₋₃-alkyle ;
**R²** représente un C₁₋₄-alkyle ;
**R³** représente un 2,2,2-trifluoro-éthyle ; et
**-L-R⁴** représente un 3-(2-hydroxy-1,1-diméthyl-éthyl)-1,2,4-oxadiazol-5-yle ;
ou sel pharmaceutiquement acceptable de celui-ci.

4. Composé selon l'une quelconque des revendications 1 à 3, où **R¹** représente un méthyle ; ou sel pharmaceutiquement acceptable de celui-ci.

5. Composé selon l'une quelconque des revendications 1 à 4, où **R²** représente un méthyle ; ou sel pharmaceutiquement acceptable de celui-ci.

6. Composé selon la revendication 1, où
• **R¹** représente un méthyle ;
**R²** représente un méthyle ;
**R³** représente un 2,2,2-trifluoro-éthyle ; et
**-L-R⁴** représente un 5-(4-hydroxy-cyclohexyl)-1,2,4-oxadiazol-3-yle, 5-(3-hydroxyméthyl-bicyclo[1.1.1]pentan-1-yl)-1,2,4-oxadiazol-3-yle, 3-(3-hydroxyméthyl-bicyclo[1.1.1]pentan-1-yl)-1,2,4-oxadiazol-5-yle, 2-(4-hydroxy-cyclohexyl)-éthyn-1-yle, 3-hydroxy-3-(pyrimidin-4-yl)-but-1-yn-1-yle, 5-(4-carbamoyl-cyclohexyl)-1,2,4-oxadiazol-3-yle, 5-(4-hydroxy-bicyclo[2.2.2]octan-1-yl)-1,2,4-oxadiazol-3-yle, 5-(3-acétamido-bicyclo[1.1.1]pentan-1-yl)-1,2,4-oxadiazol-3-yle, 5-(2-acétyl-2-azaspiro[3.3]heptan-6-yl)-1,2,4-oxadiazol-3-yle, 5-(4-acétamido-cyclohexyl)-1,2,4-oxadiazol-3-yle, | 5-(4-(éthyl-carbonyl-amino)-cyclohexyl)-1,2,4-oxadiazol-3-yle ou 5-(4-carbamoyl-bicyclo[2.2.2]octan-1-yl)-1,2,4-oxadiazol-3-yle ; ou
• **R¹** représente un méthyle ;
**R²** représente un méthyle ;
**R³** représente un isopropyle ; et
**-L-R⁴** représente un 2-(4-hydroxy-cyclohexyl)-éthyn-1-yle, 3-hydroxy-3-(pyrimidin-4-yl)-but-1-yn-1-yle, 5-(4-carbamoyl-cyclohexyl)-1,2,4-oxadiazol-3-yle, 5-(4-hydroxy-bicyclo[2.2.2]octan-1-yl)-1,2,4-oxadiazol-3-yle, 5-(3-acétamido-bicyclo[1.1.1]pentan-1-yl)-1,2,4-oxadiazol-3-yle, 5-(2-acétyl-2-azaspiro[3.3]heptan-6-yl)-1,2,4-oxadiazol-3-yle, 5-(4-acétamido-cyclohexyl)-1,2,4-oxadiazol-3-yle, 5-(4-(éthyl-carbonyl-amino)-cyclohexyl)-1,2,4-oxadiazol-3-yle ou 5-(4-carbamoyl-bicyclo[2.2.2]octan-1-yl)-1,2,4-oxadiazol-3-yle ; ou
• **R¹** représente un méthyle ;
**R²** représente un méthyle ;
**R³** représente un 2,2,2-trifluoro-éthyle ; et
**-L-R⁴** représente un 3-(2-hydroxy-1,1-diméthyl-éthyl)-1,2,4-oxadiazol-5-yle ;
ou sel pharmaceutiquement acceptable de celui-ci.

7. Composé selon la revendication 1, où
**R¹** représente un méthyle ;
**R²** représente un méthyle ;
**R³** représente un isopropyle ; et
**-L-R⁴** représente un 2-(4-hydroxy-cyclohexyl)-éthyn-1-yle, 3-hydroxy-3-(pyrimidin-4-yl)-but-1-yn-1-yle, 5-(4-carbamoyl-cyclohexyl)-1,2,4-oxadiazol-3-yle, 5-(4-hydroxy-bicyclo[2.2.2]octan-1-yl)-1,2,4-oxadiazol-3-yle, 5-(3-acétamido-bicyclo[1.1.1]pentan-1-yl)-1,2,4-oxadiazol-3-yle, 5-(2-acétyl-2-azaspiro[3.3]heptan-6-yl)-1,2,4-oxadiazol-3-yle, 5-(4-acétamido-cyclohexyl)-1,2,4-oxadiazol-3-yle, 5-(4-(éthyl-carbonyl-amino)-cyclohexyl)-1,2,4-oxadiazol-3-yle ou 5-(4-carbamoyl-bicyclo[2.2.2]octan-1-yl)-1,2,4-oxadiazol-3-yle ;
ou sel pharmaceutiquement acceptable de celui-ci.

8. Composé selon la revendication 1, où
**R¹** représente un méthyle ;
**R²** représente un méthyle ;
**R³** représente un isopropyle ; et
**-L-R⁴** représente un 5-(4-acétamido-cyclohexyl)-1,2,4-oxadiazol-3-yle ;
ou sel pharmaceutiquement acceptable de celui-ci.

9. Composé selon l'une quelconque des revendications 1 à 8, où l'atome de carbone asymétrique qui porte le groupement hydroxy a la configuration absolue illustrée à la Formule (II) 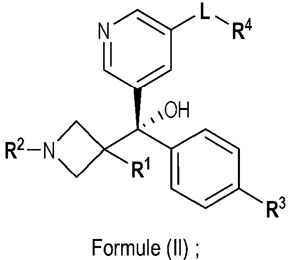 ou sel pharmaceutiquement acceptable de celui-ci.

10. Composé selon la revendication 1, qui est l'un des suivants :
trans-4-[3-(5-{(R)-(1,3-Diméthyl-azétidin-3-yl)-hydroxy-[4-(2,2,2-trifluoro-éthyl)-phényl]-méthyl}-pyridin-3-yl)-[1,2,4]oxadiazol-5-yl]-cyclohexanol ;
cis-4-[3-(5-{(R)-(1,3-Diméthyl-azétidin-3-yl)-hydroxy-[4-(2,2,2-trifluoro-éthyl)-phényl]-méthyl}-pyridin-3-yl)-[1,2,4]oxadiazol-5-yl]-cyclohexanol ;
(R)-(1,3-Diméthyl-azétidin-3-yl)-{5-[5-(3-hydroxyméthyl-bicyclo[1.1.1]pent-1-yl)-[1,2,4]oxadiazol-3-yl]-pyridin-3-yl}-[4-(2,2,2-trifluoro-éthyl)-phényl]-méthanol ;
N-[3-(3-{5-[(R)-(1,3-Diméthyl-azétidin-3-yl)-hydroxy-(4-isopropyl-phényl)-méthyl]-pyridin-3-yl}-[1,2,4]oxadiazol-5-yl)-bicyclo[1.1.1]pent-1-yl]-acétamide ;
4-(3-{5-[(R)-(1,3-Diméthyl-azétidin-3-yl)-hydroxy-(4-isopropyl-phényl)-méthyl]-pyridin-3-yl}-[1,2,4]oxadiazol-5-yl)-bicyclo[2.2.2]octan-1-ol ;
2-[5-(5-{(R)-(1,3-Diméthyl-azétidin-3-yl)-hydroxy-[4-(2,2,2-trifluoro-éthyl)-phényl]-méthyl}-pyridin-3-yl)-[1,2,4]oxadiazol-3-yl]-2-méthyl-propan-1-ol ;
1-[6-(3-{5-[(R)-(1,3-Diméthyl-azétidin-3-yl)-hydroxy-(4-isopropyl-phényl)-méthyl]-pyridin-3-yl}-[1,2,4]oxadiazol-5-yl)-2-aza-spiro[3.3]hept-2-yl]-éthanone ;
trans-N-[4-(3-{5-[(R)-(1,3-Diméthyl-azétidin-3-yl)-hydroxy-(4-isopropyl-phényl)-méthyl]-pyridin-3-yl}-[1,2,4]oxadiazol-5-yl)-cyclohexyl]-propionamide ;
cis-N-[4-(3-{5-[(R)-(1,3-Diméthyl-azétidin-3-yl)-hydroxy-(4-isopropyl-phényl)-méthyl]-pyridin-3-yl}-[1,2,4]oxadiazol-5-yl)-cyclohexyl]-propionamide ;
cis-N-[4-(3-{5-[(R)-(1,3-Diméthyl-azétidin-3-yl)-hydroxy-(4-isopropyl-phényl)-méthyl]-pyridin-3-yl}-[1,2,4]oxadiazol-5-yl)-cyclohexyl]-acétamide ;
trans-N-[4-(3-{5-[(R)-(1,3-Diméthyl-azétidin-3-yl)-hydroxy-(4-isopropyl-phényl)-méthyl]-pyridin-3-yl}-[1,2,4]oxadiazol-5-yl)-cyclohexyl]-acétamide ;
(R)-(1,3-Diméthyl-azétidin-3-yl)-{5-[3-(3-hydroxyméthyl-bicyclo[1.1.1]pent-1-yl)-[1,2,4]oxadiazol-5-yl]-pyridin-3-yl}-[4-(2,2,2-trifluoro-éthyl)-phényl]-méthanol ;
(R)-4-{5-[(R)-(1,3-Diméthyl-azétidin-3-yl)-hydroxy-(4-isopropyl-phényl)-méthyl]-pyridin-3-yl}-2-pyrimidin-4-yl-but-3-yn-2-ol ;
(S)-4-{5-[(R)-(1,3-Diméthyl-azétidin-3-yl)-hydroxy-(4-isopropyl-phényl)-méthyl]-pyridin-3-yl}-2-pyrimidin-4-yl-but-3-yn-2-ol ;
trans-4-{5-[(R)-(1,3-Diméthyl-azétidin-3-yl)-hydroxy-(4-isopropyl-phényl)-méthyl]-pyridin-3-yléthynyl}-cyclohexanol ; cis-4-{5-[(R)-(1,3-Diméthyl-azétidin-3-yl)-hydroxy-(4-isopropyl-phényl)-méthyl]-pyridin-3-yléthynyl}-cyclohexanol ;
Amide de l'acide 4-(3-{5-[(R)-(1,3-diméthyl-azétidin-3-yl)-hydroxy-(4-isopropyl-phényl)-méthyl]-pyridin-3-yl}-[1,2,4]oxadiazol-5-yl)-bicyclo[2.2.2]octan-1-carboxylique ;
Amide de l'acide cis-4-(3-{5-[(R)-(1,3-diméthyl-azétidin-3-yl)-hydroxy-(4-isopropyl-phényl)-méthyl]-pyridin-3-yl}-[1,2,4]oxadiazol-5-yl)-cyclohexanecarboxylique ; ou
Amide de l'acide trans-4-(3-{5-[(R)-(1,3-diméthyl-azétidin-3-yl)-hydroxy-(4-isopropyl-phényl)-méthyl]-pyridin-3-yl}-[1,2,4]oxadiazol-5-yl)-cyclohexanecarboxylique ;
ou sel pharmaceutiquement acceptable de celui-ci.

11. Composé selon la revendication 1, qui est le cis-N-[4-(3-{5-[(R)-(1,3-Diméthyl-azétidin-3-yl)-hydroxy-(4-isopropyl-phényl)-méthyl]-pyridin-3-yl}-[1,2,4]oxadiazol-5-yl)-cyclohexyl]-acétamide ; ou sel pharmaceutiquement acceptable de celui-ci.

12. Composé selon la revendication 1, qui est le trans-N-[4-(3-{5-[(R)-(1,3-Diméthyl-azétidin-3-yl)-hydroxy-(4-isopropyl-phényl)-méthyl]-pyridin-3-yl}-[1,2,4]oxadiazol-5-yl)-cyclohexyl]-acétamide ; ou sel pharmaceutiquement acceptable de celui-ci.

13. Composition pharmaceutique comprenant un composé selon l'une quelconque des revendications 1 à 12 ou un sel pharmaceutiquement acceptable de celui-ci et comprenant en outre au moins un véhicule pharmaceutiquement acceptable.

14. Composé selon l'une quelconque des revendications 1 à 12 ou sel pharmaceutiquement acceptable de celui-ci pour une utilisation en tant que médicament.

15. Composé selon l'une quelconque des revendications 1 à 12 ou sel pharmaceutiquement acceptable de celui-ci pour une utilisation dans la prévention ou le traitement de maladies, d'états pathologiques ou d'affections inflammatoires/auto-immunes.

16. Composé selon l'une quelconque des revendications 1 à 12 ou sel pharmaceutiquement acceptable de celui-ci pour une utilisation dans la prévention ou le traitement d'un cancer.

17. Composé selon l'une quelconque des revendications 1 à 12 ou sel pharmaceutiquement acceptable de celui-ci pour une utilisation selon la revendication 15 dans la prévention ou le traitement des états pathologiques suivants : polyarthrite rhumatoïde ; spondylarthrite ankylosante ; spondyloarthrite ; psoriasis ; rhumatisme psoriasique ; maladies inflammatoires cutanées ; rosacée ; maladie de Crohn ; rectocolite hémorragique ; syndrome de l'intestin irritable ; affection intestinale inflammatoire ; sécheresse oculaire ; sclérose en plaques ; lupus érythémateux aigu disséminé ; syndrome de Sjögren ; hépatite auto-immune ; cholangite sclérosante primitive ; psoriasis ; psoriasis en plaques ; psoriasis en gouttes ; psoriasis inversé ; psoriasis pustuleux ; psoriasis érythrodermique ; kératite auto-immune ; kératite filamenteuse ; uvéite auto-immune ; conjonctivite allergique ; asthme ; troubles gastro-intestinaux liés à une allergie ; diabète de type 1 ; endométriose ; dysfonctionnement des glandes de Meibomius ; ou maladie du greffon contre l'hôte.

18. Composé selon l'une quelconque des revendications 1 à 12 ou sel pharmaceutiquement acceptable de celui-ci pour une utilisation selon la revendication 16 dans la prévention ou le traitement des états pathologiques suivants : lymphome ; lymphome à cellules T ; lymphome médiastinal primitif à cellules B ; cancer du cerveau ; gliome ; glioblastome ; cancer du sein ; cancer du sein triple négatif ; cancer colorectal ; hépatocarcinome ; carcinome à cellules rénales ; cancer du poumon ; cancer du poumon non à petites cellules ; cancer du poumon à petites cellules ; cancer de l'estomac ; mélanome ; carcinome à cellules de Merkel ; carcinome épidermoïde de la peau ; mélanoblastome ; cancer de la vessie ; cancer de la tête et du cou ; cancer de la tête et du cou à cellules squameuses ; lymphome de Hodgkin ; cancer du col de l'utérus ; cancer de l'endomètre ; cancer du côlon ; tumeurs gastrointestinales stromales ; cancer du pancréas ; cancer de la prostate ; leucémie ; leucémie myéloïde aiguë ; cancer de l'ovaire ; carcinomes oesophagiens ; mésothéliome ; neuroblastome ; sarcome ; ostéosarcome de haut grade ; astrocytome ; myélome ; carcinome urothélial ; carcinome urothélial localement avancé et métastatique ; tumeurs MSI-H ou dMMR ; cancer du rectum ; cancer du larynx ; adénocarcinome des glandes salivaires ; myélome multiple ; cholangiocarcinome ; carcinome oral à cellules squameuses ; cancer de la thyroïde ; ou cancer de la jonction oesogastrique.

19. Composé selon l'une quelconque des revendications 1 à 12 ou sel pharmaceutiquement acceptable de celui-ci pour une utilisation selon la revendication 15 dans la prévention ou le traitement de maladies/d'affections inflammatoires/auto-immunes sélectionnées parmi les suivantes : psoriasis ; rhumatisme psoriasique ; polyarthrite rhumatoïde ; spondylarthrite ankylosante ; spondyloarthrite ; maladies inflammatoires cutanées, y compris la rosacée ; maladie de Crohn ; rectocolite hémorragique ; syndrome de l'intestin irritable ; sécheresse oculaire ; sclérose en plaques ; lupus érythémateux aigu disséminé ; syndrome de Sjögren ; hépatite auto-immune ; et cholangite sclérosante primitive.

20. Composé selon l'une quelconque des revendications 1 à 12 ou sel pharmaceutiquement acceptable de celui-ci pour une utilisation selon la revendication 16 dans la prévention ou le traitement d'un cancer sélectionné parmi les suivants : lymphome, y compris lymphome à cellules T ; cancer du cerveau, y compris gliome ou glioblastome ; cancer du sein ; cancer colorectal ; hépatocarcinomes ; carcinome à cellules rénales ; cancer du poumon ; et cancer de l'estomac.
